(19) Europäisches Patentamt
European Patent Office
Office européen des brevets

(11) Publication number: **0 029 707**
**B1**

(12) **EUROPEAN PATENT SPECIFICATION**

(45) Date of publication of patent specification: **01.02.84**

(21) Application number: **80304147.4**

(22) Date of filing: **19.11.80**

(51) Int. Cl.³: **C 07 D 401/04,**
C 07 D 405/14,
C 07 D 413/04,
C 07 D 413/14,
C 07 D 417/04,
C 07 D 417/14,
A 61 K 31/445,
A 61 K 31/505,
A 61 K 31/54,
A 61 K 31/535

(54) **Novel piperidine derivatives, method for the preparation thereof and pharmaceutical compositions containing them.**

(30) Priority: **21.11.79 JP 150056/79**
**10.03.80 JP 29106/80**
**16.05.80 JP 65094/80**
**21.05.80 JP 66531/80**
**21.05.80 JP 66532/80**
**27.05.80 JP 69619/80**
**22.08.80 JP 114759/80**

(43) Date of publication of application:
**03.06.81 Bulletin 81/22**

(45) Publication of the grant of the patent:
**01.02.84 Bulletin 84/5**

(84) Designated Contracting States:
**DE FR GB IT**

(56) References cited:
**DE - A - 2 507 639**
**US - A - 4 144 344**
**US - A - 4 148 796**
**US - A - 4 166 117**

(73) Proprietor: **KYOWA HAKKO KOGYO CO., LTD**
**Ohtemachi Bldg. Ohtemachi 1-chome Chiyoda-ku**
**Tokyo (JP)**

(72) Inventor: **Teranishi, Masayuki**
**2-13-24, Narusedai**
**Machida-shi, Tokyo (JP)**
Inventor: **Nakamizo, Nobuhiro**
**1704-22, Honmachida**
**Machida-shi Tokyo (JP)**
Inventor: **Obase, Hiroyuki**
**3-9-9, Naka-.machi**
**Machida-shi, Tokyo (JP)**
Inventor: **Kubo, Kazuhiro**
**Fruithoflaan 74**
**B-2600 Berchem (BE)**
Inventor: **Takai, Haruki**
**Green Haitsu Yurigaoka 401 210-13 Takaishi**
**Tama-ku Kawasaki-shi Kanagawa-ken (JP)**
Inventor: **Kasuya, Yutaka**
**1-61, Komukainishi-machi**
**Saiwai-ku Kawasaki-shi Kanagawa-ken (JP)**

(74) Representative: **Lambert, Hugh Richmond et al,**
**D. YOUNG & CO. 10 Staple Inn**
**London, WC1V 7RD (GB)**

**EP 0 029 707 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Novel piperidine derivatives, method for the preparation thereof and pharmaceutical compositions containing them

The present invention is concerned with piperidine derivatives, acid-addition salts thereof and pharmaceutical compositions containing them.

The piperidine derivatives of the present invention are compounds represented by the formula[I]:

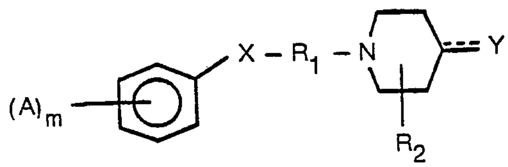

[I]

[where A is hydroxy, halogen, $C_{1-5}$ alkyl, $C_{1-5}$ alkoxy, alkenyloxy containing up to 5 carbon atoms, alkynyloxy containing up to 5 carbon atoms, $C_{1-5}$ alkylthio, carboxy, $C_{2-6}$ alkoxycarbonyl, nitro, amino, $C_{1-5}$ alkylamino, $C_{1-5}$ alkanoylamino, sulfamoyl, mono- or di-($C_{1-5}$ alkyl)aminosulfonyl, $C_{1-5}$ alkylsulfonyl, carbamoyl, cyano or trifluoromethyl, m is 0 or an integer of 1—5, and when m is 2 or more, A is the same or different or two A's may combine to form $C_{1-5}$ alkylenedioxy; X is carbonyl, hydroxymethylene or methylene; $R_1$ is straight-chain alkylene having 1—4 carbon atoms with or without $C_{1-5}$ alkyl substituent(s); $R_2$ is hydrogen or $C_{1-5}$ alkyl; and Y is a monovalent or divalent group and is one of the following groups:

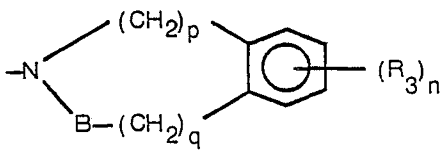

(wherein p and q are 0 or 1 provided that p and q are not 1 at the same time, B is

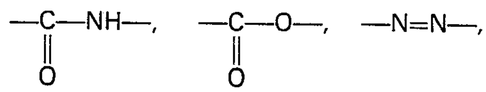

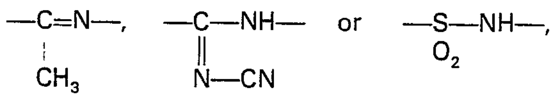

or

when p and q are 0, B is

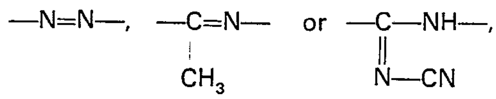

or

when p is 1 and q is 0, B is

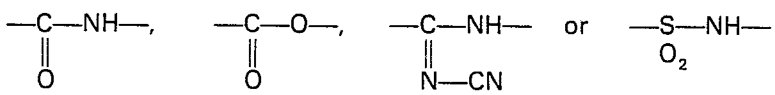

or

and when q is 1 and p is 0, B is

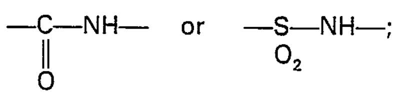

or ; and

$R_3$ is hydroxy, $C_{1-5}$ alkoxy, halogen, trifluoromethyl, trifluoromethoxy, trifluoromethylthio, nitro or amino, n is 0 or an integer of 1—4, and when n is 2 or more, $R_3$ is the same or different or two $R_3$'s may combine to form $C_{1-5}$ alkylenedioxy)

2)

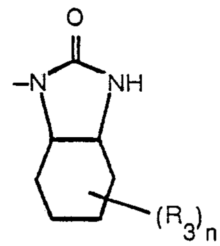

(wherein $R_3$ and n have the same significance as defined above) and

3)

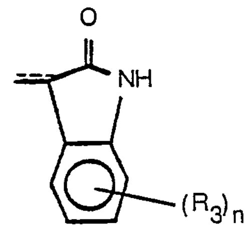

(wherein $R_3$ and n have the same significance as defined above)] (hereinafter referred to as Compound [I] and terms like this shall apply to other compounds).

The compounds represented by the formula [I] and the pharmaceutically acceptable acid addition salts thereof have a hypotensive activity, and therefore are useful as medicine.

Heretofore, the following compounds, each having a piperidine ring, were commercially available as tranquilizers.

benperidol

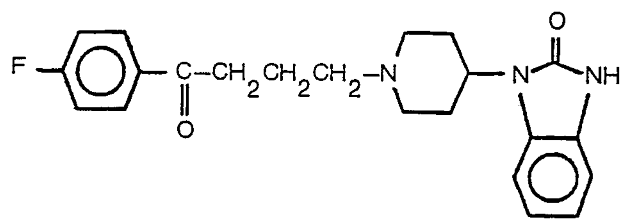

droperidol

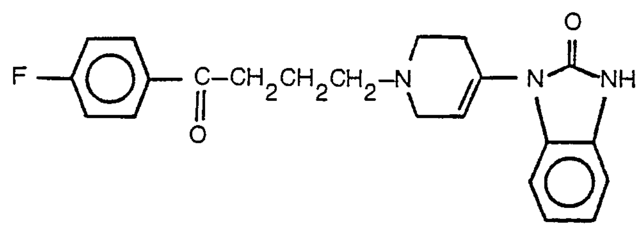

pimozide

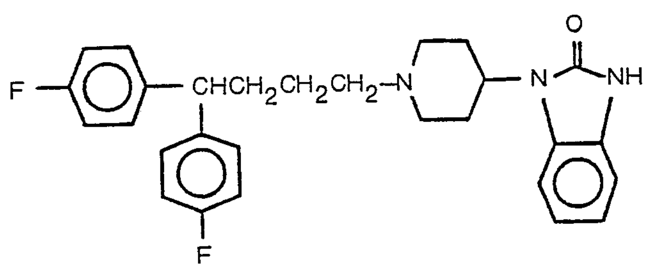

U.S. Patent No. US—A—4166117 discloses compounds represented by the following formula:

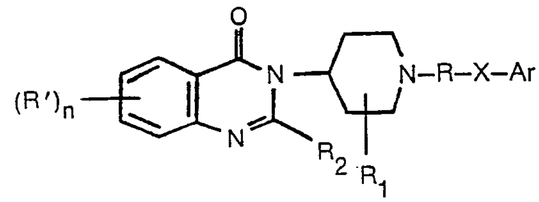

where X is oxygen, sulfur or a substituted or unsubstituted amino group.

U.S. Patent No. US—A—4,144,344 discloses compounds represented by the following formula:

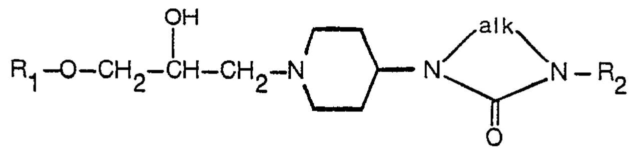

where $R_1$ is a substituted or unsubstituted aryl group.

Further an application for patent relating to compounds which have a hypotensive activity and are substantially represented by the following general formula:

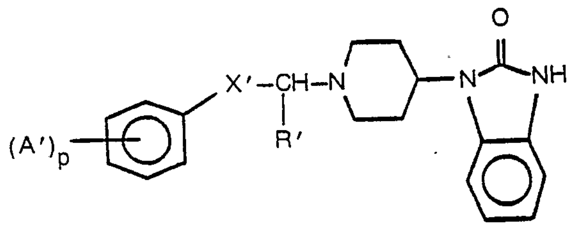

(wherein p is 0 or an integer of 1—3; A′ is method or two A′ 's may combine to form methylenedioxy; X′ is carbonyl, hydroxymethylene or methylene; and R′ is hydrogen or methyl) is pending (European Patent Specification EP—A—0018417).

Compounds having excellent pharmacological activity are always in demand. In order to obtain such compounds, studies have been made on piperidine derivatives and as a result, it has been found that novel piperidine derivatives represented by the formula [I] have a hypotensive activity. Further, it has been found that some compounds in the compounds represented by the formula [I] have an antiulcer activity, an antiplatelet aggregation activity, a cholesterol-lowering activity, antihistaminic activity or trachea relaxant activity.

Among the halogens in the definition of A and $R_3$ in Compound [I] are chlorine and bromine; among the $C_{1-5}$ groups, $C_{1-3}$ groups are preferred; $C_{2-6}$ alkoxycarbonyl preferably has 2—4 carbon atoms.

Compound [I] includes all of the optical isomers.

Examples of pharmacologically acceptable acid-addition salts of Compound [I] are inorganic acid-addition salts such as hydrochloride, hydrobromide, hydriodide, nitrate, sulfate and phosphate and organic acid-addition salts such as acetate, benzoate, maleate, fumarate, succinate, tartrate, citrate, oxalate, glyoxylate, aspartate, methanesulfonate, ethanesulfonate, propanesulfonate, methanedisulfonate, $\alpha,\beta$-ethanedisulfonate and benzenesulfonate.

Especially preferred compounds in Compound [I] are represented by the formula [I′]:

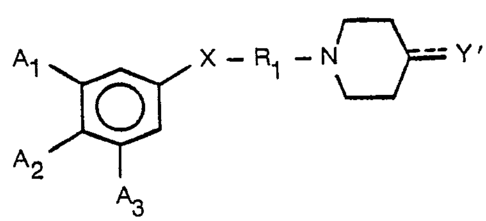

[I′]

[where $A_1$, $A_2$ and $A_3$ are the same or different, and are hydrogen or have the same significance as that of the above-mentioned A; X and $R_1$ are as defined above and Y′ is a monovalent or divalent group and is one of the following groups:

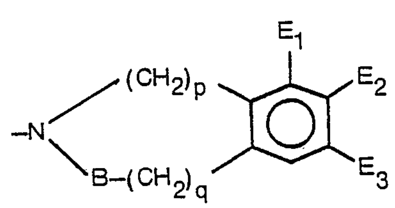

(where p, q and B have the same significance as defined above and $E_1$, $E_2$ and $E_3$ are hydrogen or are as defined above for $R_3$

2) 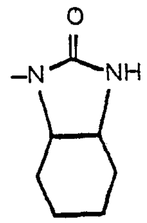 and 3) 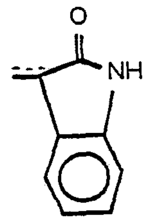 ]

or are acid-addition salts thereof.

Examples of Compound [I] are tabulated in the following Table 1. Table 2 shows structures and Tables 3—1, 2 and 3 show properties of the present compounds.

TABLE 1

| Compound No. | Compound |
|---|---|
| 1 | 1-(3,4-dimethoxybenzoylmethyl)-4-[3,4-dihydro-2(1H)-quinazolinon-3-yl]piperidine |
| 2 | 1-(3,4-methylenedioxybenzoylmethyl)-4-[3,4-dihydro-2(1H)-quinazolinon-3-yl]piperidine |
| 3 | 1-[2-(3,4-dimethoxyphenyl)-2-hydroxyethyl]-4-[3,4-dihydro-2(1H)-quinazolinon-3-yl]piperidine |
| 4 | 1-[2-(3,4-methylenedioxyphenyl)-2-hydroxyethyl]-4-[3,4-dihydro-2(1H)-quinazolinon-3-yl]piperidine |
| 5 | 1-[1-(3,4-dimethoxybenzoyl)ethyl]-4-[3,4-dihydro-2(1H)-quinazolinon-3-yl]piperidine |
| 6 | 1-[3-(3,4-dimethoxyphenyl)-3-hydroxypropyl-2]-4-[3,4-dihydro-2(1H)-quinazolinon-3-yl]piperidine |
| 7 | 1-[2-(3,4,5-trimethoxyphenyl)-2-hydroxyethyl]-4-[3,4-dihydro-2(1H)-quinazolinon-3-yl]piperidine |
| 8 | 1-(3,4-dimethoxybenzoylmethyl)-4-[3,4-dihydro-2(1H)-quinazolinon-1-yl]piperidine |
| 9 | 1-(3,4-methylenedioxybenzoylmethyl-4-[3,4-dihydro-2(1H)-quinazolinon-1-yl]piperidine |
| 10 | 1-[2-(3,4-dimethoxyphenyl)-2-hydroxyethyl]-4-[3,4-dihydro-2(1H)-quinazolinon-1-yl]piperidine |
| 11 | 1-[2-(3,4-methylenedioxyphenyl)-2-hydroxyethyl-4-[3,4-dihydro-2(1H)-quinazolinon-1-yl]piperidine |
| 12 | 1-[1-(3,4-dimethoxybenzoyl)ethyl]-4-[3,4-dihydro-2(1H)-quinazolinon-1-yl]piperidine |
| 13 | 1-[3-(3,4-dimethoxyphenyl)-3-hydroxypropyl-2]-4-[3,4-dihydro-2(1H)-quinazolinon-1-yl]piperidine |
| 14 | 1-benzoylmethyl-4-[3,4-dihydro-2(1H)-quinazolinon-3-yl]piperidine |
| 15 | 1-(3-chlorobenzoylmethyl)-4-[3,4-dihydro-2(1H)-quinazolinon-3-yl]piperidine |
| 16 | 1-(4-chlorobenzoylmethyl)-4-[3,4-dihydro-2(1H)-quinazolinon-3-yl]piperidine |
| 17 | 1-(4-methoxybenzoylmethyl)-4-[3,4-dihydro-2(1H)-quinazolinon-3-yl]piperidine |
| 18 | 1-(2-phenyl-2-hydroxyethyl)-4-[3,4-dihydro-2(1H)-quinazolinon-3-yl]piperidine |
| 19 | 1-[2-(3-chlorophenyl)-2-hydroxyethyl]-4-[3,4-dihydro-2(1H)-quinazolinon-3-yl]piperidine |

TABLE 1 (continued)

| Compound No. | Compound |
|---|---|
| 20 | 1-[2-(4-chlorophenyl)-2-hydroxyethyl]-4-[3,4-dihydro-2(1H)-quinazolinon-3-yl]piperidine |
| 21 | 1-[2-(4-methoxyphenyl)-2-hydroxyethyl]-4-[3,4-dihydro-2(1H)-quinazolinon-3-yl]piperidine |
| 22 | 1-[2-(3,4-dichlorophenyl)-2-hydroxyethyl]-4-[3,4-dihydro-2(1H)-quinazolinon-3-yl]piperidine |
| 23 | 1-(3,4-dimethoxybenzoylmethyl)-4-(2-oxo-3,4-dihydro-2H-1,3-benzoxazin-3-yl)piperidine |
| 24 | 1-(3,4-methylenedioxybenzoylmethyl)-4-(2-oxo-3,4-dihydro-2H-1,3-benzoxazin-3-yl)piperidine |
| 25 | 1-[2-(3,4-dimethoxyphenyl)-2-hydroxyethyl]-4-(2-oxo-3,4-dihydro-2H-1,3-benzoxazin-3-yl)piperidine |
| 26 | 1-[2-(3,4-methylenedioxyphenyl)-2-hydroxyethyl]-4-(2-oxo-3,4-dihydro-2H-1,3-benzoxazin-3-yl)piperidine |
| 27 | 1-[1-(3,4-dimethoxybenzoyl)-ethyl]-4-(2-oxo-3,4-dihydro-2H-1,3-benzoxazin-3-yl)piperidine |
| 28 | 1-[3-(3,4-dimethoxyphenyl)-3-hydroxypropan-2-yl]-4-(2-oxo-3,4-dihydro-2H-1,3-benzoxazin-3-yl)piperidine |
| 29 | 1-(3,4,5-trimethoxybenzoylmethyl)-4-(2-oxo-3,4-dihydro-2H-1,3-benzoxazin-3-yl)piperidine |
| 30 | 1-[2-(3,4,5-trimethoxyphenyl)-2-hydroxyethyl]-4-(2-oxo-3,4-dihydro-2H-1,3-benzoxazin-3-yl)piperidine |
| 31 | 1-[2-(3,4-methylenedioxyphenyl)-2-oxo-ethyl]-4-(1H-benzotriazol-1-yl)piperidine |
| 32 | 1-[2-(3,4-dimethoxyphenyl)-2-oxoethyl]-4-(1H-benzotriazol-1-yl)piperidine |
| 33 | 1-[1-(3,4-dimethoxybenzoyl)ethyl]-4-(1H-benzotriazol-1-yl)piperidine |
| 34 | 1-[2-(3,4-methylenedioxyphenyl)-2-hydroxy-ethyl]-4-(1H-benzotriazol-1-yl)piperidine |
| 35 | 1-[2-(3,4-dimethoxyphenyl)-2-hydroxy-ethyl]-4-(1H-benzotriazol-1-yl)piperidine |
| 36 | 1-[3-(3,4-dimethoxyphenyl)-3-hydroxypropyl-2]-4-(1H-benzotriazol-1-yl)piperidine |
| 37 | 1-[2-(3,4-methylenedioxyphenyl)-2-oxo-ethyl]-4-(2-methyl-benzimidazol-3-yl)piperidine |
| 38 | 1-[2-(3,4-dimethoxyphenyl)-2-oxo-ethyl]-4-(2-methyl-benzimidazol-3-yl)piperidine |
| 39 | 1-[1-(3,4-dimethoxybenzoyl)ethyl]-4-(2-methyl-benzimidazol-3-yl)piperidine |
| 40 | 1-[2-(3,4-methylenedioxyphenyl)-2-hydroxyethyl]-4-(2-methyl-benzimidazol-3-yl)piperidine |
| 41 | 1-[2-(3,4-dimethoxyphenyl)-2-hydroxyethyl]-4-(2-methyl-benzimidazol-3-yl)piperidine |
| 42 | 1-[3-(3,4-dimethoxyphenyl)-3-hydroxypropyl-2]-4-(2-methyl-benzimidazol-3-yl)piperidine |

TABLE 1 (continued)

| Compound No. | Compound |
|---|---|
| 43 | 1-(3,4-methylenedioxybenzoylmethyl)-4-(2-cyanoimino-3,4-dihydro-1H-quinazolin-3-yl)piperidine |
| 44 | 1-(3,4-dimethoxybenzoylmethyl)-4-(2-cyanoimino-3,4-dihydro-1H-quinazolin-3-yl)piperidine |
| 45 | 1-[2-(3,4-dimethoxyphenyl)-2-oxo-1-methylethyl]-4-(2-cyanoimino-3,4-dihydro-1H-quinazolin-3-yl)piperidine |
| 46 | 1-(3,4,5-trimethoxybenzoylmethyl)-4-(2-cyanoimino-3,4-dihydro-1H-quinazolin-3-yl)piperidine |
| 47 | 1-[2-(3,4,5-trimethoxyphenyl)-2-oxo-1-methylethyl]-4-(2-cyanoimino-3,4-dihydro-1H-quinazolin-3-yl)piperidine |
| 48 | 1-[2-(3,4-methylenedioxyphenyl)-2-oxo-ethyl]-4-(2-cyanoimino-1H-benzimidazol-1-yl)piperidine |
| 49 | 1-[2-(3,4-dimethoxyphenyl)-2-oxo-ethyl]-4-(2-cyanoimino-1H-benzimidazol-1-yl)piperidine |
| 50 | 1-[2-(3,4-methylenedioxyphenyl)-2-hydroxy-ethyl]-4-(2-cyanoimino-3,4-dihydro-1H-quinazolin-3-yl)piperidine |
| 51 | 1-[2-(3,4-dimethoxyphenyl)-2-hydroxy-ethyl]-4-(2-cyanoimino-3,4-dihydro-1H-quinazolin-3-yl)piperidine |
| 52 | 1-[2-(3,4-dimethoxyphenyl)-2-hydroxy-1-methyl-ethyl]-4-(2-cyanoimino-3,4-dihydro-1H-quinazolin-3-yl)piperidine |
| 53 | 1-[2-(3,4,5-trimethoxyphenyl)-2-hydroxy-ethyl]-4-(2-cyanoimino-3,4-dihydro-1H-quinazolin-3-yl)piperidine |
| 54 | 1-[2-(3,4,5-trimethoxyphenyl)-2-hydroxy-1-methyl-ethyl]-4-(2-cyanoimino-3,4-dihydro-1H-quinazolin-3-yl)piperidine |
| 55 | 1-[2-(3,4-methylenedioxyphenyl)-2-hydroxy-ethyl]-4-(2-cyanoimino-1H-benzimidazol-1-yl)piperidine |
| 56 | 1-[2-(3,4-dimethoxyphenyl)-2-hydroxy-ethyl]-4-(2-cyanoimino-1H-benzimidazol-1-yl)piperidine |
| 57 | 1-(3,4-dimethoxybenzoylmethyl)-4-(3,4-dihydro-2,2-dioxo-1H-2,1,3-benzothiadiazin-3-yl)piperidine |
| 58 | 1-[2-(3,4-dimethoxyphenyl)-2-hydroxyethyl]-4-(3,4-dihydro-2,2-dioxo-1H-2,1,3-benzothiadiazin-3-yl)piperidine |
| 59 | 1-(3,4-methylenedioxybenzoylmethyl)-4-(3,4-dihydro-2,2-dioxo-1H-2,1,3-benzothiadiazin-3-yl)piperidine |
| 60 | 1-[2-(3,4-methylenedioxyphenyl)-2-hydroxyethyl]-4-(3,4-dihydro-2,2-dioxo-1H-2,1,3-benzothiadiazin-3-yl)piperidine |
| 61 | 1-(3,4-dimethoxybenzoylmethyl)-4-(3,4-dihydro-2,2-dioxo-1H-2,1,3-benzothiadiazin-1-yl)piperidine |
| 62 | 1-(3,4-methylenedioxybenzoylmethyl)-4-(3,4-dihydro-2,2-dioxo-1H-2,1,3-benzothiadiazin-1-yl)piperidine |

TABLE 1 (continued)

| Compound No. | Compound |
|---|---|
| 63 | 1-[2-(3,4-dimethoxyphenyl)-2-hydroxyethyl]-4-(3,4-dihydro-2,2-dioxo-1H-2,1,3-benzothiadiazin-1-yl)piperidine |
| 64 | 1-[2-(3,4-methylenedioxyphenyl)-2-hydroxyethyl]-4-(3,4-dihydro-2,2-dioxo-1H-2,1,3-benzothiadiazin-1-yl)piperidine |
| 65 | 1-[1-(3,4-dimethoxybenzoyl)ethyl]-4-(3,4-dihydro-2,2-dioxo-1H-2,1,3-benzothiadiazin-1-yl)piperidine |
| 66 | 1-[3-(3,4-dimethoxyphenyl)-3-hydroxypropan-2-yl]-4-(3,4-dihydro-2,2-dioxo-1H-2,1,3-benzothiadiazin-1-yl)piperidine |
| 67 | 1-[(3,4-methylenedioxy)benzoylmethyl]-4-(2-oxyindol-3-yl)piperidine |
| 68 | 1-[(3,4-dimethoxy)benzoylmethyl]-4-(2-oxyindol-3-yl)piperidine |
| 69 | 1-[2-(3,4-dimethoxyphenyl)-2-oxo-1-methylethyl]-4-(2-oxyindol-3-yl)piperidine |
| 70 | 3-[1-(3,4-methylenedioxy)benzoylmethyl-4-piperidylidene-2-oxyindole |
| 71 | 3-[1-(3,4-dimethoxy)benzoylmethyl-4-piperidylidene]-2-oxyindole |
| 72 | 1-[2-(3,4-methylenedioxyphenyl)-hydroxyethyl]-4-(2-oxyindol-3-yl)piperidine |
| 73 | 1-[2-(3,4-dimethoxyphenyl)-hydroxyethyl]-4-(2-oxyindol-3-yl)piperidine |
| 74 | 1-[2-(3,4-dimethoxyphenyl)-2-hydroxy-1-methylethyl]-4-(2-oxyindol-3-yl)piperidine |
| 75 | 3-{1-[2-(3,4-methylenedioxyphenyl)-2-hydroxyethyl]-4-piperidylidene}-2-oxyindole |
| 76 | 3-{1-[2-(3,4-dimethoxyphenyl)-2-hydroxyethyl]-4-piperidylidane}-2-oxyindole |
| 77 | 1-[2-(3,4-dimethoxyphenyl)-2-oxoethyl]-4-(octahydro-2H-benzimidazol-2-one-1-yl)piperidine |
| 78 | 1-[2-(3,4-dimethoxyphenyl)-2-oxo-1-methylethyl]-4-(octahydro-2H-benzimidazol-2-one-1-yl)piperidine |
| 79 | 1-[1-(3,4,5-trimethoxybenzoyl)ethyl]-4-(octahydro-2H-benzimidazol-2-one-1-yl)piperidine |
| 80 | 1-[2-(3,4,5-trimethoxyphenyl)-2-oxoethyl]-4-(octahydro-2H-benzimidazol-2-one-1-yl)piperidine |
| 81 | 1-[2-(3,4-dimethoxyphenyl)-2-hydroxyethyl]-4-(octahydro-2H-benzimidazol-2-one-1-yl)piperidine |
| 82 | 1-[2-(3,4-dimethoxyphenyl)-2-hydroxy-1-methylethyl]-4-(octahydro-2H-benzimidazol-2-one-1-yl)piperidine |
| 83 | 1-[2-(3,4,5-trimethoxyphenyl)-2-hydroxy-1-methylethyl]-4-(octahydro-2H-benzimidazol-2-one-1-yl)piperidine |
| 84 | 1-[2-(3,4,5-trimethoxyphenyl)-2-hydroxyethyl]-4-(octahydro-2H-benzimidazol-2-one-1-yl)piperidine |

TABLE 2

Structure of compound

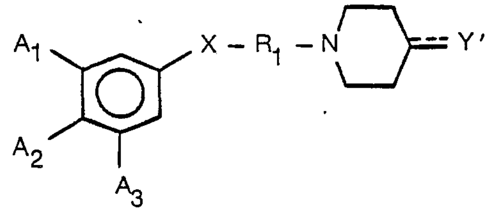

wherein Y′ is

1) 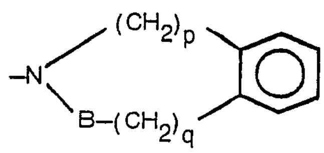

2) 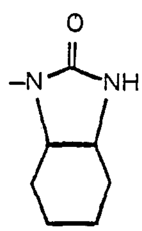 or 3) 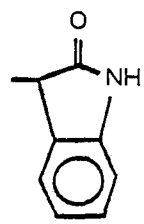

| Compound No. | Structure | | | | | | | | |
|---|---|---|---|---|---|---|---|---|---|
| | $A_1$ | $A_2$ | $A_3$ | X | $R_1$ | Y' | B | p | q |
| 1 | OMe | OMe | H | –C–<br>‖<br>O | $-CH_2-$ | 1) | –C–NH–<br>‖<br>O | 1 | 0 |
| 2 | 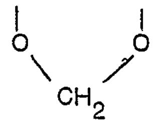 | | H | –C–<br>‖<br>O | $-CH_2-$ | 1) | –C–NH–<br>‖<br>O | 1 | 0 |
| 3 | OMe | OMe | H | –CH–<br>OH | $-CH_2-$ | 1) | –C–NH–<br>‖<br>O | 1 | 0 |
| 4 | 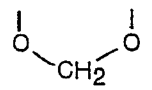 | | H | –CH–<br>OH | $-CH_2-$ | 1) | –C–NH–<br>‖<br>O | 1 | 0 |
| 5 | OMe | OMe | H | –C–<br>‖<br>O | –CH–<br>Me | 1) | –C–NH–<br>‖<br>O | 1 | 0 |
| 6 | OMe | OMe | H | –CH–<br>OH | –CH–<br>Me | 1) | –C–NH–<br>‖<br>O | 1 | 0 |
| 7 | OMe | OMe | OMe | –CH–<br>OH | $-CH_2-$ | 1) | –C–NH–<br>‖<br>O | 1 | 0 |
| 8 | OMe | OMe | H | –C–<br>‖<br>O | $-CH_2-$ | 1) | –C–NH–<br>‖<br>O | 0 | 1 |
| 9 | 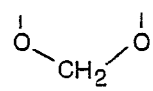 | | H | –C–<br>‖<br>O | $-CH_2-$ | 1) | –C–NH–<br>‖<br>O | 0 | 1 |
| 10 | OMe | OMe | H | –CH–<br>OH | $-CH_2-$ | 1) | –C–NH–<br>‖<br>O | 0 | 1 |
| 11 | 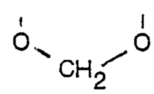 | | H | –CH–<br>OH | $-CH_2-$ | 1) | –C–NH–<br>‖<br>O | 0 | 1 |
| 12 | OMe | OMe | H | –C–<br>‖<br>O | –CH–<br>Me | 1) | –C–NH–<br>‖<br>O | 0 | 1 |

| Compound No. | Structure | | | | | | | | |
|---|---|---|---|---|---|---|---|---|---|
| | $A_1$ | $A_2$ | $A_3$ | X | $R_1$ | Y′ | B | p | q |
| 13 | OMe | OMe | H | –CH–<br>OH | –CH–<br>Me | 1) | –C–NH–<br>O | 0 | 1 |
| 14 | H | H | H | –C–<br>O | $-CH_2-$ | 1) | –C–NH–<br>O | 1 | 0 |
| 15 | Cl | H | H | –C–<br>O | $-CH_2-$ | 1) | –C–NH–<br>O | 1 | 0 |
| 16 | H | Cl | H | –C–<br>O | $-CH_2-$ | 1) | –C–NH–<br>O | 1 | 0 |
| 17 | H | OMe | H | –C–<br>O | $-CH_2-$ | 1) | –C–NH–<br>O | 1 | 0 |
| 18 | H | H | H | –CH–<br>OH | $-CH_2-$ | 1) | –C–NH–<br>O | 1 | 0 |
| 19 | Cl | H | H | –CH–<br>OH | $-CH_2-$ | 1) | –C–NH–<br>O | 1 | 0 |
| 20 | H | Cl | H | –CH–<br>OH | $-CH_2-$ | 1) | –C–NH–<br>O | 1 | 0 |
| 21 | H | OMe | H | –CH–<br>OH | $-CH_2-$ | 1) | –C–NH–<br>O | 1 | 0 |
| 22 | Cl | Cl | H | –CH–<br>OH | $-CH_2-$ | 1) | –C–NH–<br>O | 1 | 0 |
| 23 | OMe | OMe | H | –C–<br>O | $-CH_2-$ | 1) | –C–O–<br>O | 1 | 0 |
| 24 | 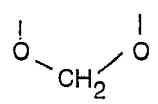 | | H | –C–<br>O | $-CH_2-$ | 1) | –C–O–<br>O | 1 | 0 |
| 25 | OMe | OMe | H | –CH–<br>OH | $-CH_2-$ | 1) | –C–O–<br>O | 1 | 0 |
| 26 | 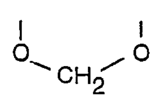 | | H | –CH–<br>OH | $-CH_2-$ | 1) | –C–O–<br>O | 1 | 0 |

| Compound No. | Structure | | | | | | | | |
|---|---|---|---|---|---|---|---|---|---|
| | A1 | A2 | A3 | X | R1 | Y' | B | p | q |
| 27 | OMe | OMe | H | –C–<br>‖<br>O | –CH–<br>Me | 1) | –C–O–<br>‖<br>O | 1 | 0 |
| 28 | OMe | OMe | H | –CH–<br>OH | –CH–<br>Me | 1) | –C–O–<br>‖<br>O | 1 | 0 |
| 29 | OMe | OMe | OMe | –C–<br>‖<br>O | $-CH_2-$ | 1) | –C–O–<br>‖<br>O | 1 | 0 |
| 30 | OMe | OMe | OMe | –CH–<br>OH | $-CH_2-$ | 1) | –C–O–<br>‖<br>O | 1 | 0 |
| 31 | 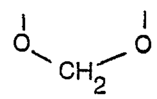 | | H | –C–<br>‖<br>O | $-CH_2-$ | 1) | –N=N– | 0 | 0 |
| 32 | OMe | OMe | H | –C–<br>‖<br>O | $-CH_2-$ | 1) | –N=N– | 0 | 0 |
| 33 | OMe | OMe | H | –C–<br>‖<br>O | –CH–<br>Me | 1) | –N=N– | 0 | 0 |
| 34 | 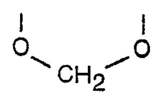 | | H | –CH–<br>OH | $-CH_2-$ | 1) | –N=N– | 0 | 0 |
| 35 | OMe | OMe | H | –CH–<br>CH | $-CH_2-$ | 1) | –N=N– | 0 | 0 |
| 36 | OMe | OMe | H | –CH–<br>OH | –CH–<br>Me | 1) | –N=N– | 0 | 0 |
| 37 | 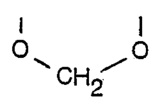 | | H | –C–<br>‖<br>O | $-CH_2-$ | 1) | –C=N–<br>Me | 0 | 0 |
| 38 | OMe | OMe | H | –C–<br>‖<br>O | $-CH_2-$ | 1) | –C=N–<br>Me | 0 | 0 |
| 39 | OMe | OMe | H | –C–<br>‖<br>O | –CH–<br>Me | 1) | –C=N–<br>Me | 0 | 0 |

| Compound No. | Structure | | | | | | | |
|---|---|---|---|---|---|---|---|---|
| | A1 | A2 | A3 | X | R1 | Y′ | B | p | q |
| 40 | 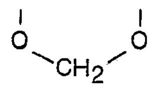 | | H | −CH−<br>OH | $-CH_2-$ | 1) | −C=N−<br>Me | 0 | 0 |
| 41 | OMe | OMe | H | −CH−<br>OH | $-CH_2-$ | 1) | −C=N−<br>Me | 0 | 0 |
| 42 | OMe | OMe | H | −CH−<br>OH | −CH−<br>Me | 1) | −C=N−<br>Me | 0 | 0 |
| 43 | 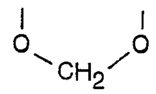 | | H | −C−<br>O | $-CH_2-$ | 1) | −C−NH−<br>N−CN | 1 | 0 |
| 44 | OMe | OMe | H | −C−<br>O | $-CH_2-$ | 1) | −C−NH−<br>N−CN | 1 | 0 |
| 45 | OMe | OMe | H | −C−<br>O | −CH−<br>Me | 1) | −C−NH−<br>N−CN | 1 | 0 |
| 46 | OMe | OMe | OMe | −C−<br>O | $-CH_2-$ | 1) | −C−NH−<br>N−CN | 1 | 0 |
| 47 | OMe | OMe | OMe | −C−<br>O | −CH−<br>Me | 1) | −C−NH−<br>N−CN | 1 | 0 |
| 48 | 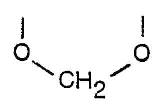 | | H | −C−<br>O | $-CH_2-$ | 1) | −C−NH−<br>N−CN | 0 | 0 |
| 49 | OMe | OMe | H | −C−<br>O | $-CH_2-$ | 1) | −C−NH−<br>N−CN | 0 | 0 |
| 50 | 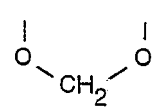 | | H | −CH−<br>OH | $-CH_2-$ | 1) | −C−NH−<br>N−CN | 1 | 0 |
| 51 | OMe | OMe | H | −CH−<br>OH | $-CH_2-$ | 1) | −C−NH−<br>N−CN | 1 | 0 |
| 52 | OMe | OMe | H | −CH−<br>OH | −CH−<br>Me | 1) | −C−NH−<br>N−CN | 1 | 0 |

| Compound No. | Structure | | | | | | | | |
|---|---|---|---|---|---|---|---|---|---|
| | $A_1$ | $A_2$ | $A_3$ | X | $R_1$ | Y′ | B | p | q |
| 53 | OMe | OMe | OMe | –CH–<br>OH | –$CH_2$– | 1) | –C–NH–<br>N–CN | 1 | 0 |
| 54 | OMe | OMe | OMe | –CH–<br>OH | –CH–<br>Me | 1) | –C–NH–<br>N–CN | 1 | 0 |
| 55 | 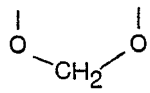 | | H | –CH–<br>OH | –$CH_2$– | 1) | –C–NH–<br>N–CN | 0 | 0 |
| 56 | OMe | OMe | H | –CH–<br>OH | –$CH_2$– | 1) | –C–NH–<br>N–CN | 0 | 0 |
| 57 | OMe | OMe | H | –C–<br>O | –$CH_2$– | 1) | –S–NH–<br>$O_2$ | 1 | 0 |
| 58 | OMe | OMe | H | –CH–<br>OH | –$CH_2$– | 1) | –S–NH–<br>$O_2$ | 1 | 0 |
| 59 | 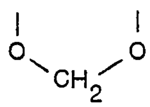 | | H | –C–<br>O | –$CH_2$– | 1) | –S–NH–<br>$O_2$ | 1 | 0 |
| 60 | 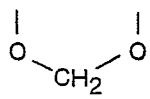 | | H | –CH–<br>OH | –$CH_2$– | 1) | –S–NH–<br>$O_2$ | 1 | 0 |
| 61 | OMe | OMe | H | –C–<br>O | –$CH_2$– | 1) | –S–NH–<br>$O_2$ | 0 | 1 |
| 62 | 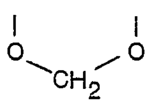 | | H | –C–<br>O | –$CH_2$– | 1) | –S–NH–<br>$O_2$ | 0 | 1 |
| 63 | OMe | OMe | H | –CH–<br>OH | –$CH_2$– | 1) | –S–NH–<br>$O_2$ | 0 | 1 |
| 64 | 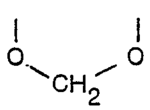 | | H | –CH–<br>OH | –$CH_2$– | 1) | –S–NH–<br>$O_2$ | 0 | 1 |
| 65 | OMe | OMe | H | –C–<br>O | –CH–<br>Me | 1) | –S–NH–<br>$O_2$ | 0 | 1 |

| Compound No. | Structure | | | | | | | | |
|---|---|---|---|---|---|---|---|---|---|
| | $A_1$ | $A_2$ | $A_3$ | X | $R_1$ | Y′ | B | p | q |
| 66 | \|<br>OMe | \|<br>OMe | H | −CH−<br>\|<br>OH | −CH−<br>\|<br>Me | 1) | −S−NH−<br>$O_2$ | 0 | 1 |
| 67 | 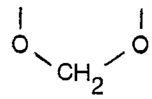 | | H | −C−<br>‖<br>O | $-CH_2-$ | 3)<br>single<br>bond | − | − | − |
| 68 | \|<br>OMe | \|<br>OMe | H | −C−<br>‖<br>O | $-CH_2-$ | 3)<br>single<br>bond | − | − | − |
| 69 | \|<br>OMe | \|<br>OMe | H | −C−<br>‖<br>O | −CH−<br>‖<br>Me | 3)<br>single<br>bond | − | − | − |
| 70 | 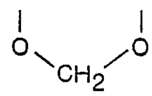 | | H | −C−<br>‖<br>O | $-CH_2-$ | 3)<br>double<br>bond | − | − | − |
| 71 | \|<br>OMe | \|<br>OMe | H | −C−<br>‖<br>O | $-CH_2-$ | 3)<br>double<br>bond | − | − | − |
| 72 | 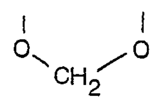 | | H | −CH−<br>‖<br>OH | $-CH_2-$ | 3)<br>single<br>bond | − | − | − |
| 73 | \|<br>OMe | \|<br>OMe | H | −CH−<br>\|<br>OH | $-CH_2-$ | 3)<br>single<br>bond | − | − | − |
| 74 | \|<br>OMe | \|<br>OMe | H | −CH−<br>\|<br>OH | −CH−<br>\|<br>Me | 3)<br>single<br>bond | − | − | − |
| 75 | 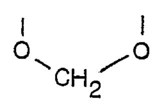 | | H | −CH−<br>\|<br>OH | $-CH_2-$ | 3)<br>double<br>bond | − | − | − |
| 76 | \|<br>OMe | \|<br>OMe | H | −CH−<br>\|<br>OH | $-CH_2-$ | 3)<br>double<br>bond | − | − | − |
| 77 | \|<br>OMe | \|<br>OMe | H | −C−<br>‖<br>O | $-CH_2-$ | 2) | − | − | − |
| 78 | \|<br>OMe | \|<br>OMe | H | −C−<br>‖<br>O | −CH−<br>\|<br>Me | 2) | − | − | − |

| Compound No. | Structure | | | | | | | | |
|---|---|---|---|---|---|---|---|---|---|
| | $A_1$ | $A_2$ | $A_3$ | X | $R_1$ | Y' | B | p | q |
| 79 | \|<br>OMe | \|<br>OMe | \|<br>OMe | –C–<br>‖<br>O | –CH–<br>\|<br>Me | 2) | – | – | – |
| 80 | \|<br>OMe | \|<br>OMe | \|<br>OMe | –C–<br>‖<br>O | $-CH_2-$ | 2) | – | – | – |
| 81 | \|<br>OMe | \|<br>OMe | H | –CH–<br>\|<br>OH | $-CH_2-$ | 2) | – | – | – |
| 82 | \|<br>OMe | \|<br>OMe | H | –CH–<br>\|<br>OH | –CH–<br>\|<br>Me | 2) | – | – | – |
| 83 | \|<br>OMe | \|<br>OMe | \|<br>OMe | –CH–<br>\|<br>OH | –CH–<br>\|<br>Me | 2) | – | – | – |
| 84 | \|<br>OMe | \|<br>OMe | \|<br>OMe | –CH–<br>\|<br>OH | $-CH_2-$ | 2) | – | – | – |

TABLE 3

Properties (melting point, IR, NMR and Elementary analysis)

(1) The term "form" means the state of a compound subjected to the determination of properties.

| | | |
|---|---|---|
| Blank | : | free base |
| $H_2O$ | : | water of crystallization |
| EtOH | : | ethanol of crystallization |
| HCl | : | hydrochloride |

(2) The values in the column of infrared absorption spectrum (IR) show characteristic maximum absorption of the compounds measured in KBr tablet, except of Compound Nos. 39 and 68 where the measurement was conducted by using chloroform.

(3) The values in the table of nuclear magnetic resonance spectrum (NMR) and $\delta$ values based on TMS in $CDCl_3$ (Compound Nos. 1—6, 8—14, 17, 18, 23—30, 33—39, 43—49, 51, 53, 54, 56—58, 61, 63, 69, 76, 77, 80, 81 and 83, $d_6$-dimethylsulfoxide ($d_6$-DMSO) (Compound Nos. 7, 15, 16, 19—22, 31, 32, 40—42, 50, 59, 60, 62, 64, 67, 68, 70—75, 78, 79, 82 and 84), $d_6$-DMSO + $CD_3OD$ (Compound No. 52), $CDCl_3$ + $CD_3OD$ (Compound No. 55), or $CD_3OD$ + $D_2O$ (Compound Nos. 65 and 66)

(4) Elementary analysis
A: Calculated
F: Found

TABLE 3—1

| Compound No. | Form | m.p. (°C) | IR ($cm^{-1}$) |
|---|---|---|---|
| 1 | | 161—163.5 | 1680, 1665 |
| 2 | | 187—189.5 | 1685, 1668 |
| 3 | | 196.5—197.1 | 1670 |
| 4 | | 229.5—231.5 | 1668 |
| 5 | | 173.0—174.5 | 1680—1660 |
| 6 | 1/2 $H_2O$ | 220—223.0 | 1665 |
| 7 | | 236.0—238.5 | 1660 |
| 8 | 1/2 EtOH | 146.0—148.0 | 1685—1670 |
| 9 | 1/2 EtOH | 171.0—173.0 | 1660—1690 |
| 10 | | 173.5—175.0 | 1668 |
| 11 | | 193.0—194.5 | 1675 |
| 12 | | 154.0—156.0 | 1660—1690 |
| 13 | | 243.0—244.0 | 1670 |
| 14 | | 171.0—172.0 | 1680—1665 |
| 15 | | 172.0—174.5 | 1685, 1660 |
| 16 | | 193.0—195.5 | 1690, 1660 |
| 17 | 1/4 $H_2O$ | 199.0—201.0 | 1680, 1650 |
| 18 | | 219.5—221.0 | 1665 |
| 19 | | 227.5—229.5 | 1660 |
| 20 | | 240.0—241.0 | 1665 |
| 21 | | 233.0—235.0 | 1655 |
| 22 | | 241.8—242.3 | 1660 |
| 23 | | 143,0—144.5 | 1680, 1715 1735 |
| 24 | | 163.0—164.0 | 1680, 1710 1720(sh) |
| 25 | | 169.0—170.0 | 1715 |
| 26 | | 185.7—186.2 | 1715 |
| 27 | | 149.0—151.5 | 1715, 1720(sh) 1680 |
| 28 | | 201.0—202.0 | 1700 |

TABLE 3—1 (continued)

| Compound No. | Form | m.p. (°C) | IR ($cm^{-1}$) |
|---|---|---|---|
| 29 | | 152.0—153.5 | 1690, 1720 |
| 30 | | 203.0—204.0 | 1705 |
| 31 | | 144.5—146.0 | 1675, 1455, 1440, 1261, 1040 |
| 32 | monofumarate | 184—186 | 1685, 1278, 1158 |
| 33 | | 174.5—177 | 1665, 1260, 1164, 1125 |
| 34 | | 148—149 | 1493, 1255, 1245, 1040 |
| 35 | | 173—174 | 3370, 1265, 1242, 1150 |
| 36 | | 222—224 | 3400, 1504, 1255, 1140—1138 |
| 37 | | 174—177 | 1691, 1255 |
| 38 | | 153—155 | 1673, 1255 |
| 39 | | oil | 1667, 1256 |
| 40 | difumarate | 167—169 | 1690, 1235 |
| 41 | difumarate | 214—215.2 | 1695, 1235 |
| 42 | difumarate | 179—181 | 1685, 1255 |
| 43 | | 200—202 | 2300, 1678, 1623, 1588 |
| 44 | | 202—204 | 2320, 1685, 1628, 1590 |
| 45 | | 195—198 | 2300, 1660 1628, 1588 |
| 46 | | 185—187 | 2300, 1688—1678, 1624, 1588 |
| 47 | | 193—195 | 2300, 1670, 1624, 1585 |
| 48 | | 196—198 | 2300, 1674, 1625, 1601 |
| 49 | | 137—139 | 2300, 1680, 1626, 1601 |

TABLE 3—1 (continued)

| Compound No. | Form | m.p. (°C) | IR ($cm^{-1}$) |
|---|---|---|---|
| 50 | | 239—241 | 2300, 1630, 1590 |
| 51 | | 249—251 | 2300, 1630, 1589 |
| 52 | | 247—249 | 2301, 1628, 1590 |
| 53 | | 218—220 | 2300, 1628, 1589 |
| 54 | | 252—254 | 2301, 1628, 1589 |
| 55 | | 232—233.5 | 2300, 1629 1601 |
| 56 | | 218—219 | 2300, 1628, 1601 |
| 57 | | 173.5—175.5 | 1670, 1345, 1170 |
| 58 | | 171.0—172.5 | 1350, 1340, 1170 |
| 59 | | 184.0—185.5 | 1680, 1342, 1170 |
| 60 | | 178.0—179.0 | 1350, 1340, 1170 |
| 61 | 3/2 EtOH | 102.0—104.0 | 1680, 1350, 1340, 1170 |
| 62 | | 185.0—187.0 | 1685, 1335, 1165 |
| 63 | | 144.5—146.0 | 1350, 1340, 1180 |
| 64 | | 194.5—195.0 | 1345, 1175 |
| 65 | HCl·EtOH | 229.0—231.5 | 1660, 1348, 1332, 1160 |
| 66 | HCl | 221.0—222.0 | 1350(sh), 1342, 1165 |
| 67 | | 158—165 | 1695—1692 1673, 1243 |
| 68 | | oil | 1695 |
| 69 | | 174—176 | 1690, 1660—1650 1264 |

TABLE 3—1 (continued)

| Compound No. | Form | m.p. (°C) | IR ($cm^{-1}$) |
|---|---|---|---|
| 70 | | 167.5—171 | 1698, 1680, 1255 |
| 71 | | 145—146 | 1690, 1670, 1270 |
| 72 | | 177—178 | 1700 |
| 73 | succinate | 166—167 | 1703 |
| 74 | fumarate | 148—152 | 1690, 1250 |
| 75 | | 176—178 | 1692—1245 |
| 76 | | 98—99 | 1700, 1690, 1473, 1240 |
| 77 | | 179—181 | 1682 |
| 78 | fumarate | 194—196.5 | 1680 |
| 79 | fumarate | 197—199 | 1680 |
| 80 | | 183—187 | 1682 |
| 81 | | 171—172 | 1689—1680 |
| 82 | fumarate | 208.5—209.5 | 1700—1675 |
| 83 | | 123—126 | 1700—1680 |
| 84 | fumarate | 153—155 | 1685—1675 |

TABLE 3—2

| Compound No. | Form | NMR (ppm) |
|---|---|---|
| 1 | | 1.4—2.6, 2.8—3.3, 3.8, 3.95, 4.35, 4.3—4.7, 6.6—7.4, 7.5—7.8, 8.07 |
| 2 | | 1.4—2.6, 2.75—3.4, 3.75, 4.35, 4.0—4.8, 6.05, 6.5—7.35, 7.35—7.80, 8.1 |
| 3 | | 1.55—3.7, 3.90, 3.93, 4.40, 4.45—5.0, 6.55—7.8 |
| 4 | | 1.5—4.0, 4.35, 4.2—4.8, 5.93, 6.50—7.4, 7.85 |
| 5 | | 1.3(d), 1.46—3.3, 3.95, 4.3, 4.0—4.7, 6.6—7.35, 7.55—7.95, 8.25 |
| 6 | 1/2 $H_2O$ | 0.8(d), 1.45—3.35, 3.87, 3.90, 4.0—4.8, 4.4, 6.6—7.4, 8.05 |
| 7 | | 1.27—2.40, 2.50(d), 2.83—3.30, 3.70, 3.83, 4.33, 6.53—7.40, 9.17 |
| 8 | 1/2 EtOH | 1.55—3.35, 3.80, 3.93, 4.30, 5.65, 6.7—7.9 |
| 9 | 1/2 EtOH | 1.46—3.50, 3.76, 4.26, 6.03, 6.76—7.75 |
| 10 | | 1.53—3.64, 3.90, 3.93, 4.3, 4.75(trip), 3.64—4.4, 5.65, 6.8—7.6 |
| 11 | | 1.53—3.50, 3.50—4.15, 4.27, 4.65(trip), 5.70, 5.90, 6.56—7.46 |
| 12 | | 1.30(d), 1.53—3.33, 3.97, 4.27, 5.90, 6.7—8.0 |
| 13 | | 0.80(d), 1.7—3.3, 3.87, 3.90, 4.0—4.37, 5.63, 6.7—7.3 |
| 14 | | 1.43—2.53, 2.73—3.27, 3.73, 4.27, 4.1—4.7, 6.6—8.1, 8.17 |
| 15 | | 1.20—2.60, 2.80—3.27, 3.85, 4.20(broad), 4.30, 6.70—8.00, 9.15 |
| 16 | | 1.25—2.47, 2.53—3.10, 3.85, 4.2(broad), 4.33, 6.63—8.17, 9.20 |
| 17 | 1/4 $H_2O$ | 1.47—2.70, 2.93—3.37, 3.80, 3.87, 4.37, 6.7—8.1 |
| 18 | | 1.50—3.50, 4.37, 4.20—4.90, 6.60—7.50, 7.73 |

TABLE 3—2 (continued)

| Compound No. | Form | NMR (ppm) |
|---|---|---|
| 19 | | 1.20—2.30, 2.47(d), 2.80—3.20, 3.80—4.50, 4.30, 4.50—4.90, 5.07, 6.63—7.43, 9.20 |
| 20 | | 1.10—2.30, 2.47(d), 2.80—3.20, 3.80—4.50, 4.30, 4.50—4.90, 5.07, 6.63—7.50, 7.43, 9.20 |
| 21 | | 1.20—2.30, 2.43(d), 2.80—3.20, 3.75, 3.80—4.50, 4.30, 4.50—5.03, 6.65—7.45, 10.17 |
| 22 | | 1.20—2.27, 2.47(d), 2.80—3.27, 3.80—4.53, 4.30, 4.53—4.97, 5.22, 6.65—7.80, 9.15 |
| 23 | | 1.5—2.6, 2.9—3.4, 3.80, 3.93, 4.40, 6.8—7.8 |
| 24 | | 1.6—2.6, 2.9—3.4, 3.73, 4.37, 6.03, 6.73—7.70 |
| 25 | | 1.6—3.50, 2.50(d), 3.83, 3.86, 4.36, 4.13—4.83, 6.70—7.46 |
| 26 | | 1.6—4.1, 4.1—4.83, 4.40, 5.93, 6.60—7.50 |
| 27 | | 1.30(d), 1.50—3.30, 3.96, 4.35, 3.70—4.60, 6.80—7.90 |
| 28 | | 0.78(d), 1.60—3.30, 3.86, 3.90, 4.41, 3.60—4.60, 6.66—7.46 |
| 29 | | 1.30—2.63, 2.83—3.36, 3.86, 3.93, 4.40, 4.1—4.6, 6.9—7.5 |
| 30 | | 1.50—3.60, 2.53(d), 3.83, 3.86, 4.40, 4.06—4.86, 6.60, 6.80—7.46 |
| 31 | | 1.5—3.4, 3.33, 3.6—4.0, 4.0—4.4, 4.6—5.1, 6.13, 6.8—8.2 |
| 32 | monofumarate | 1.5—3.5, 3.85, 4.08, 4.6—5.2 6.9—8.2 |
| 33 | | 1.33, 1.9—3.5, 3.95, 4.0—5.0, 6.8—8.2 |
| 34 | | 1.6—4.0, 4.4—5.0, 5.92, 6.6—8.3 |
| 35 | | 2.0—4.0, 3.88, 3.95, 4.4—5.0, 6.65—8.3 |
| 36 | | 0.8, 2.0—3.4, 3.85, 4.25, 4.4—5.2, 6.68—8.3 |

TABLE 3—2 (continued)

| Compound No. | Form | NMR (ppm) |
|---|---|---|
| 37 | | 1.6—3.4, 2.6, 3.4—4.6, 3.8, 6.02, 6.7—7.9 |
| 38 | | 1.65—3.4, 2.63, 3.6—4.6, 3.88, 3.98, 6.7—7.9 |
| 39 | | 1.33, 1.4—3.4, 2.60, 3.6—4.6, 3.97, 6.70—8.0 |
| 40 | difumarate | 1.6—4.0, 2.63, 4.2—5.2, 6.03, 6.7—8.05 |
| 41 | difumarate | 1.7—4.0, 2.63, 3.75, 3.80, 4.2—5.2, 6.8—8.1 |
| 42 | difumarate | 0.81, 1.6—4.0, 2.6, 3.76, 3.80, 4.2—4.7, 6.8—8.1 |
| 43 | | 1.4—3.4, 3.76, 4.2—4.9, 4.4, 6.05, 6.7—7.8, 8.65 |
| 44 | | 1.4—3.4, 3.79, 3.95, 4.2—4.8, 4.4, 6.7—7.8, 8.74 |
| 45 | | 1.25, 1.4—3.4, 3.94, 4.0—4.6, 4.34, 6.8—7.9, 8.4 |
| 46 | | 1.4—3.4, 3.78, 3.88, 4.2—4.8, 4.36, 6.8—7.2, 8.95 |
| 47 | | 1.3, 1.4—3.4, 3.95, 4.0—4.6, 4.38, 6.8—7.6, 8.62 |
| 48 | | 1.4—4.2, 4.3—4.9, 6.2, 6.9—8.0 |
| 49 | | 1.5—4.1, 3.9, 4.0, 4.3—4.9, 6.8—8.0 |
| 50 | | 1.3—3.4, 4.0—5.0, 4.39, 5.85, 6.6—7.4, 9.92 |
| 51 | | 1.4—4.0, 3.85, 3.90, 6.6—7.4, 8.90 |
| 52 | | 0.75, 1.4—3.4, 3.80, 3.90, 4.1—5.0, 4.1—5.0, 6.6—7.4 |
| 53 | | 1.4—4.0, 3.8, 3.85, 4.1—4.8, 4.35, 6.59, 6.8—7.33, 8.96 |
| 54 | | 0.8, 1.4—4.0, 3.8, 3.85, 4.0—4.8, 4.41, 6.6, 6.8—7.33, 9.00 |
| 55 | | 1.7—5.0, 6.0, 6.8—7.8 |
| 56 | | 1.6—5.0, 3.88, 3.91, 6.8—7.8 |
| 57 | | 1.50—2.50, 2.80—3.30, 3.75, 3.90 3.93, 4.67, 6.20—7.75 |

TABLE 3—2 (continued)

| Compound No. | Form | NMR (ppm) |
|---|---|---|
| 58 | | 1.43—2.0, 2.10—3.33, 3.50—4.10, 3.86, 4.36—5.20, 4.70, 6.50—7.36 |
| 59 | | 1.30—3.10, 3.20—3.90, 3.67, 4.66, 6.10, 6.6—7.7 |
| 60 | | 1.30—2.43, 2.66—3.13, 3.20—3.76, 4.36—4.80, 5.93, 6.56—7.30 |
| 61 | 3/2 EtOH | 1.53—2.60, 2.76—3.40, 3.75, 3.90, 3.93, 4.46, 5.13(broad) 6.73—7.73 |
| 62 | | 1.53—2.40, 2.70—3.40, 3.53—4.13, 3.73, 4.36, 6.12, 6.90—7.76 |
| 63 | | 1.70—3.33, 3.81, 3.85, 3.90—4.76, 4.41, 6.63—7.50 |
| 64 | | 1.46—2.60, 2.76—3.46, 3.60—4.13, 4.20—5.10, 4.40, 5.96, 6.60—7.80 |
| 65 | Hcl·EtOH | 1.50(d), 2.10—2.80, 3.06—3.70, 3.96, 4.0, 4.56, 5.28(quartet), 7.10—7.93 |
| 66 | HCl | 1.25(d), 2.12—2.75, 3.05—4.18, 3.89, 4.56,5.43(broad), 6.92—7.47 |
| 67 | | 1.0—3.5, 3.62, 6.1, 6.7—7.8, 10.26 |
| 68 | | 1.0—3.5, 3.65, 3.8, 3.85, 6.7—7.8, 10.26 |
| 69 | | 1.0—3.5, 1.23, 3.8—4.2, 6.7—7.2, 7.6—8.0, 9.1 |
| 70 | | 2.3—3.6, 3.8, 6.1, 6.7—7.8, 10.32 |
| 71 | | 2.4—3.6, 3.3, 3.82, 6.7—7.85, 10.3 |
| 72 | | 1.0—3.4, 4.4—4.8, 5.92, 6.6—7.4, 10.21 |
| 73 | succinate | 1.2—3.5, 3.75, 4.5—4.95, 6.67—7.5, 10.25 |
| 74 | fumarate | 1.0, 1.2—3.5, 3.75, 4.1—4.4, 6.6—7.4, 10.3 |
| 75 | | 2.3—3.2, 3.2—3.6, 4.4—5.15, 5.95, 6.6—7.7, 10.35 |
| 76 | | 2.3—3.4, 3.4—4.0, 3.93, 4.4—5.0, 6.68—7.75, 8.65 |
| 77 | | 1.0—4.0, 3.77, 3.95, 4.95, 6.77—7.8 |

TABLE 3—2 (continued)

| Compound No. | Form | NMR (ppm) |
|---|---|---|
| 78 | fumarate | 1.0—4.0, 1.12, 3.8, 6.9—7.88 |
| 79 | fumarate | 1.0—4.0, 1.18, 3.8, 3.85, 7.5 |
| 80 | | 1.0—4.0, 3.8, 3.9, 7.42 |
| 81 | | 1.0—4.0, 3.85, 3.90, 4.4—5.0, 6.65—7.07 |
| 82 | fumarate | 0.73, 1.0—3.3, 3.75, 4.3, 6.7—7.08 |
| 83 | | 0.75, 1.0—3.2, 3.82, 3.85, 4.12, 4.88, 6.59 |
| 84 | fumarate | 1.0—4.0, 3.9, 6.5—7.0 |

TABLE 3—3

A: Calculated, F: Found

| Compound No. | Rational formula | | Elementary analysis (%) C | H | N |
|---|---|---|---|---|---|
| 1 | $C_{23}H_{27}N_3O_4$ | A<br>F | 67.46<br>67.38 | 6.65<br>6.84 | 10.26<br>10.18 |
| 2 | $C_{22}H_{23}N_3O_4$ | A<br>F | 67.16<br>67.15 | 5.89<br>5.84 | 10.68<br>10.54 |
| 3 | $C_{23}H_{29}N_3O_4$ | A<br>F | 67.13<br>66.96 | 7.10<br>7.13 | 10.21<br>10.17 |
| 4 | $C_{22}H_{25}N_3O_4$ | A<br>F | 66.82<br>66.56 | 6.37<br>6.27 | 10.63<br>10.51 |
| 5 | $C_{24}H_{29}N_3O_4$ | A<br>F | 68.07<br>67.91 | 6.90<br>6.84 | 9.92<br>9.90 |
| 6 | $C_{24}H_{31}N_3O_4 \cdot 1/2H_2O$ | A<br>F | 66.34<br>66.48 | 7.42<br>7.54 | 9.67<br>9.36 |
| 7 | $C_{24}H_{31}N_3O_5$ | A<br>F | 65.29<br>65.17 | 7.08<br>7.07 | 9.52<br>9.41 |
| 8 | $C_{23}H_{27}N_3O_4 \cdot 1/2EtOH$ | A<br>F | 66.65<br>66.72 | 6.99<br>6.65 | 9.72<br>9.80 |
| 9 | $C_{22}H_{23}N_3O_4 \cdot 1/2EtOH$ | A<br>F | 66.33<br>66.46 | 6.29<br>6.08 | 10.09<br>10.29 |
| 10 | $C_{23}H_{29}N_3O_4$ | A<br>F | 67.13<br>66.85 | 7.10<br>7.25 | 10.21<br>10.01 |

TABLE 3—3 (continued)

| Compound No. | Rational formula | | Elementary analysis (%) C | H | N |
|---|---|---|---|---|---|
| 11 | $C_{22}H_{25}N_3O_4$ | A<br>F | 66.82<br>66.80 | 6.37<br>6.41 | 10.63<br>10.51 |
| 12 | $C_{24}H_{29}N_3O_4$ | A<br>F | 68.07<br>67.96 | 6.90<br>6.97 | 9.92<br>9.76 |
| 13 | $C_{24}H_{31}N_3O_4$ | A<br>F | 67.74<br>67.60 | 7.34<br>7.39 | 9.87<br>9.71 |
| 14 | $C_{21}H_{23}N_3O_2$ | A<br>F | 72.18<br>71.95 | 6.63<br>6.64 | 12.03<br>11.90 |
| 15 | $C_{21}H_{22}N_3O_2Cl$ | A<br>F | 65.71<br>65.83 | 5.78<br>5.64 | 10.95<br>10.75 |
| 16 | $C_{21}H_{22}N_3O_2Cl$ | A<br>F | 65.71<br>65.44 | 5.78<br>5.74 | 10.95<br>10.86 |
| 17 | $C_{22}H_{25}N_3O_3 \cdot 1/4H_2O$ | A<br>F | 68.82<br>68.78 | 6.70<br>6.61 | 10.94<br>10.83 |
| 18 | $C_{21}H_{25}N_3O_2$ | A<br>F | 71.77<br>71.54 | 7.17<br>7.24 | 11.96<br>11.76 |
| 19 | $C_{21}H_{24}N_3O_2Cl$ | A<br>F | 65.36<br>65.44 | 6.27<br>6.27 | 10.89<br>10.62 |
| 20 | $C_{21}H_{24}N_3O_2Cl$ | A<br>F | 65.36<br>65.09 | 6.27<br>6.26 | 10.89<br>10.93 |
| 21 | $C_{22}H_{27}N_3O_3$ | A<br>F | 69.27<br>69.11 | 7.13<br>7.14 | 11.02<br>11.18 |
| 22 | $C_{21}H_{23}N_3O_2Cl_2$ | A<br>F | 60.01<br>59.88 | 5.51<br>5.46 | 10.00<br>9.73 |
| 23 | $C_{23}H_{26}N_2O_5$ | A<br>F | 67.30<br>67.11 | 6.39<br>6.51 | 6.82<br>6.64 |
| 24 | $C_{22}H_{22}N_2O_5$ | A<br>F | 66.99<br>67.05 | 5.62<br>5.59 | 7.10<br>6.96 |
| 25 | $C_{23}H_{28}N_2O_5$ | A<br>F | 66.97<br>66.97 | 6.84<br>6.87 | 6.79<br>6.77 |
| 26 | $C_{22}H_{24}N_2O_5$ | A<br>F | 66.65<br>66.61 | 6.10<br>6.01 | 7.07<br>7.04 |
| 27 | $C_{24}H_{28}N_2O_5$ | A<br>F | 67.91<br>67.73 | 6.65<br>6.76 | 6.60<br>6.40 |
| 28 | $C_{24}H_{30}N_2O_5$ | A<br>F | 67.59<br>67.67 | 7.09<br>7.27 | 6.57<br>6.52 |
| 29 | $C_{24}H_{28}N_2O_6$ | A<br>F | 65.44<br>65.62 | 6.41<br>6.51 | 6.36<br>6.23 |

TABLE 3—3 (continued)

| Compound No. | Rational formula | | Elementary analysis (%) C | H | N |
|---|---|---|---|---|---|
| 30 | $C_{24}H_{30}N_2O_6$ | A<br>F | 65.14<br>65.30 | 6.83<br>7.00 | 6.33<br>6.26 |
| 31 | $C_{20}H_{20}N_4O_3$ | A<br>F | 65.92<br>65.86 | 5.53<br>5.52 | 15.38<br>15.68 |
| 34 | $C_{20}H_{22}N_4O_3$ | A<br>F | 65.55<br>65.32 | 6.05<br>6.04 | 15.29<br>15.44 |
| 38 | $C_{23}H_{27}N_3O_3$ | A<br>F | 70.20<br>70.22 | 6.92<br>6.99 | 10.68<br>10.56 |
| 41 | $C_{23}H_{29}N_3O_3 \cdot C_8H_8O_8$ | A<br>F | 59.32<br>59.11 | 5.94<br>5.94 | 6.69<br>6.66 |
| 43 | $C_{23}H_{23}N_5O_3$ | A<br>F | 66.17<br>66.09 | 5.55<br>5.43 | 16.78<br>16.63 |
| 47 | $C_{26}H_{31}N_5O_4$ | A<br>F | 65.39<br>65.31 | 6.54<br>6.58 | 14.67<br>14.42 |
| 48 | $C_{22}H_{21}N_5O_3$ | A<br>F | 65.49<br>65.22 | 5.25<br>5.34 | 17.36<br>17.35 |
| 50 | $C_{23}H_{25}N_5O_3$ | A<br>F | 65.85<br>65.77 | 6.01<br>6.02 | 16.70<br>16.61 |
| 55 | $C_{22}H_{21}N_5O_3$ | A<br>F | 65.49<br>65.22 | 5.25<br>5.34 | 17.36<br>17.35 |
| 57 | $C_{22}H_{27}N_3O_5S$ | A<br>F | 59.31<br>59.45 | 6.11<br>6.14 | 9.43<br>9.17 |
| 58 | $C_{22}H_{29}N_3O_5S$ | A<br>F | 59.04<br>59.31 | 5.53<br>6.63 | 9.39<br>9.09 |
| 59 | $C_{21}H_{23}N_3O_5S$ | A<br>F | 58.59<br>58.87 | 5.62<br>5.38 | 9.76<br>9.49 |
| 60 | $C_{21}H_{25}N_3O_5S$ | A<br>F | 58.32<br>58.58 | 6.06<br>5.94 | 9.72<br>9.57 |
| 61 | $C_{21}H_{27}N_3O_5S.3/2E_tOH$ | A<br>F | 58.35<br>58.61 | 7.05<br>6.83 | 8.16<br>8.12 |
| 62 | $C_{21}H_{23}N_3O_5S$ | A<br>F | 58.59<br>58.81 | 5.62<br>5.39 | 9.76<br>9.58 |
| 63 | $C_{22}H_{29}N_3O_5S$ | A<br>F | 59.04<br>59.31 | 6.53<br>6.58 | 9.39<br>9.31 |
| 64 | $C_{21}H_{25}N_3O_5S$ | A<br>F | 58.32<br>58.08 | 6.06<br>5.86 | 9.72<br>9.33 |
| 65 | $C_{23}H_{29}N_3O_5S \cdot HCl \cdot E_tOH$ | A<br>F | 55.39<br>55.19 | 6.69<br>6.57 | 7.75<br>8.05 |

TABLE 3—3 (continued)

| Compound No. | Rational formula | | Elementary analysis (%) C | H | N |
|---|---|---|---|---|---|
| 66 | $C_{23}H_{31}N_3O_5S \cdot HCl$ | A<br>F | 55.47<br>55.50 | 6.48<br>6.70 | 8.44<br>8.18 |
| 67 | $C_{22}H_{22}N_2O_4$ | A<br>F | 69.82<br>69.73 | 5.86<br>5.77 | 7.40<br>7.40 |
| 69 | $C_{24}H_{28}N_2O_4$ | A<br>F | 70.56<br>70.33 | 6.91<br>7.12 | 6.86<br>6.85 |
| 70 | $C_{22}H_{20}N_2O_4$ | A<br>F | 70.20<br>69.96 | 5.36<br>5.29 | 7.44<br>7.33 |
| 72 | $C_{22}H_{24}N_2O_4$ | A<br>F | 69.45<br>69.30 | 6.36<br>6.37 | 7.36<br>7.07 |
| 74 | $C_{28}H_{34}N_2O_8$ | A<br>F | 63.86<br>63.77 | 6.51<br>6.63 | 5.32<br>5.30 |
| 75 | $C_{22}H_{22}N_2O_4$ | A<br>F | 69.82<br>69.67 | 5.86<br>5.84 | 7.40<br>7.33 |
| 77 | $C_{22}H_{31}N_3O_4$ | A<br>F | 65.81<br>65.75 | 7.78<br>7.91 | 10.47<br>10.20 |
| 81 | $C_{22}H_{33}N_3O_4$ | A<br>F | 65.48<br>65.31 | 8.24<br>8.43 | 10.41<br>10.41 |

Hypotensive activity and acute toxicity of Compound [I] are illustrated below as experiments.

Experiment 1

This experiment is conducted according to the method described in "Spontaneously Hypertensive Rats (SHR) Guidelines for Breeding, Care and Use" (published by SHR Conference) (1976) p. 11.

Five spontaneously hypertensive rats (15 weeks old, 180 mmHg or more in blood pressure) are used as one group. Each of the test compound is added to 0.3% (w/v) CMC aqueous solution in a concentration of 3 mg/ml. Each of the mixtures is orally administered to the rats in a dose 1 mg/100 g. Changes in blood pressure are measured according to the method of tail artery plethysmography (see the literature cited above). The maximum reduction (mmHg) in blood pressure after the administration on the basis of the pressure immediately before the administration is shown in Table 4.

TABLE 4

| Compound No. | Maximum reduction in blood pressure (mmHg) | Compound No. | Maximum reduction in blood pressure (mmHg) |
|---|---|---|---|
| 1 | 29 | 26 | 30[*1] 45[*2] |
| 2 | 40 | 27 | 3 |
| 3 | 32 | 28 | 17 |
| 4 | 41 | 29 | 13 |
| 5 | 22 | 30 | 12 |
| 6 | 24 | 31 | 15 |
| 7 | 28 | 32 | 19 |
| 8 | 17 | 33 | 22 |
| 9 | 17 | 34 | 23 |
| 10 | 10 | 35 | 28 |
| 11 | 18 | 36 | 30 |
| 12 | 26 | 37 | 21[*2] |
| 13 | 15 | 38 | 11[*2] |
| 14 | 23 | 40 | 5[*2] |
| 15 | 27 | 41 | 17 |
| 16 | 37 | 42 | 13 |
| 17 | 48 | 43 | 14 |
| 18 | 55 | 44 | 22 |
| 19 | 27 | 45 | 38 |
| 20 | 41 | 46 | 18 |
| 21 | 55 | 47 | 8 |
| 22 | 45 | 48 | 25 |
| 23 | 40[*2] | 49 | 31 |
| 25 | 25[*1] | 50 | 38 |
| 51 | 32 | 68 | 11 |
| 52 | 37 | 69 | 13 |
| 53 | 40 | 70 | 17.5[*3] |
| 54 | 33 | 71 | 30 |
| 55 | 19 | 72 | 7 |

TABLE 4 (continued)

| Compound No. | Maximum reduction in blood pressure (mmHg) | Compound No. | maximum reduction in blood pressure (mmHg) |
|---|---|---|---|
| 56 | 23 | 73 | 11 |
| 57 | 25 | 74 | 25 |
| 58 | 15 | 75 | 5 |
| 59 | 17 | 76 | 5 |
| 60 | 27 | 77 | 38 |
| 61 | 22 | 78 | 17 |
| 62 | 30 | 79 | 33 |
| 63 | 30 | 80 | 30 |
| 64 | 18 | 81 | 20 |
| 65 | 17 | 82 | 22 |
| 66 | 10 | 83 | 27 |
| 67 | 6 | 84 | 28 |

*1 25 mg/kg administration; *2 50 mg/kg administration
*3 100 mg/kg administration

Experiment 2

Three male dd-strain mice (weight 20 ± 1 g) are used for each test compound.

Each of the compounds is added to aqueous physiological sodium chloride, and the mixture is orally (P.O.) administered to the mice in a dose of 1000 mg/kg. After the observation for 7 days, the numbers of deaths are counted, and the results are shown in Table 5.

TABLE 5

| Compound No. | Numbers of deaths | Compound - No. | Number of deaths |
|---|---|---|---|
| 18 | 0/3 | 21 | 1/3 |
| 20 | 0/3 | 22 | 1/3 |

Now, the process for preparing Compound [I] is described below.

Compound [I] may be prepared by reacting a compound represented by the formula [II]:

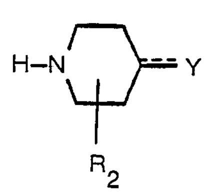

[II]

(wherein, $R_2$ and Y have the same significance as defined above) or Compound [II] wherein $R_3$ is protected, with a compound represented by the formula [III]

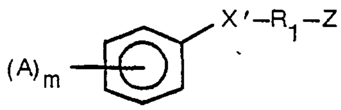

[III]

(wherein A, m and $R_1$ have the same significance as defined above; X' is oxygen, sulfur, carbonyl or methylene; and Z is halogen or an eliminable group) or Compound [III] wherein A is protected, and further if necessary, by reducing the resulting product and if necessary, by eliminating the protective group therefrom.

In the definition of Z, halogen includes chlorine, bromine or iodine; and the eliminable group includes alkylsulfonyloxy (for example, methanesulfonyloxy), arylsulfonyloxy (for example, benzenesulfonyloxy or p-toluenesulfonyloxy), etc.

The reaction of Compound [I] or the protected one with Compound [III] or the protected one is carried out in an inert solvent. Ketone (e.g. acetone), halogenated hydrocarbon (e.g. chloroform and methylene chloride), amide (e.g. dimethylformamide), sulfoxide (e.g. dimethylsulfoxide), substituted or unsubstituted aromatic hydrocarbon (e.g. benzene, toluene and chlorobenzene), lower alcohol (e.g. methanol, ethanol and isopropanol), etc. may be used alone or in combination as an inert solvent. The reaction is carried out at 0—150°C, preferably at a temperature between room temperature and the boiling point of the solvent depending on the reactivity of the group Z which is exchangeable. The reaction usually proceeds very smoothly in the presence of a base such as lower alcoholate (e.g. sodium methylate and sodium ethylate), alkali hydroxide (e.g. sodium hydroxide), alkali carbonate (e.g. sodium carbonate and potassium carbonate), tertiary amine (e.g. triethylamine and pyridine), etc. The amount of the base is usually 1.0 to 1.2 times the equivalent amount based on Compound [II]. When an acid addition salt of Compound [II] such as hydrochloride is used, it goes without saying that the base supplementary enough to neutralize the acid is added thereto. Use of reaction-promoting agents such as potassium iodide is effective for smooth proceeding of the reaction.

When either $R_3$ or A, or both is (are) hydroxy, amino or lower alkylamino, these groups are protected in a conventional manner prior to the above reaction. After completion of the reaction, the protective group is eliminated in a conventional manner to obtain the desired product.

When X' is carbonyl, the resulting product is reduced to obtain Compound [I] where X is hydroxymethylene. The reaction may be carried out by reacting Compound [I] wherein X is carbonyl with a complex metal hydride such as sodium borohydride in a lower alcohol such as methanol, ethanol and isopropanol at −10 to 100°C, preferably at a temperature between 0°C and the boiling piont of the used solvent. Alternatively, the reaction may be carried out by subjecting Compound [I] wherein X is carbonyl to catalytic reduction using hydrogenating catalyst such as palladium carbon, Raney nickel, platinum black, platinum carbon and platinum oxide in lower alcohol such as methanol and ethanol, lower aliphatic acid such as acetic acid, water or a mixed solvent thereof. These reactions may be carried out either in an open vessel or in a closed vessel under pressure. When the carbon atom of $R_1$ adjacent to X is an asymmetrical carbon in the above reduction, Compound [I] is stereo-selectively obtained according to the reduction method. That is, Compound [I] in threo form is obtained when a complex metal hydride is used and Compound [I] in erythro form is obtained according to catalytic reduction in an acidic condition. The acidic condition is brought with inorganic acids such as hydrochloric acid or organic acids such as acetic acid, propionic acid and succinic acid.

Compound [II] used as a starting compound of Compound [I] is also new compound. Compound [II] is classified as described in Table 6 for convenience.

TABLE 6

| Y | p | q | B | Compound No. | Formula |
|---|---|---|---|---|---|
| 1) | 0 | 0 | –N=N– | II–1 | 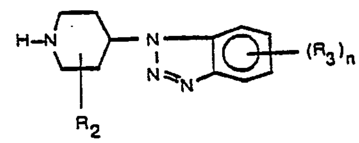 |
| 1) | 0 | 0 | –C=N–<br>\|<br>Me | II–2 | 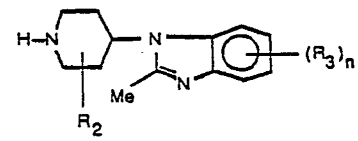 |
| 1) | 0 | 0 | –C–NH–<br>‖<br>N–CN | II–3 | 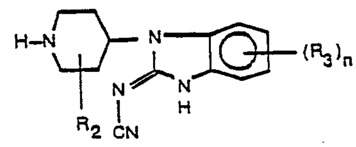 |
| 1) | 1 | 0 | –C–NH–<br>‖<br>O | II–4 | 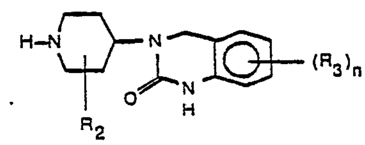 |
| 1) | 1 | 0 | –C–O–<br>‖<br>O | II–5 | 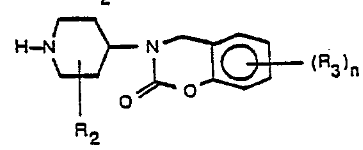 |
| 1) | 1 | 0 | –C–NH–<br>‖<br>N–CN | II–6 | 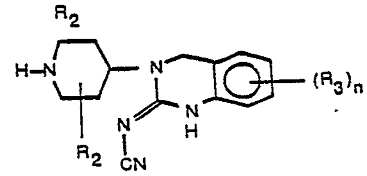 |
| 1) | 1 | 0 | –S–NH–<br>$O_2$ | II–7 | 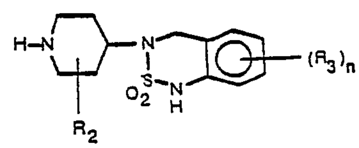 |
| 1) | 0 | 1 | –C–NH–<br>‖<br>O | II–8 | 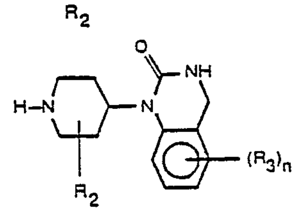 |
| 1) | 0 | 1 | –S–NH–<br>$O_2$ | II–9 | 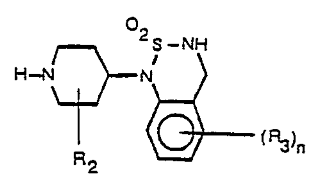 |
| 2) | – | – | – | II–10 | 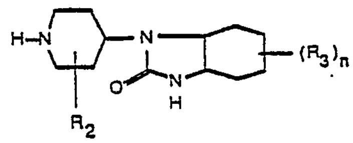 |
| 3) | – | – | – | II–11 | 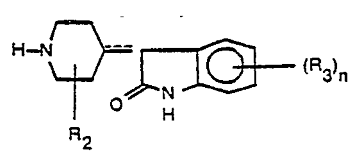 |

The compound [II-1] is produced as shown below.
First, a compound represented by the general formula [V]:

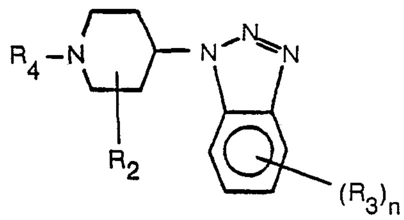

[V]

(wherein, $R_2$, $R_3$ and n have the same significance as defined above and $R_4$ is a protective group for the amino group) is prepared by reacting a nitrite such as sodium nitrite with an o-phenylenediamine derivative represented by the formula [IV]:

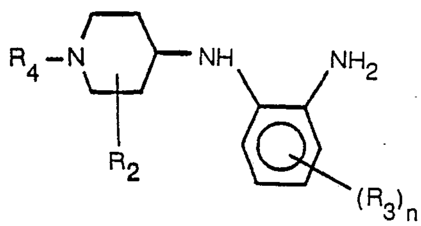

[IV]

(wherein, $R_2$, $R_3$, $R_4$ and n have the same significance as defined above) under an acidic condition.

It is proper to use sodium nitrite in an amount of 1 to 1.1 mols per mol of Compound [IV].

This reaction can be effectively carried out in an appropriate solvent such as water and acetic acid in combination thereof or alone at −10°C to 10°C.

Examples of the substituent $R_4$ in Compound [IV] are acyl (e.g. acetyl and benzoyl), alkyloxycarbonyl (e.g. tert.-butoxycarbonyl and ethoxycarbonyl), benzyl, tosyl and mesyl.

Finally, Compound [V] is converted to Compound [II-1] by being subjected to the usual reaction for eliminating the amino-protecting group.

Compound [II-2] is produced as shown below. First, an acetyl derivative represented by the formula [VII]:

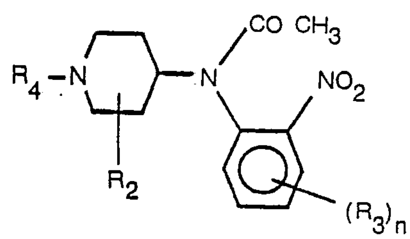

[VII]

(wherein, $R_2$, $R_3$, $R_4$ and n have the same significance as defined above) is prepared by acetylating a piperidine derivative represented by the formula [VI]:

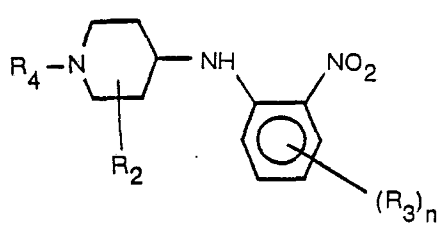

[VI]

(wherein, $R_2$, $R_3$, $R_4$ and n have the same significance as defined above). The acetylation can be effectively performed by heating Compound [VI] and an acetyl halide such as acetyl chloride, acetyl bromide or acetyl iodide in an inactive solvent such as dioxane and dimethylformamide. It is proper to use the acetyl halide in an amount of 3 to 10 equivalent weights of Compound [VI]. The reaction temperature is properly within the approximate range of from 50°C to 120°C.

Then, a benzimidazole derivative represented by the formula [VIII]:

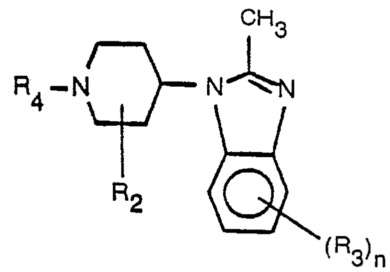

[VIII]

(wherein, $R_2$, $R_3$, $R_4$ and n have the same significance as defined above) is obtained by acidifying the reaction mixture after reduction of the nitro group of Compound [VII] to an amino group or by reducing Compound [VII] under an acidic condition.

The conversion of the nitro group into the amino group can be accomplished by the methods generally adopted for the purpose, for example, catalytic reduction method using palladium carbon or Raney nickel as a catalyst, methods using tin or tin compounds under an acidic condition and methods using complexes metal hydride such as lithium aluminum hydride. From the compound which has resulted from the conversion into the amino group, Compound [VIII] can be readily derived by directly acidifying the reaction mixture. When the reduction is carried out under an acidic condition as in the use of a tin compound, the reduction of the nitro group and the ring-closing to benzimidazole can be simultaneously accomplished. The method to be adopted for the reduction of the nitro group may be suitably selected depending on the kind of the protective group $R_4$ in the formula [VII]. When a protective group such as the benzyl group which can be removed by hydrogenation is selected as the substituent $R_4$, the reduction of the nitro group and the removal of the protective group, or the reduction of the nitro group, the ring-closing into a benzimidazole derivative and the removal of the protective group can be simultaneously effected.

Of the aforementioned various methods available for the reduction, that which involves use of palladium carbon or a tin compound will be explained in detail below. The reduction using palladium carbon is carried in an inert solvent under continuous introduction of hydrogen generally at room temperature to 50°C for 2 to 10 hours. Examples of the solvent usable in this reaction are lower alcohols such as methanol, ethanol and isopropanol, lower fatty acids such as acetic acid, water, dioxane, etc. and mixtures thereof.

The reduction using a tin compound is carried out in an inert solvent in the presence of a mineral acid such as hydrochloric acid or a lower fatty acid such as acetic acid generally at room temperature to 80°C for 2 to 10 hours. Examples of the solvent usable in this reaction are water, dioxane, lower alcohols, etc. and mixtures thereof.

Finally, Compound [VIII] is converted into the compound [II-2] by being subjected to the usual reaction for eliminating the amino-protective group.

Compound [II-3] is produced as shown below. First, a compound represented by the formula [IX]:

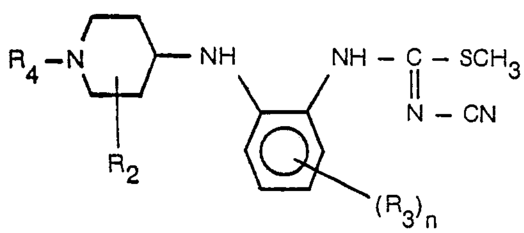

[IX]

(wherein, $R_2$, $R_3$, $R_4$ and n have the same significance as defined above) is prepared by reacting Compound [IV] with dimethyl-N-cyanodithioimino-carbonate

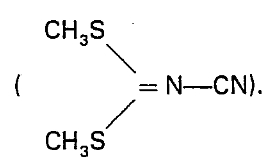

This reaction is carried out at room temperature to 150°C. The molar ratio of Compound [IV] to dimethyl-N-cyanodithioimino-carbonate is desirably about 1:1. The reaction is usually completed in 2 to 24 hours. Examples of the reaction solvent advantageously usable in the reaction include alcohols such as methanol, ethanol and isopropanol, dioxane, dimethoxy methane, dimethoxy ethane, dimethylsulfoxide and dimethylformamide.

Then, a compound represented by the formula [X]:

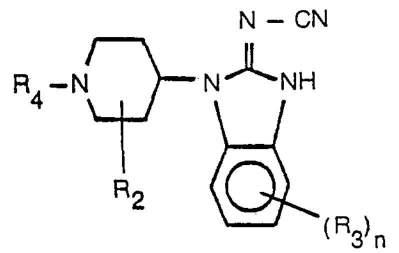

[X]

(wherein, $R_2$, $R_3$, $R_4$ and n have the same significance as defined above) is prepared by subjecting Compound [IX] to a ring-closing reaction in an inert solvent in the presence of a metal ion. Examples of suitable metal ions include $Hg^{2+}$ and $Ag^+$. Mercuric acetate, mercuric chloride, etc. are usable as sources of $Hg^{2+}$ and silver acetate, silver trifluoroacetate, silver fluoroborate, silver methanesulfonate, etc., as sources of $Ag^+$. The reaction temperature is desirably from 0°C to 80°C. The proper concentration of metal ion is from 1 to 1.2 mols per mol of the compound [X]. Generally, the reaction is completed in 15 minutes to 2 hours. The reaction solvents advantageously usable include alcohols) e.g. methanol, ethanol and isopropanol), chloroform, dichloromethane, dioxane, etc.

Finally, Compound [X] is converted to Compound [II-3] by being subjected to the usual reaction for eliminating the amino-protecting group.

Compound [11-4] is produced as shown below. First, a compound represented by the formula [XIII]:

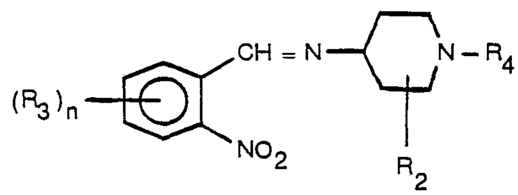

[XIII]

(wherein, $R_2$, $R_3$, $R_4$ and n have the same significance as defined above) is prepared by reacting the 4-amino-piperidine derivative represented by the formula [XI]:

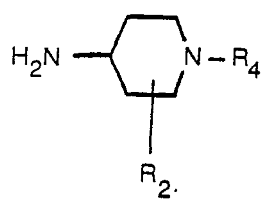

[XI]

(wherein, $R_2$ and $R_4$ have the same meanings as indicated above) with a compound represented by the formula [XII]:

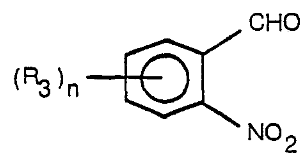

[XII]

(wherein, $R_3$ and n have the same significance as defined above).

The reaction can be carried out either without any solvent or in a lower alkanol (e.g. methanol, ethanol and propanol), an aromatic hydrocarbon (e.g. benzene, toluene and xylene), a halogenated hydrocarbon (e.g. methylene chloride and chloroform) or a mixture thereof. The reaction in an alkanol has the advantage that the conversion to a compound represented by the formula [XIV] below can be carried out without isolation of Compound [XIII] from the reaction solution. As regards the amounts of the reactants to be used, it is proper to use Compound [XII] in an amount of 1.0 to 1.2 equivalent weights, preferably 1.0 equivalent weight, of Compound [XI]. Although the reaction proceeds at room temperature in a short time, it may be carried out at an elevated temperature, if necessary.

Then, a 4-substituted piperidine derivative represented by the formula [XIV]:

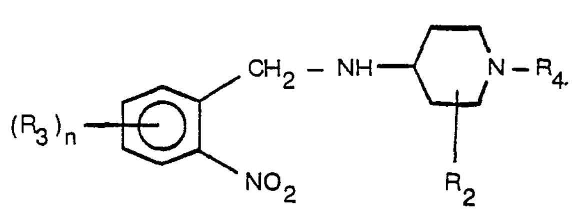

[XIV]

(wherein, $R_2$, $R_3$, $R_4$ and n have the same significance as defined above) is prepared by reducing Compound [XIII] with a complex metal hydride (e.g. sodium borohydride and sodium cyanoborohydride) in a lower alkanol (e.g. methanol, ethanol and isopropanol). This reaction is advantageously carried out at from −10°C to 100°C, preferably from 0°C to room temperature.

Then, a compound represented by the formula [XV]:

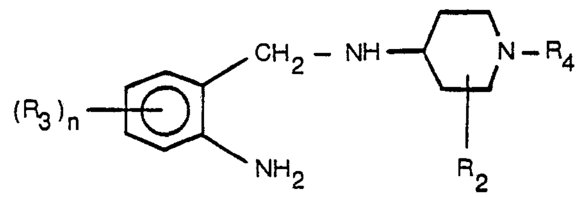

[XV]

(wherein, $R_2$, $R_3$, $R_4$ and n have the same significance as defined above) is prepared by reducing Compound [XIV]. This reaction can be performed by the methods generally adopted for the purpose of reducing a nitro group into an amino group, for example, the method involving combined use of a metal such as Sn, Fe or Zn and a mineral acid such as hydrochloric acid and sulfuric acid or an organic acid such as acetic acid, the method resorting to use of a sulfide or hydrazine and the catalytic reduction in the presence of a catalyst such as palladium carbon. When the reduction is performed by the catalytic method, the reaction is effected by causing the Compound [XIV] to absorb an equivalent weight of hydrogen in water, a lower alkanol such as methanol and ethanol, or a mixture thereof. The reaction is preferably carried out from 20°C to 60°C, especially around room temperature.

Thereafter, a compound represented by the formula [XVI]:

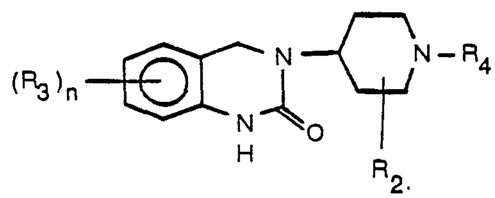

[XVI]

(wherein, $R_2$, $R_3$, $R_4$ and n have the same significance as defined above) is prepared by reacting Compound [XV] with a carbonic acid derivative such as phosgene, trichloromethylchloroformate, alkyl chlorocarbonate, 1,1′-carbonyldiimidazole and urea.

All of the above reactions can be carried out according to conventional methods and the reaction wherein 1,1′-carbonyldiimidazole is used is particularly described hereinafter. The reaction is carried out in an aprotic polar solvent such as halogenated hydrocarbon (e.g. methylene chloride and chloroform), ether (e.g. ethyl ether, tetrahydrofuran and dioxane), acetonitrile, dimethylformamide and dimethylsulfoxide, in combination thereof or alone, preferably with stirring. Preferably, the amount of 1,1′-carbonyldiimidazole is 1.0 to 2.0 times the equivalent amount based on Compound [VIII]. The reaction is carried out at a temperature of from room temperature to the boiling point of the used solvent. The reaction is usually completed in 1 to 3 hours when treated at the boiling point of the solvent and is completed in 10 to 15 hours, when treated at room temperature.

Finally, Compound [XVI] is converted to Compound [II-4] by eliminating the group $R_4$ by the usual methods.

Compound [II-5] is produced as shown below. First, a compound represented by the formula [XVIII]:

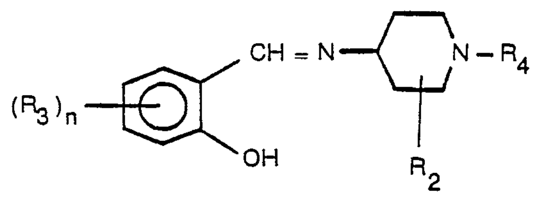

[XVIII]

(wherein, $R_2$, $R_3$, $R_4$ and n have the same significance as defined above) is prepared by reacting Compound [XI] with salicylaldehyde or a derivative thereof represented by the formula [XVII]:

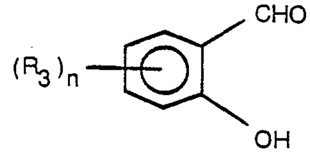

[XVII]

(wherein, $R_3$ and n have the same significance as defined above). This reaction can be performed similarly to that in the preparation of Compound [XIII] from Compound [XII].

Then, a compound represented by the formula [XIX]:

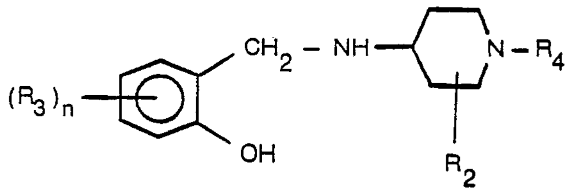

[XIX]

(wherein, $R_2$, $R_3$, $R_4$ and n have the same significance as defined above) is prepared by reducing Compound [XVIII]. This reaction can be performed similarly to that in the preparation of Compound [XIV] from Compound [XIII].

Subsequently, a compound represented by the formula [XX]:

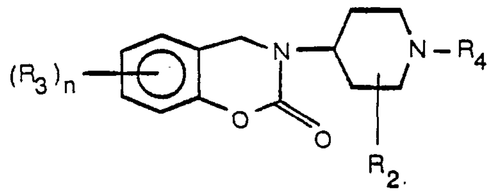

[XX]

(wherein, $R_2$, $R_3$, $R_4$ and n have the same significance as defined above) is prepared by reacting Compound [XIX] with a carbonic acid derivative. This reaction can be performed similarly to that which is involved in the preparation of Compound [XVI] from Compound [XV]. Finally, Compound [XX] is converted into Compound [II-5] by eliminating the group $R_4$ by the usual methods.

Compound [II-6] is produced as shown below.

First, a compound represented by the formula [XXI]:

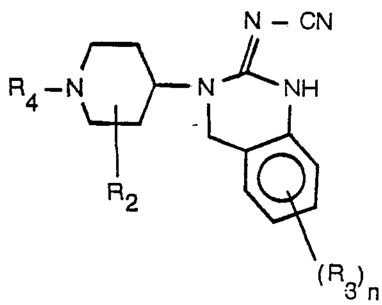

[XXI]

(wherein, $R_2$, $R_3$, $R_4$ and n have the same significance as defined above) is prepared by reacting dimethyl-N-cyanodithioiminocarbonate with Compound [XV]. This reaction can be performed similarly to that which is involved in the preparation of Compound [X] from Compound [IV]. Then, Compound [XXI] is converted into Compound [II-6] by eliminating the group $R_4$ by the usual methods.

Compound [II-7] is produced as shown below. First, a compound represented by the formula [XXII]:

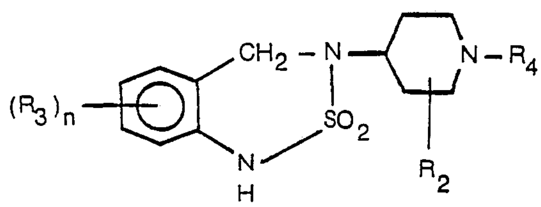

[XXII]

(wherein, $R_2$, $R_3$, $R_4$ and n have the same significance as defined above) is prepared by reacting Compound [XV] with sulfrylamide ($H_2NSO_2NH_2$).

This reaction is performed in pyridine. As concerns the amounts of the reactants involved in this reaction, it is advantageous to use the sulfurylamide in the amount of 1.0 to 4.0 equivalent weights of Compound [XV]. The reaction is generally performed under thermal reflux and is completed in a period of 2 to 10 hours. Then, Compound [XXII] is converted into Compound [II-7] by eliminating the group $R_4$ by the usual methods.

Compound [II-8] is produced as shown below.

First, a compound represented by the formula [XXV]:

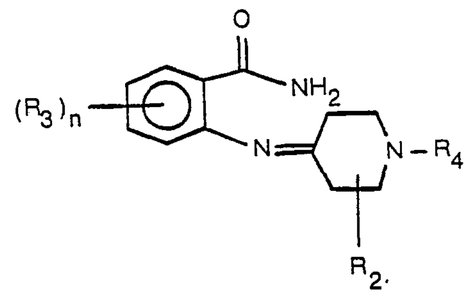

[XXV]

(wherein, $R_2$, $R_3$, $R_4$ and n have the same significance as defined above) is prepared by reacting a 4-oxo-piperidine derivative represented by the formula [XXIII]:

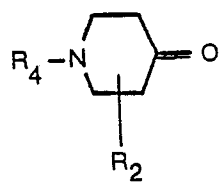

[XXIII]

(wherein, $R_2$ and $R_4$ have the same significance as defined above) with a compound represented by the formula [XXIV]:

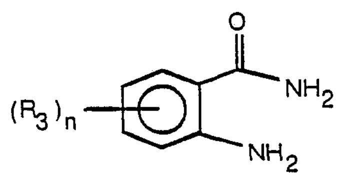

[XXIV]

(wherein, $R_3$ and n have the same significance as defined above) in an aromatic hydrocarbon type solvent such as benzene, toluene and xylene in the presence of sulfuric acid, an alkane-sulfonic acid (e.g. methanesulfonic acid) or an aryl-sulfonic acid (e.g. p-toluenesulfonic acid) as a catalyst.

The reaction mentioned above is advantageously performed by using p-toluenesulfonic acid as a catalyst and removing the water through distillation under reflux.

Then, a compound represented by the formula [XXVI]:

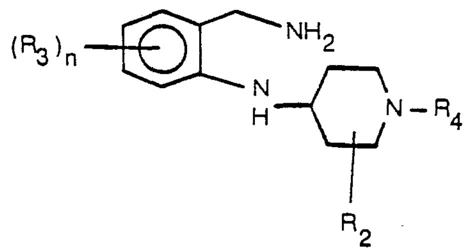

[XXVI]

(wherein, $R_2$, $R_3$, $R_4$ and n have the same significance as defined above) is prepared by reducing Compound [XXV] with a complex metal hydride, preferably lithium aluminum hydride, in an ether solvent such as ethyl ether, dioxane and tetrahydrofuran.

Subsequently, a compound represented by the formula [XXVII]:

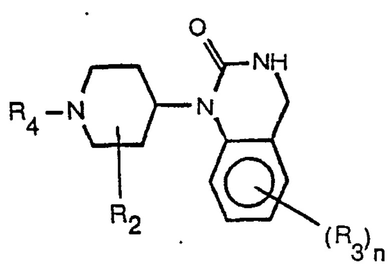

[XXVII]

(wherein, $R_2$, $R_3$, $R_4$ and n have the same significance as defined above) is produced by reacting Compound [XXVI] with a carbonic acid derivative. This reaction can be performed similarly to that in the

preparation of Compound [XVI] from Compound [XV]. Finally, Compound [XXVII] is converted into Compound [II-8] by eliminating the group $R_4$ by the usual methods.

Compound [II-9] is produced as shown below. First, a compound represented by the formula [XXVIII]:

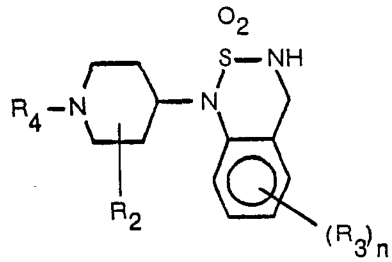

[XXVIII]

(wherein, $R_2$, $R_3$, $R_4$ and n have the same significance as defined above) is prepared by reacting Compound [XXVI] with sulfurylamide ($H_2NSO_2NH_2$). This reaction can be performed similarly to that in the preparation of Compound [XXII] from Compound [XV]. Then, Compound [XXVIII] is converted to Compound [II-9] by eliminating the group $R_4$ by the usual methods.

Compound [II-10] is produced as shown below. First, a compound represented by the formula [XXX]:

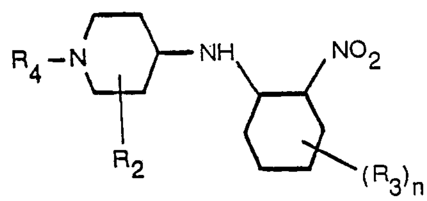

[XXX]

(wherein, $R_2$, $R_3$, $R_4$ and n have the same significance as defined above) is prepared by reacting Compound [XI] with a 1-nitrocyclohexene represented by the formula [XXIX]:

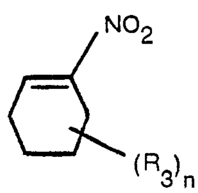

[XXIX]

(wherein, $R_3$ and n have the same significance as defined above). This reaction is performed in an inert organic solvent such as benzene, toluene, chloroform, dichloromethane, ether, tetrahydrofuran dimethylformamide and dimethylsulfoxide at a temperature of from 0°C to 100°C, preferably around room temperature, for a period of from 2 to 24 hours.

Then, a compound represented by the formula [XXXI]:

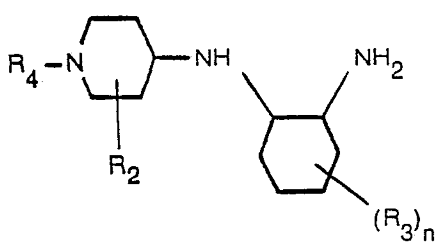

[XXXI]

(wherein, $R_2$, $R_3$, $R_4$ and n have the same significance as defined above) is produced by reducing Compound [XXX]. This reduction can be performed similarly to that in the preparation of Compound [XV] from Compound [XIV]. Subsequently, a compound represented by the formula [XXXII]:

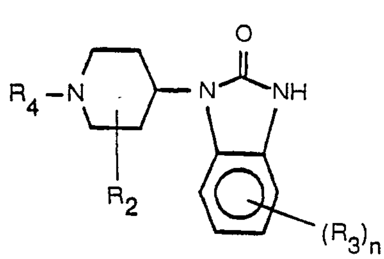

[XXXII]

(wherein, $R_2$, $R_3$, $R_4$ and n have the same significance as defined above) is produced by reacting Compound [XXXI] with a carbonic acid derivative. This reaction can be performed similarly to that in the preparation of Compound [XVI] from Compound [XV]. Finally, Compound [XXXII] is converted into Compound [II-10] by eliminating the group $R_4$ by the usual methods.

Compound [II-11] is produced as shown below. First, a compound represented by the formula [XXXIV]:

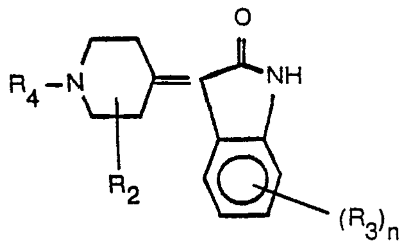

[XXXIV]

(wherein, $R_2$, $R_3$, $R_4$ and n have the same significance as defined above) is prepared by reacting Compound [XXIII] with a compound represented by the formula [XXXIII]:

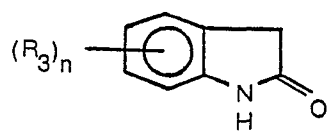

[XXXIII]

(wherein, $R_3$ and n have the same significance as defined above). The reaction mentioned above can be performed in a lower alkanol (e.g. methanol, ethanol and propanol) in the presence of a base such as ammonia at a temperature of from 0°C to 100°C. Then, Compound [XXXIV] is converted to Compound [II-11] by a method generally adopted for the purpose of removing a protective group from an amino group.

In this case, the choice between the presence and absence of the exo double bond at the 4-position of the piperidine in Compound [II-11] can be freely made by suitably selecting the kind of the protective group $R_4$ and the method for the removal of this group.

Isolation and purification of Compound [I] as well as the above-mentioned intermediates are carried out according to conventional methods in the field of organic synthetic chemistry, for example, concentration, extraction, recrystallization and chromatography. Specifically, since Compound [I] readily crystallizes in general, it can be isolated and purified by distilling off the solvent from the reaction mixture and recrystallizing the residue from a suitable solvent such as ethanol.

A pharmacologically acceptable acid addition salt of Compound [I] may be obtained by reacting Compound [I] with a suitable acid in a suitable solvent such as ethanol.

The pharmaceutical compositions of the present invention are described below.

It is obvious from the foregoing various experimental data that Compound [I] has a hypotensive activity.

In view of the hypotensive activity, the compounds of the present invention may be used in various pharmaceutical forms for administration. Pharmaceutical compositions of the present invention are prepared by uniformly mixing an effective amount of the compound in the form of a base or a acid addition salt as an active ingredient with a pharmaceutically acceptable carrier. According to the pharmaceutical forms suitable for administration, the carrier may take various forms. It is desirable that the pharmaceutical compositions are in single administration form suitable for administration per os or by injection.

In preparation of the composition for oral administration, any useful pharmaceutical carrier may be used. For example, water, glycols, oils, alcohols, etc. may be used to prepare oral liquid preparations such as suspensions and syrups, and excipients, lubricants, binders, disintegrators, etc. may be used to prepare powders, pills, capsules and tablets. Examples of the carriers are glucose and lactose as the excipients, starch and sodium alginate as the disintegrators, magnesium stearate, paraffin sulfate and talc as the lubricants, and syrup, ethanol and gelatin as the binders. The active ingredient is orally administered in a dose of 1—100 mg, particularly 10—60 mg, per day for an adult.

The preparation of Compound [I] and the present pharmaceutical compositions are illustrated by the following examples, and the preparation of the intermediates is illustrated by the following reference examples.

Example 1

1-(3,4-Dimethoxybenzoylmethyl)-4-[3,4-dihydro-2(1H)-quinazolinon-3-yl]-piperidine:

In this example, 2.31 g of 4-[3,4-dihydro-2-(1H)-quinazolinon-3-yl]-piperidine, 2.59 g of ω-bromo-3,4-dimethoxyacetophenone, 1.01 g of triethylamine and 70 ml of chloroform are mixed and

stirred at room temperature for 8 hours. Then, the resultant solution is concentrated to dryness. The residual crystals are mixed with water, then separated therefrom by filtration and dried to obtain 3.80 g of a crude product. The crude product is recrystallized from hot ethanol to obtain 3.17 g of the desired product.

Example 2

1-(3,4-Methylenedioxybenzoylmethyl)-4-[3,4-dihydro-2(1H)-quinazolinon-3-yl]-piperidine:

In this example, the procedure of Example 1 is repeated except that 2.31 g of 4-[3,4-dihydro-2-(1H)-quinazolinon-3-yl]-piperidine and 2.43 g of $\omega$-bromo-3,4-methylenedioxyacetophenone is used to produce a crude product. The crude product is recrystallized from hot ethanol to obtain 3.18 g of the desired product.

Example 3

1-[2-(3,4-Dimethoxyphenyl)-2-hydroxyethyl]-4-[3,4-dihydro-2(1H)-quinazolinon-3-yl]-piperidine:

In this example, 2.80 g of 1-(3,4-dimethoxybenzoylmethyl)-4-[3,4-dihydro-2(1H)-quinazolinon-3-yl]-piperidine and 150 ml of methanol are mixed and stirred at room temperature. Then, 460 mg of sodium borohydride is added to the stirred mixture over a period of 30 minutes. Thereafter, the mixture is stirred overnight at room temperature. The white crystals deposited are separated by filtration, successively washed with methanol and water and dried to obtain 2.02 g of a crude product. The crude product is crystallized from hot ethanol to obtain 1.86 g of the desired product.

Example 4

1-[2-(3,4-Methylenedioxyphenyl)-2-hydroxyethyl]-4-[3,4-dihydro-2(1H)-quinazolinon-3-yl]-piperidine:

In this example, the procedure of Example 3 is repeated except that 2.2 g of 1-(3,4-methylenedioxybenzoylmethyl)-4-[3,4-dihydro-2-(1H)-quinazolinon-3-yl]-piperidine and 410 mg of sodium borohydride are used to obtain 1.69 g of a crude product. The crude product is recrystallized from hot ethanol to obtain 1.50 g of the desired product.

Example 5

1-[1-(3,4-Dimethoxybenzoyl)-ethyl]-4-[3,4-dihydro-2(1H)-quinazolinon-3-yl]-piperidine:

In this example, 3.46 g of 4-[3,4-dihydro-2-(1H)-quinazolinon-3-yl]-piperidine, 4.10 g of $\alpha$-bromo-3,4-dimethoxypropiophenone, 2.1 ml of triethylamine and 30 ml of dimethylformamide are mixed and stirred at room temperature for a half and 2 hours. Then, the reaction solution is poured into 150 ml of ice water and the mixture is stirred for 30 minutes. The white crystals deposited are separated by filtration, washed with water and dried to obtain 5.87 g of a crude product. The crude product is recrystallized from hot ethanol to obtain 4.44 g of the desired product.

Example 6

1-[3-(3,4-Dimethoxyphenyl)-3-hydroxypropyl-2]-4-[3,4-dihydro-2(1H)-quinazolinon-3-yl]-piperidine:

In this example, 3.0 g of 1-[1-(3,4-dimethoxybenzoyl)-ethyl]-4-[3,4-dihydro-2(1H)-quinazolinon-3-yl]-piperidine and 150 ml of methanol are mixed and stirred under ice-cooling. Then, 1.50 g of sodium borohydride is added to the stirred mixture over a period of 3 hours. Thereafter, the mixture is stirred overnight at room temperature. Then, the white crystals deposited are separated by filtration, successively washed with methanol and water and dried to obtain 2.03 g of a crude product. On the other hand, the filtrate is concentrated and mixed with water. The white crystals deposited are separated by filtration, washed with water and dried to obtain 0.77 g of a crude product. The crude products are combined and recrystallized from hot ethanol to obtain 2.08 g of the desired product.

Example 7

1-[2-(3,4,5-Trimethoxyphenyl)-2-hydroxyethyl]-4-[3,4-dihydro-2(1H)-quinazolinon-3yl]-piperidine:

In this example, 3.47 g of 4-[3,4-dihydro-2(1H)-quinazolinon-3-yl]-piperidine, 4.33 g of $\omega$-bromo-3,4,5-trimethoxyacetophenone, 2.1 ml of triethylamine and 100 ml of methanol are mixed and stirred at room temperature for one hour and 40 minutes. Then, the reaction solution is concentrated and the resulting residue is crystallized by addition of water. The crystals are separated by filtration, washed with water and dried to obtain 6.50 g of crude crystals of 1-(3,4,5-trimethoxybenzoyl)-4-[3,4-dihydro-2(1H)-quinazolinon-3-yl]-piperidine. Then, 3.20 g of the crude crystals is suspended in 150 ml of methanol and the suspension is stirred under ice-cooling. Then, 2.5 g of sodium borohydride is added to the stirred suspension over a period of 3 hours. Thereafter, the resultant mixture is brought back to room temperature and then stirred overnight. The white crystals deposited are separated by filtration, successively washed with methanol and water, and dried to obtain 2.27 g of a crude product. The crude product is recrystallized from a mixed solvent of chloroform and ethanol to obtain 1.86 g of the desired product.

Example 8

1-(3,4-Dimethoxybenzoylmethyl)-4-[3,4-dihydro-2(1H)-quinazolinon-1-yl]-piperidine:

In this example, the procedure of Example 1 is repeated except that 2.77 g of 4-[3,4-dihydro-2(1H)-quinazolinon-1-yl]-piperidine, 3.11 g of ω-bromo-3,4-dimethoxyacetophenone and 1.21 g of triethylamine are used to obtain 4.15 g of a crude product. The crude product is recrystallized from hot ethanol to obtain 2.58 g of the desired product.

Example 9

1-(3,4-Methylenedioxybenzoylmethyl-4-[3,4-dihydro-2(1H)-quinazolinon-1-yl]-piperidine:

In this example, 3.46 g of 4-[3,4-dihydro-2(1H)-quinazolinon-1-yl]-piperidine, 3.64 g of ω-bromo-3,4-methylenedioxyacetophenone, 2.1 ml of triethylamine and 30 ml of dimethylformamide are mixed and stirred at room temperature for 3 hours. The resultant solution is poured into ice water. The crystals deposited are separated by filtration, washed with water and dried to obtain 5.23 g of a crude product. The crude product is recrystallized from hot ethanol to obtain 3.87 g of the desired product.

Example 10

1-[2-(3,4-Dimethoxyphenyl)-2-hydroxyethyl]-4-[3,4-dihydro-2(1H)-quinazolinon-1-yl]-piperidine:

In this example, 2.20 g of 1-(3,4-methylenedioxybenzoylmethyl)-4-[3,4-dihydro-2(1H)-quinazolinon-1-yl]-piperidine and 150 ml of methanol are mixed and stirred at room temperature. To the stirred mixture, 200 mg of sodium borohydride is added over a period of 30 minutes. Thereafter, the mixture is stirred at room temperature overnight and the resultant reaction solution is concentrated. The residue is mixed with water. The crystals deposited are separated by filtration, washed with water and dried to obtain 1.68 g of a crude product. The crude product is recrystallized from hot ethanol to obtain 1.23 g of the desired product.

Example 11

1-[2-(3,4-Methylenedioxyphenyl)-2-hydroxyethyl]-4-[3,4-dihydro-2(1H)-quinazolinon-1-yl]-piperidine:

In this example, 2.70 g of 1-(3,4-methylenedioxybenzoylmethyl)-4-[3,4-dihydro-2(1H)-quinazolinon-1-yl]piperidine and 150 ml of methanol are mixed and stirred under ice-cooling. Then, 2.0 g of sodium borohydride is added to the stirred mixture over a period of one hour. Subsequently, the mixture is brought back to room temperature and stirred for 3 hours at room temperature and the reaction solution is concentrated. The resultant residue is mixed with 50 ml of water and the mixture is extracted with ethyl acetate (100 ml x 2 and 50 ml x 1). The extract is washed with water, dried and concentrated. The resultant viscous residue is mixed with 5 ml of methanol. The crystals deposited are separated by filtration, washed with methanol and dried to obtain 2.5 g of a crude product. The crude product is recrystallized from hot ethanol to obtain 1.74 g of the desired product.
separated by filtration, washed with methanol and dried to obtain 2.05 g of a crude product. The crude product is recrystallized from hot ethanol to obtain 1.74 g of the desired product.

Example 12

1-[1-(3,4-Dimethoxybenzoyl)-ethyl]-4-[3,4-dihydro-2(1H)-quinazolinon-1-yl]-piperidine:

In this example, 3.46 g of 4-[3,4-dihydro-2(1H)-quinazolinon-1-yl]-piperidine, 4.10 g of α-bromo-3,4-dimethoxypropiophenone, 2.1 ml of triethylamine and 30 ml of dimethylformamide are mixed and stirred for 3 hours at room temperature. Then, the resultant solution is poured into ice water. The insoluble substance deposited is separated by filtration, washed with water and mixed with methanol. The crystals deposited are separated by filtration, washed with methanol and dried to obtain 4.68 g of a crude product. On the other hand, the water layer is further extracted with chloroform and the organic layer is washed with water, dried and concentrated. The residue is mixed with 2 ml of methanol. The resultant crystals are separated by filtration and dried to obtain 0.46 g of a crude product. These crude products are combined and recrystallized from hot ethanol to obtain 3.73 g of the desired product.

Example 13

1-[3-(3,4-Dimethoxyphenyl)-3-hydroxypropyl-2]-4-[3,4-dihydro-2(1H)-quinazolinon-1-yl]-piperidine:

In this example, 2.70 g of 1-[1-(3,4-dimethoxybenzoyl)-ethyl]-4-[3,4-dihydro-2(1H)-quinazolinon-1-yl]-piperidine and 150 ml of methanol are mixed and stirred under ice-cooling. Then, 2.0 g of sodium borohydride is added to the stirred mixture over a period of one hour. The resultant mixture is brought back to room temperature and stirred at room temperature for 3 hours. The white crystals deposited are separated by filtration, successively washed with methanol and dried to obtain 2.07 g of a crude product. This crude product is recrystallized from a mixed solvent of chloroform and ethanol to obtain 1.87 g of the desired product.

Example 14

1-Benzoylmethyl-4-[3,4-dihydro-2(1H)-quinazolinon-3-yl]-piperidine:

In this example, 4.0 g of 4-[3,4-dihydro-2(1H)-quinazolinon-3-yl]-piperidine mono-hydrochloride, 3.0 g of ω-bromoacetophenone, 4.2 ml of triethylamine and 60 ml of methanol are mixed and

stirred at room temperature overnight. Then, the white crystals deposited are separated by filtration, successively washed with methanol and water and dried to obtain 4.38 g of a crude product. The crude product is recrystallized from chloroform-ethanol to obtain 3.40 g of the desired product.

Example 15

1-(3-Chlorobenzoylmethyl)-4-[3,4-dihydro-2(1H)-quinazolinon-3-yl]-piperidine:

In this example, 2.67 g of 4-[3,4-dihydro-2(1H-quinazolinon-3-yl]-piperidine mono-hydrochloride, 2.33 g of $\alpha$-bromo-m-chloroacetophenone, 2.8 ml of triethylamine and 30 ml of methanol are mixed and stirred at room temperature overnight. Then, the white crystals deposited are separated by filtration, successively washed with methanol and water and dried to obtain 2.89 g of a crude product. The crude product is recrystallized from a mixed solvent of chloroform and ethanol to obtain 1.93 g of the desired product.

Example 16

1-(4-Chlorobenzoylmethyl)-4-[3,4-dihydro-2(1H)-quinazolinon-3-yl]-piperidine:

In this example, 4.0 g of 4-[3,4-dihydro-2(1H)-quinazolinon-3-yl]-piperidine mono-hydrochloride, 3.5 g of $\alpha$-bromo-p-chloroacetophenone, 4.2 ml of triethylamine and 60 ml of methanol are mixed and stirred at room temperature overnight. The white crystals deposited are separated by filtration, successively washed with methanol and water and dried to obtain 5.17 g of a crude product. The crude product is recrystallized from a mixed solvent of chloroform and ethanol to obtain 3.21 g of the desired product.

Example 17

1-(4-Methoxybenzoylmethyl)-4-[3,4-dihydro-2-(1H)-quinazolinon-3-yl]-piperidine:

In this example, 4.00 g of 4-[3,4-dihydro-2(1H)-quinazolinon-3-yl]-piperidine mono-hydrochloride, 3.44 g of $\alpha$-bromo-p-methoxyacetophenone, 4.2 ml of triethylamine and 60 ml of methanol are mixed and stirred at room temperature overnight. Then, the white crystals deposited are separated by filtration, successively washed with methanol and water and dried to obtain 4.71 g of a crude product. The crude product is recrystallized from a mixed solvent of chloroform and ethanol to obtain 3.79 g of the desired product.

Example 18

1-(2-Phenyl-2-hydroxyethyl)-4-[3,4-dihydro-2(1H)-quinazolinon-3-yl]-piperidine:

In this example, 2.30 g of 1-benzoylmethyl-4-[3,4-dihydro-2(1H)-quinazolinon-3-yl]-piperidine and 100 ml of methanol are mixed and stirred at room temperature. To the stirred mixture. 1.5 g of sodium borohydride is added over a period of 5 hours. Then, the mixture is stirred at room temperature overnight. The white crystals deposited are separated by filtration, successively washed with methanol and water and dried to obtain 2.06 g of a crude product. The crude product is recrystallized from a mixed solvent of chloroform and ethanol to obtain 1.47 g of the desired product.

Example 19

1-[2-(3-Chlorophenyl)-2-hydroxyethyl]-4-[3,4-dihydro-2(1H)-quinazolinon-3-yl]-piperidine:

In this example, 1.50 g of 1-(3-chlorobenzoylmethyl)-4-[3,4-dihydro-2(1H)-quinazolinon-3-yl]-piperidine and 75 ml of methanol are mixed and stirred at room temperature. To the stirred mixture, 1.0 g of sodium borohydride is added over a period of 5 hours. Then, the mixture is stirred overnight at room temperature. The white crystals deposited are separated by filtration, successively washed with methanol and water and dried to obtain 1.21 g of a crude product. The crude product is recrystallized from a mixed solvent of chloroform and ethanol to obtain 0.87 g of the desired product.

Example 20

1-[2-(4-Chlorophenyl)-2-hydroxyethyl]-4-[3,4-dihydro-2(1H)-quinazolinon-3-yl]-piperidine:

In this example, 2.70 g of 1-(4-chlorobenzoylmethyl)-4-[3,4-dihydro-2(1H)-quinazolinon-3-yl]-piperidine and 150 ml of methanol are mixed and stirred at room temperature. To the stirred mixture, 2.0 g of sodium borohydride is added over a period of 5 hours. Then, the resultant mixture is stirred overnight at room temperature. The white crystals deposited are separated by filtration, successively washed with methanol and water and dried to obtain 2.07 g of a crude product. The crude product is recrystallized from a mixed solvent of chloroform and ethanol to obtain 1.59 g of the desired product.

Example 21

1-[2-(4-Methoxyphenyl)-2-hydroxyethyl]-4-[3,4-dihydro-2(1H)-quinazolinon-3-yl]-piperidine:

In this example, 2.70 g of 1-(4-methoxybenzoyl)-4-[3,4-dihydro-2(1H)-quinazolinon-3-yl]-piperidine and 150 ml of methanol are mixed and stirred at room temperature. To the stirred mixture, 2.0 g of sodium borohydride is added over a period of 5 hours. Then, the mixture is stirred overnight at room temperature. The white crystals deposited are separated by filtration, successively washed with methanol and water and dried to obtain 2.40 g of a crude product. The crude product is recrystallized

from a mixed solvent of chloroform and ethanol to obtain 1.41 g of the desired product.

Example 22

1-[2-(3,4-Dichlorophenyl)-2-hydroxyethyl]-4-[3,4-dihydro-2(1H)-quinazolinon-3-yl]-piperidine:

In this example, 3.20 g of 4-[3,4-dihydro-2(1H)-quinazolinon-3-yl]-piperidine hydrochloride, 3.22 g of ω-bromo-3,4-dichloroacetophenone, 3.36 ml of triethylamine and 48 ml of methanol are mixed and stirred overnight. The resultant crystals are separated by filtration, washed with 5 ml of methanol and dried to obtain 4.55 g of 1-(3,4-dichlorobenzoylmethyl)-4-[3,4-dihydro-2(1H)-quinazolinon-3-yl]-piperidine as crude crystals. Then, 4.00 g of the crude crystals are suspended in 200 ml of ethanol and stirred at room temperature. Then, 2.0 g of sodium borohydride is added to the stirred suspension and the mixture is stirred overnight. Further, 1.0 g of sodium borohydride is added to the mixture. The mixture is refluxed with heating for 3 hours and then brought back to room temperature. The resultant white crystals are separated by filtration, successively washed with methanol and water and dried to obtain 3.5 g of a crude product. The crude product is twice recrystallized from a mixed solvent of dimethylformamide and methanol to obtain 1.88 g of the desired product.

Example 23

1-(3,4-Dimethoxybenzoylmethyl)-4-(2-oxo-3,4-dihydro-2H-1,3-benzoxazin-3-yl)-piperidine:

In this example, 4.03 g of 4-(2oxo-3,4-dihydro-2H-1,3-benzoxazin-3-yl)-piperidine hydrochloride, 3.88 g of ω-bromo-3,4-dimethoxy-acetophenone, 4.2 ml of triethylamine and 30 ml of N,N-dimethylformamide are mixed and stirred at room temperature for 2 hours and 30 minutes. Subsequently, the reaction mixture is poured into 150 ml of ice water and stirred therein for 3 hours. The crystals deposited are separated by filtration and dried to obtain 5.36 g of a crude product. The crude product is recrystallized from hot ethanol to obtain 4.94 g of the desired product.

Example 24

1-(3,4-Methylenedioxybenzoylmethyl)-4-(2-oxo-3,4-dihydro-2H-1,3-benzoxazin-3-yl)-piperidine:

In this example, 4.03 g of 4-(2-oxo-3,4-dihydro-2H-1,3-benzoxazin-3-yl)-piperidine hydrochloride, 3.64 g of ω-bromo-3,4-methylenedioxy-acetophenone, 4.2 ml of triethylamine and 30 ml of N,N-dimethylformamide are mixed and stirred at room temperature for 2 hours and 30 minutes. Subsequently, the reaction mixture is poured into 150 ml of ice water and stirred therein for 3 hours. The crystals deposited are separated by filtration and dried to obtain 5.43 g of a crude product. The crude product is recrystallized from hot ethanol to obtain 4.82 g of the desired product.

Example 25

1-[2-(3,4-Dimethoxyphenyl)-2-hydroxyethyl]-4-(2-oxo-3,4-dihydro-2H-1,3-benzoxazin-3-yl)-piperidine:

In this example, 3.0 g of 1-(3,4-dimethoxybenzyolmethyl)-4-(2-oxo-3,4-dihydro-2H-1,3-benzoxazin-3-yl)-piperidine obtained in Example 23 is mixed with 150 ml of methanol. While this mixture is stirred at 0—10°C, 0.8 g of sodium borohydride is added thereto over a period of 3 hours. The reaction solution is stirred overnight at room temperature. Then, the solution is further mixed with 0.4 g of sodium borohydride and stirred at room temperature for 3 hours. The white crystals deposited are separated by filtration, washed with methanol and water and dried to obtain 2.77 g of a crude product. The crude product is recrystallized from ethanol to obtain 2.52 g of the desired product.

Example 26

1-[2-(3,4-Methylenedioxyphenyl)-2-hydroxyethyl]-4-(2-oxo-3,4-dihydro-2H-1,3-benzoxazin-3-yl)-piperidine:

In this example, 3.0 g of 1-(3,4-methylenedioxybenzoylmethyl)-4-(2-oxo-3,4-dihydro-2H-1,3-benzoxazine-3-yl)-piperidine obtained in Example 24 is mixed with 150 ml of methanol. While the mixture is stirred at 0 to 10°C, 0.8 g of sodium borohydride is added thereto over a period of 3 hours. Then, the mixture is stirred overnight at room temperature. The mixture is further mixed with 0.4 g of sodium borohydride and stirred at room temperature for 3 hours. The white crystals deposited are separated by filtration, washed with methanol and water and dried to obtain 2.36 g of a crude product. In the meantime, the filtrate is concentrated under reduced pressure. The residue is mixed with water. The crystals deposited are separated by filtration, washed with water and dried to obtain 0.37 g of a crude product. The two crude products are combined and recrystallized from ethanol to obtain 2.49 g of the desired product.

Example 27

1-[1-(3,4-Dimethoxybenzoyl)-ethyl]-4-(2-oxo-3,4-dihydro-2H-1,3-benzoxazin-3-yl)-piperidine:

In this example, 4.03 g of 4-(2-oxo-3,4-dihydro-2H-1,3-benzoxazin-3-yl)-piperidine hydrochloride, 4.10 g of α-bromo-3,4-dimethoxypropiophenone, 4.2 ml of triethylamine and 30 ml of N,N-dimethylformamide are mixed and stirred at room temperature for 6 hours. Then, the reaction mixture is poured into 150 ml of ice water. The oily substance formed is separated by decantation of the water phase. The oily substance is dissolved in 100 ml of ethyl acetate. The solution is washed with a saturated aqueous sodiom chloride, dried and concentrated under reduced pressure to obtain 5.0 g of a crude product. The crude product is recrystallized from ethanol to obtain 4.21 g of the desired product.

Example 28

1-[3-(3,4-Dimethoxyphenyl)-3-hydroxypropan-2-yl]-4-(2-oxo-3,4-dihydro-2H-1,3-benzoxazin-3-yl)-piperidine:

In this example, 2.8 g of 1-[1-(3,4-dimethoxybenzoyl)-ethyl]-4-(2-oxo-3,4-dihydro-2H-1,3-benzoxazin-3-yl)-piperidine obtained in Example 27 is mixed with 150 ml of methanol. While the mixture is stirred at 0 to 10°C, 2 g of sodium borohydride is added thereto over a period of one hour. The mixture is further stirred for 3 hours at that temperature and then stirred overnight at room temperature. The white crystals deposited are separated by filtration, washed with methanol and water and dried to obtain 1.90 g of a crude product. The crude product is recrystallized from ethanol to obtain 1.67 g of the desired product.

Example 29

1-(3,4,5-Trimethoxybenzoylmethyl)-4-(2-oxo-3,4-dihydro-2H-1,3-benzoxazin-3-yl)-piperidine:

In this example, 4.03 g of 4-(2-oxo-3,4-dihydro-2H-1,3-benzoxazin-3-yl)-piperidine hydrochloride, 4.33 g of $\omega$-bromo-3,4,5-trimethoxy-acetophenone, 42 ml of triethylamine and 100 ml of methanol are mixed and stirred at room temperature for one hour and 40 minutes. The reaction solution is concentrated under reduced pressure and the residue is mixed with water. The crystals deposited are separated by filtration, washed with water and dried to obtain 5.8 g of a crude product. The crude product is recrystallized from ethanol to obtain 2.75 g of the desired product.

Example 30

1-[2-(3,4,5-Trimethoxyphenyl)-2-hydroxyethyl]-4-(2-oxo-3,4-dihydro-2H-1,3-benzoxazin-3-yl)-piperidine:

In this example, 2.8 g of 1-(3,4,5-trimethoxybenzoylmethyl)-4-(2-oxo-3,4-dihydro-2H-1,3-benzoxazin-3-yl)-piperidine is mixed with 150 ml of methanol. While this mixture is stirred at 0 to 10°C, 1.5 g of sodium borohydride is added thereto over a period of 2 hours. Then, the mixture is stirred overnight at room temperature. The mixture is further mixed with 1 g of sodium borohydride and stirred overnight. The white crystals deposited are separated by filtration, washed with methanol and water and dried to obtain 1.96 g of a crude product. The crude product is recrystallized from ethanol to obtain 1.76 g of the desired product.

Example 31

1-[2-(3,4-methylenedioxyphenyl)-2-oxo-ethyl]-4-(1H-benzotriazol-1-yl)-piperidine:

In this example, 3.7 g of $\omega$-bromo-3,4-methylenedioxy-acetophenone is added to a solution of 4.0 g of 4-(1H-benzotriazol-1-yl)-piperidine hydrochloride and 3.1 g of triethylamine in 50 ml of methanol and the mixture is stirred overnight at room temperature. The reaction mixture is concentrated and the residue is mixed with 50 ml of ethyl acetate and 50 ml of water to separate an organic layer. The organic layer is washed with water several times, dried and concentrated. The residue is recrystallized from isopropyl alcohol to obtain 3.2 g of the desired product.

Example 32

1-[2-(3,4-Methylene-dioxyphenyl)-2-hydroxyethyl]-4-(1H-benzotriazol-1-yl)-piperidine:

In this example, 1.75 g of 1-[2-(3,4-methylene-dioxyphenyl)-2-oxo-ethyl]-4-(1H-benzotriazol-1-yl)piperidine obtained in Example 31 is suspended in 30 ml of methanol. To this suspension, 190 mg of sodium borohydride is added at room temperature over a period of 30 minutes. After completion of the addition, the reaction mixture is heated to 40°C and stirred for one hour. The reaction mixture is concentrated. The residue is mixed with 20 ml of water, stirred and then filtered. The cake is dried to obtain 1.61 g of crude crystals. The crude crystals are recrystallized from ethanol to obtain 1.4 g of the desired product.

Example 33

1-[2-(3,4-Dimethoxyphenyl)-2-oxo-ethyl]-4-(2-methylbenzimidazol-3-yl)-piperidine:

A solution of 2.8 g of 4-(2-methyl-benzimidazol-3-yl)-piperidine, 1.32 g of triethylamine and 3.37 g of $\omega$-dimethoxy-acetophenone in 50 ml of methanol is stirred at room temperature for 10 hours. The reaction mixture is concentrated. The residue is mixed with ethyl acetate and water and shaken and then the organic layer is separated. The organic layer is washed with 30 ml of water four times and then dried. The dried organic layer is concentrated to dryness under reduced pressure. The residue is recrystallized from isopropyl alcohol twice to obtain 2.72 g of the desired product.

Example 34

1-[2-(3,4-Dimethoxyphenyl)-2-hydroxyethyl]-4-(2-methyl-benzimidazol-3-yl)-piperidine difumarate:

In this example, 1.38 g of 1-[2-(3,4-dimethoxyphenyl)-2-oxoethyl]-4-(2-methyl-benzimidazol-3-yl)-piperidine obtained in Example 33 is dissolved in 40 ml of methanol. To this solution, 0.13 g of sodium borohydride is added with stirring over a period of 30 minutes. The resultant mixture is further stirred for 2 hours and then concentrated. The residue is mixed with a small amount of water. The mixture is extracted with 20 ml of chloroform three times. The chloroform layer is washed with water and dried. The layer is concentrated to dryness under reduced pressure. The residual oily substance is dissolved in 10 ml of ethanol. The solution is mixed with 0.8 g of fumaric acid and stirred. The crystals deposited are separated by filtration and dried. The crude crystals are recrystallized from methanol to obtain 1.28 g of the desired product.

Example 35

1-(3,4-Methylenedioxybenzoylmethyl)-4-(2-cyanoimino-3,4-dihydro-1H-quinazolin-3-yl)-piperidine:

In this example, 1.74 g of ω-bromo-3,4-methylenedioxy-acetophenone is added to a solution of 2.4 g of 4-(2-cyanoimino-3,4-dihydro-1H-quinazolin-3-yl)-piperidine hydrochloride and 2.5 g of triethylamine in 50 ml of methanol. The mixture is stirred overnight at room temperature. The reaction mixture is concentrated. The residue is mixed with 10 ml of water and stirred and the crystals deposited are separated by filtration. The crystals are washed with water and methanol and dried. The crude crystals are recrystallized from methanol to obtain 1.9 g of the desired product.

Example 36

1-[2-(3,4-Methylene-dioxyphenyl)-2-hydroxyethyl]-4-(2-cyanoimino-3,4-dihydro-1H-quinazolin-3-yl)-piperidine:

In this example, 1.16 g of the product obtained in Example 35 is suspended in 30 ml of ethanol. While the suspension is stirred, 2.0 g of sodium borohydride is added thereto over a period of one hour. After the addition, the resultant mixture is heated to 50°C and allowed to undergo reaction for 2 hours. The reaction mixture is concentrated to dryness under reduced pressure. The residue is mixed with 10 ml of water and the deposited crystals are separated by filtration. The crystals are washed with water and ethanol and then dried. The crude crystals are recrystallized from chloroform-methanol to obtain 923 mg of the desired product.

Example 37

1-[2-(3,4,5-Trimethoxyphenyl)-2-oxo-1-methylethyl]-4-(2-cyanoimino-3,4-dihydro-1H-quinazolin-3-yl)-piperidine:

In this example, 2.17 g of ω-bromo-3,4,5-trimethoxypropiophenone is added to a solution of 2.4 g of 4-(2-cyanoimino-3,4-dihydro-1H-quinazolin-3-yl)-piperidine hydrochloride and 2.5 g of triethylamine in 50 ml of methanol. The mixture is allowed to react at room temperature for 12 hours. The solution is concentrated, the residue is mixed with 15 ml of water and the mixture is stirred. The crystals deposited are separated by filtration. The crystals are washed with water and ethanol and dried. The crude crystals are recrystallized from methanol to obtain 1.4 g of the desired product.

Example 38

1-[2-(3,4,5-Trimethoxyphenyl)-2-hydroxy-1-methylethyl]-4-(2-cyanoimino-3,4-dihydro-1H-quinazolin-3-yl)-piperidine:

In this example, 848 mg of the product obtained in Example 37 is dissolved in 30 ml of ethanol. The solution is subjected to reaction in the same conditions as those of Example 36 by using 1 g of sodium borohydride. The reaction mixture is subjected to the same post-treatment as that of Example 36 to obtain crude crystals. The crude crystals are recrystallized from methanol to obtain 554 mg of the desired product.

Example 39

1-[2-(3,4-Methylenedioxyphenyl)-2-oxo-ethyl]-4-(2-cyanoimino-1H-benzimidazol-1-yl)-piperidine:

In this example, 1.9 g of ω-bromo-3,4-methylenedioxyacetophenone is added to a solution of 2.5 g of 4-(2-cyanoimino-1H-benzimidazol-1-yl)piperidine hydrochloride and 2.75 g of triethylamine in 20 ml of methanol. The mixture is allowed to react at room temperature for 12 hours. The solution is concentrated and the residue is mixed with 20 ml of water and stirred. The crystals formed therein are separated by filtration, washed with water and ethanol and then dried. The crude crystals are recrystallized from methanol to obtain 1.8 g of the desired product.

Example 40

1-[2-(3,4-Methylenedioxyphenyl)-2-hydroxyethyl]-4-(2-cyanoimino-1H-benzimidazol-1-yl)-piperidine:

In this example, 600 mg of sodium borohydride is added to a suspension of 715 mg of the product obtained in Example 39 in 30 ml of ethanol at room temperature. After completion of the addition, the reaction mixture is concentrated. The residue is mixed with water, and the crystals deposited are separated by filtration and dried. The crude crystals are recrystallized from methanol to obtain 632 mg of the desired product.

Example 41

1-(3,4-Dimethoxybenzoylmethyl)-4-(3,4-dihydro-2,2-dioxo-1H-2,1,3-benzothiadiazin-3-yl)-piperidine:

In this example, 4.56 g of 4-(3,4-dihydro-2,2-dioxo-1H-2,1,3-benzothiadiazin-3-yl)-piperidine hydrochloride, 3.88 g of 3,4-dimethoxy-ω-bromoacetophenone and 3.04 g of triethylamine are dissolved in 100 ml of chloroform. The solution is stirred at room temperature for 2 days. The reaction mixture is washed with water, dried and concentrated under reduced pressure. The residue is mixed with 10 ml of methanol and allowed to stand for one hour. The crystals deposited are separated by filtration, washed with methanol and dried to obtain 2.57 g of crude crystals. The crude crystals are recrystallized from ethanol to obtain 2.35 g of the desired product.

Examples 42—45

Compounds of Compound Nos. 59, 61, 62 and 65:

Since the compounds designated as Compound Nos. 59, 61, 62 and 65 in Table 1 are obtained according to a similar procedure, the procedure for the preparation of compound 61 is exemplified below. Particulars on the other compounds are shown in Table 7.

In 30 ml of N,N-dimethylformamide, 4.545 g (15 m.mols) of 4-(3,4-dihydro-2,2-dioxo-1H-2,1,3-benzothiadiazin-1-yl)-piperidine monohydrochloride, 3.884 g (15 m.mols) of ω-bromo-3,4-dimethoxy-acetophenone and 4.2 ml (30 m.mols) of triethylamine are dissolved and the solution is stirred at room temperature for 2 hours and 20 minutes. The reaction solution is mixed with 300 ml of chloroform, washed with water (100 ml × 2 and 50 ml × 2), washed with a saturated aqueous sodium chloride, dried and concentrated to dryness under reduced pressure. Since the residue still contains N,N-dimethylformamide, it is washed with 80 ml of water added thereto. The water layer is removed by decantation. The residue is mixed with 50 ml of methanol. The solvent is distilled off under reduced pressure. The residual crystals are mixed with 3 ml of methanol and separated by filtration to obtain 5.88 g of crude crystals. The crude crystals are recrystallized from ethanol to obtain 5.55 g of 1-(3,4-dimethoxybenzoylmethyl) - 4 - (3,4 - dihydro - 2,2 - dioxo - 1H - 2,1,3 - benzothiadiazin - 1 - yl)-piperidine $C_2H_5OH$ (Compound No. 61).

TABLE 7

| Compound No. | Starting compound | | Reaction solvent | Base | Solvent for recrystallization | Yield |
|---|---|---|---|---|---|---|
| 59 | 4-(3,4-Dihydro 2,2-dioxo-1H-2,1,3-benzothia-diazin-3-yl)-piperidine hydrochloride 3.04 g (10 m.mols) | ω-Bromo-3,4-methylene-dioxy acetophenone 2.43 g 10 m.moles) | DMF 20 ml | Triethylamine 2.8 ml (20 m.mols) | EtOH | 69.5% |
| 61 | 4-(3,4-Dihydro-2,2-dioxo-1H-2,1,3-benzothia-diazin-1-yl)-piperidine hydrochloride 5.454 g (15 m.mols) | ω-Bromo-3,4-dimethoxy-acetophenone 3.884 g (15 m.mols) | DMF 30 ml | Triethylamine 4.2 ml (30 m.mols) | EtOH | 83.2% |
| 62 | Same as above 4.545 g (15 m.mols) | ω-Bromo-3,4-methylene-dioxy-acetophenone 3.64 g 15 m.mols) | DMF 30 ml | Triethylamine 4.2 ml (30 m.mols) | EtOH | 68.3% |
| 65 | Same as above 4.17 g (13.7 m.mols) | α-Bromo-3,4-dimethoxy-propiophenon 3.74 g (13.7 m.mols) | DMF 30 ml | Triethylamine 3.82 ml (27.4 m.mols) | EtOH—$H_2O$ | 31.1% |

Note) The forms of the compounds are the same as those of Table 3.

Examples 46—50

Compounds of Compound Nos. 58, 60, 63, 64 and 66:

Since the compounds designated as Compound Nos. 58, 60, 63, 64 and 66 in Table 1 are prepared according to a similar procedure, the procedure for the preparation of Compound 58 is exemplified below. Particulars on the other compounds are shown in Table 8.

In 150 ml of methanol, 2.2 g of 1-(3,4-dimethoxybenzoylmethyl)-4-(3,4-dihydro-2,2-dioxo-1H-2,1,3-benzothiadiazin-3-yl)-piperidine is suspended. While this suspension is stirred at room temperature, 1.0 g of sodium borohydride is added thereto by portions over a period of four hours. The mixture is stirred overnight at room temperature and 400 mg of sodium borohydride is added thereto. Then, the mixture is stirred overnight. The reaction solution is concentrated under reduced pressure. The residue is mixed with 100 ml of water and adjusted to pH 9.3 with concentrated hydrochloric acid. The resultant mixture is extracted with chloroform. The organic layer is washed with water, dried and freed from the solvent by distillation to obtain a syrup as a residue. This residue is crystallized by adding methanol thereto. The crystals are separated by filtration to obtain 1.93 g of crude crystals. The crude crystals are recrystallized from ethanol to obtain 1.45 g of 1-[2-(3,4-dimethoxyphenyl)-2-hydroxyethyl]-4-(3,4-dihydro-2,2-dioxo-1H-2,1,3-benzothiadiazin-3-yl)-piperidine (Compound No. 58).

TABLE 8

| Compound No. | Starting compound | Reaction solvent | Reducing agent | Solvent for recrystallization | Yield |
|---|---|---|---|---|---|
| 60 | 1-(3,4-Methylenedioxybenzoylmethyl)-4-(3,4-dihydro-2,2-dioxo-1H-2,1,3-benzothiadiazin-3-yl(-piperidine 2.2 g | MeOH 150 ml | $NaBH_4$ 1.4 g | EtOH | 65.6% |
| 63 | 1-(3,4-Dimethoxybenzoylmethyl)-4-(3,4-dihydro-2,2-dioxo-1H-2,1,3-benzothiadiazin 1-yl)-piperidine 3.0 g | MeOH 150 ml | $NaBH_4$ 1.5 g | EtOH | 82.3% |
| 64 | 1-(3,4-Methylenedioxybenzoylmethyl)-4-(3,4-dihydro-2,2-dioxo-1H-2,1,3-benzothiadiazin-1-yl)-piperidine 3.0 g | MeOH 150 ml | $NaBH_4$ 1.5 g | EtOH | 67.3% |
| 66 | 1-[1-(3,4-Dimethoxybenzoyl)-ethyl]-4-(3,4-dihydro-2,2-dioxo-1H-2,1,3-benzothiadiazin-1-yl)-piperidine monohydrochloride 1.7 g | MeOH 100 ml | $NaBH_4$ 2.0 g | $EtOH/H_2O$ | 57.9% |

Note) The forms of the compounds are the same as those of Table 3.

Example 51

1-(3,4-Methylenedioxybenzoylmethyl)-4-(2-oxyindol-3-yl)-piperidine:

In this example, 4.45 g of ω-bromo-3,4-methylenedioxy-acetophenone is added to a solution of 4.62 g of 4-(2-oxyindol-3-yl)-piperidine hydrochloride and 3.8 g of triethylamine in 100 ml of methanol. The mixture is stirred overnight at room temperature. The reaction mixture is concentrated. The residue is mixed with 50 ml of water, stirred and filtered. The crystals are washed with 20 ml of water and 10 ml of ethanol and then dried. Consequently, 5.5 g of crude crystals are obtained. The crude crystals are recrystallized from ethanol to obtain 5.0 g of the desired product.

Example 52

1-[2-(3,4-Methylenedioxyphenyl)-2-hydroxyethyl]-4-(2-oxyindol-3-yl)-piperidine:

In this example, 0.3 g of sodium borohydride is added to a solution of 2.9 g of 1-(3,4-methylenedioxybenzoylmethyl)-4-(2-oxyindol-3-yl)-piperidine obtained in Example 51 in 50 ml of ethanol over a period of 30 minutes. After the addition, the mixture is stirred at room temperature for 2 hours and then concentrated. The residue is mixed with water to separate an oily substance, which is extracted with 50 ml of chloroform twice. The chloroform layer is washed with water and then dried. The dried layer is concentrated to dryness under reduced pressure and the residue is recrystallized from ethyl acetate to obtain 2.0 g of the desired product.

Example 53

1-[2-(3,4-Dimethoxyphenyl)-2-oxo-1-methylethyl]-4-(2-oxyindol-3-yl)-piperidine:

In this example, 2.53 g of ω-bromo-3,4-dimethoxy-propiophenone is added to a solution of 2.34 g of 4-(2-oxyindol-3-yl)-piperidine hydrochloride and 1.88 g of triethylamine in 40 ml of methanol. The mixture is stirred at room temperature for 24 hours. The resultant mixture is concentrated to dryness under reduced pressure, and the residue is mixed with water, stirred and then filtered. The crystals are washed with 20 ml of water and 5 ml of isopropyl alcohol and then dried to obtain 3.55 g of crude crystals. The crude crystals are recrystallized from isopropyl alcohol to obtain 3.0 g of the desired product.

Example 54

1-[2-(3,4-Dimethoxyphenyl)-2-hydroxy-1-methylethyl]-4-(2-oxyindol-3-yl)-piperidine fumarate:

In this example, 1.0 g of sodium borohydride is added to a solution of 1.6 g of 1-[2-(3,4-dimethoxyphenyl)-2-oxo-1-methylethyl]-4-(2-oxyindol-3-yl)-piperidine obtained in Example 53 in 70 ml of methanol over a period of 30 minutes. The mixture is stirred at room temperature for 2 hours and then concentrated to dryness under reduced pressure. The residue is mixed with water and the resultant crystalline substance is separated by filtration. The crystalline substance is washed with water and then dried to obtain 1.49 g of crude crystals. The crude crystals are dissolved in 15 ml of isopropyl alcohol and then 0.42 g of fumaric acid is added thereto. The crystals deposited are separated by filtration and dried. The crude crystals are recrystallized from isopropyl alcohol to obtain 1.23 g of the desired product.

Example 55

3-[1-(3,4-Methylenedioxybenzoylmethyl)-4-piperidylidene]-2-oxyindole:

In this example, 2.84 g of ω-bromo-3,4-methylenedioxyacetophenone is added to a solution of 2.5 g of 3-(4-piperidylidene)-2-oxyindole and 1.19 g of triethylamine in methanol. The mixture is stirred at room temperature for 7 hours. The reaction mixture is freed from the solvent by distillation. The residue is mixed with water, stirred and then filtered. The crystals are washed with 20 ml of water and 5 ml of ethanol and dried to obtain 3.83 g of a crude product. The crude product is recrystallized from ethanol to obtain 3.61 g of the desired product.

Example 56

3-{1-[2-(3,4-Methylenedioxyphenyl)-2-hydroxyethyl]-4-piperidylidene}-2-oxyindole:

In this example, 4 g of 3-[1-(3,4-methylenedioxybenzoylmethyl)-4-piperidylidene]-2-oxyindole obtained in the same manner as that of Example 55 is dissolved in 300 ml of methylene chloride. To the solution, a solution of 8 g of diisobutyl aluminum hydride in 100 ml of methylene chloride is added dropwise at —25°C over a period of 2 hours. The resultant mixture is further stirred at the same temperature for 5 hours. Then, 30 ml of methanol and subsequent 100 ml of water are added to the mixture, and the methylene chloride layer is separated. The methylene chloride layer is washed with 30 ml of water and then dried three times. The layer is freed from methylene chloride by distillation. The residue is mixed with 10 ml of ethyl acetate and stirred. The resultant crystals are separated by filtration and dried to obtain 4.0 g of a crude product. The crude product is recrystallized from methanol-methylene chloride to obtain 2.3 g of the desired product.

Example 57

1-[2-(3,4-Methylenedioxyphenyl)-2-hydroxyethyl]-4-(2-oxyindol-3-yl)-piperidine:

In this example, 0.6 g of sodium borohydride is added to a solution of 2.9 g of 3-[1-(3,4-Methylenedioxybenzoylmethyl)-4-piperidylidene]-2-oxyindole obtained in the same manner as that of

Example 55 in 50 ml of ethanol over a period of one hour. After the addition, the mixture is stirred at room temperature for 2 hours and then concentrated. The residue is mixed with water and the oily substance separated is extracted with 50 ml of chloroform twice. The chloroform layer is washed with water and dried. The layer is freed from chloroform by distillation. The residue is recrystallized from ethyl acetate to obtain 2.05 g of the desired product.

Example 58

1-[2-(3,4-Dimethoxyphenyl)-2-oxo-ethyl]-4-(octahydro-2H-benzimidazol-2-one-1-yl)-piperidine:

In this example, 1.03 g of $\omega$-bromo-3,4-dimethoxyacetophenone is added to a solution of 1.1 g of 4-(octahydro-2H-benzimidazol-2-one-1-yl)-piperidine hydrochloride and 810 mg of triethylamine in 10 ml of methanol. The mixture is stirred at room temperature for 2 hours. The reaction mixture is concentrated. The residue is mixed with water and the crystals formed are separated by filtration. The crystals are washed with water and then with isopropanol and subsequently dried. Consequently, 1.4 g of crude crystals are obtained. The crude crystals are recrystallized from 20 ml of ethanol to obtain 1.18 g of the desired product.

Compounds of Compound Nos. 78—80 can be prepared in a similar manner as that of this example.

Example 59

1-[2-(3,4-Dimethoxyphenyl)-2-hydroxyethyl]-4-(octahydro-2H-benzimidazol-2-one-1-yl)-piperidine:

In this example, 720 mg of 1-[2-(3,4-dimethoxyphenyl)-2-oxo-ethyl]-4-octahydro-2H-benzimidazol-2-one-1-yl)-piperidine obtained by the procedure of Example 58 is suspended in 10 ml of methanol. To this suspension, 200 mg of sodium borohydride is added by portions at room temperature over a period of 30 minutes. After completion of the addition, the mixture is further stirred at room temperature for one hour and then concentrated. The residue is mixed with water and ethyl acetate and shaken. The organic layer is separated, washed with water and dried. The layer is concentrated to dryness under reduced pressure. The residue is mixed with a small amount of ether and stirred. The crystals formed are separated by filtration and dried to obtain 590 mg of crude crystals. The crude crystals are recrystallized from ethyl acetate to obtain 520 mg of the desired product.

The compounds of Compound Nos. 82—84 can be prepared in a similar manner to that of this example.

Example 60

(Example of preparing 10,000 5 mg-tablets)

| | |
|---|---|
| Compound 18 | 50 g |
| Magnesium stearate | 4 g |
| Crystalline cellulose | 746 g |

The above-described ingredients are mixed for 5 minutes by means of a mixer. The resulting mixed powder is made into 10,000 tablets of 6.0 mm in diameter, 2.5 mm in thickness, and 80 mg in weight using a tablet-making machine (Model HU—37; made by Kikusui Seisakusho) equipped with a pestle having a plane surface and round corners.

Example 61

| | |
|---|---|
| Compound 45 | 55 g |
| Magnesium stearate | 4 g |
| Crystalline cellulose | 741 g |

The above-described ingredients are processed in the same manner as in Example 60 to obtain tablets.

Example 62

| | |
|---|---|
| Compound 53 | 56 g |
| Magnesium stearate | 4 g |
| Crystalline cellulose | 740 g |

The above-described ingredients are processed in the same manner as in Example 60 to obtain tablets.

Example 63

(Example of preparing a powder)

| | |
|---|---|
| Compound 21 | 110 g |
| Lactose | 890 g |

The above-described ingredients are mixed for 10 minutes using a mixer to obtain a uniform mixture (powder).

Example 64

| | |
|---|---|
| Compound 77 | 109 g |
| Lactose | 891 g |

The above-described ingredients are mixed for 10 minutes using a mixer to obtain a uniform mixture (powder).

Reference Example 1

1-Benzyl-4-[N-(o-nitrobenzyl)-amino]-piperidine dihydrochloride:

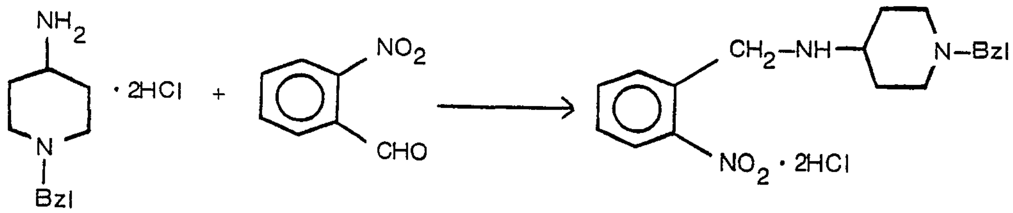

In this reference example, 5.24 g of 1-benzyl-4-amino-piperidine dihydrochloride, 3.02 g of o-nitrobenzaldehyde, 2.02 g of triethylamine and 30 ml of methanol are mixed and stirred at room temperature for one hour. The resultant solution is cooled with ice and stirred. To the stirred solution, 1 g of sodium borohydride is added little by little over a period of one hour. Then, the mixture is brought back to room temperature and stirred at room temperature for 2 hours. The resultant solution is poured into 200 ml of ice water and extracted with ether. The organic layer is washed with water, dried and concentrated. The oily residue is dissolved in 20 ml of ethanol. The solution is mixed with 10 ml of a solution of 5.7N hydrochloric acid in ethyl acetate. The white crystals deposited are separated by filtration, washed with 20 ml of ethyl acetate and dried to obtain 5.70 g of a crude product. The crude product is recrystallized from hot ethanol to obtain 2.90 g of the desired product.

Melting point: 260.0—263.5°C

Elementary analysis (%): $C_{19}H_{25}N_3O_2Cl_2$

| | C | H | N |
|---|---|---|---|
| Calculated | 57.29 | 6.33 | 10.55 |
| Found | 57.19 | 6.48 | 10.27 |

Reference Example 2

1-Benzyl-4-[N-(o-aminobenzyl)-amino]-piperidine trihydrochloride:

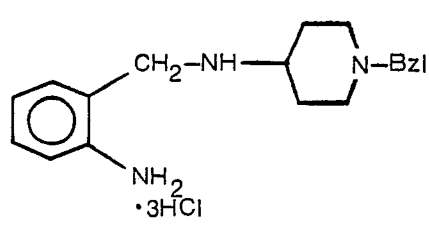

In this reference example, 31.8 g of 1-benzyl-4-[N-(o-nitrobenzyl)-amino]-piperidine dihydrochloride, 3.2 g of 10% Pd-C and 500 ml of water are mixed and stirred at room temperature.

Then, about 6 l of hydrogen gas is absorbed by the mixture. Then the reaction is discontinued and the Pd-C is removed by filtration. The aqueous solution is adjusted to pH 11 with an aqueous sodium hydroxide and then extracted with ethyl acetate. The organic layer is washed with water, dried and concentrated. The resulting oily product is dissolved in 140 ml of ethanol. Then, 70 ml of a solution of 5.7N hydrochloric acid in ethyl acetate is added thereto. The white crystals deposited are separated by filtration, washed with ethyl acetate and dried to obtain 23.5 g of a crude product. The crude product is recrystallized from a mixed solvent of methanol and ethyl acetate to obtain 15.0 g of the desired product.

Melting point: 225.0—228.0°C

Elementary analysis (%): $C_{19}H_{28}N_3Cl_3$

| | C | H | N |
|---|---|---|---|
| Calculated | 56.37 | 6.97 | 10.38 |
| Found | 56.35 | 7.00 | 10.31 |

Reference Example 3

1-Benzyl-4-[3,4-dihydro-2(1H)-quinazolinon-3-yl]-piperidine:

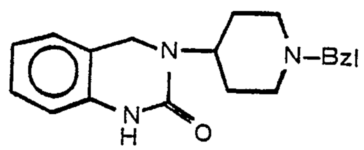

In this reference example, 4.04 g of 1-benzyl-4-[N-o-aminobenzyl)-amino]-piperidine trihydrochloride, 3.04 g of triethylamine, 2.03 g of 1,1′-carbonyldiimidazole and 50 ml of acetonitrile are mixed and stirred at room temperature for 4 hours. Then, 0.8 g of 1,1′-carbonyldiimidazole is further added to the mixture and the resultant mixture is refluxed with heating for 20 minutes. The solution is cooled to room temperature. The crystals deposited are separated by filtration, washed with water and dried to obtain 2.50 g of a crude product. The crude product is recrystallized from hot ethanol to obtain 1.75 g of the desired product.

Melting point: 204.5—205.5°C

Elementary analysis (%): $C_{20}H_{23}N_3O$

| | C | H | N |
|---|---|---|---|
| Calculated | 74.74 | 7.21 | 13.07 |
| Found | 75.02 | 7.16 | 13.21 |

Reference Example 4

4-[3,4-Dihydro-2(1H)-quinazolinon-3-yl]-piperidine hydrochloride:

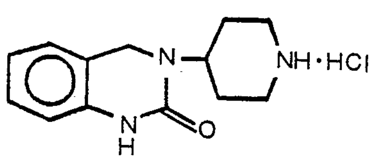

In this reference example, 1.0 g of 1-benzyl-4-[3,4-dihydro-2(1H)-quinazolinon-3-yl]-piperidine, 0.10 g of 10% Pd—C, 10 ml of acetic acid and 10 ml of methanol are mixed and stirred at room temperature. Hydrogen gas is introduced to the stirred mixture for 4 days. Then, the reaction is discontinued and the Pd—C is removed by filtration. The filtrate is concentrated. The resultant oily substance is dissolved in 5 ml of ethanol. Then, 5 ml of a solution of 5.7N hydrochloric acid in ethyl acetate is added thereto and the resultant mixture is allowed to stand overnight at room temperature. The crystals deposited are separated by filtration, washed with ethyl acetate and dried to obtain 0.75 g of a crude product. The crude product is recrystallized from a mixed solvent of methanol and ethyl acetate to obtain 0.52 g of the desired product.

Melting point: 288.0—293.0°C

Elementary analysis (%): $C_{13}H_{18}N_3OCl$

| | C | H | N |
|---|---|---|---|
| Calculated | 58.32 | 6.77 | 15.69 |
| Found | 58.26 | 6.87 | 15.65 |

Reference Example 5

1-Benzyl-4-[N-(o-aminomethylphenyl)-amino]-piperidine:

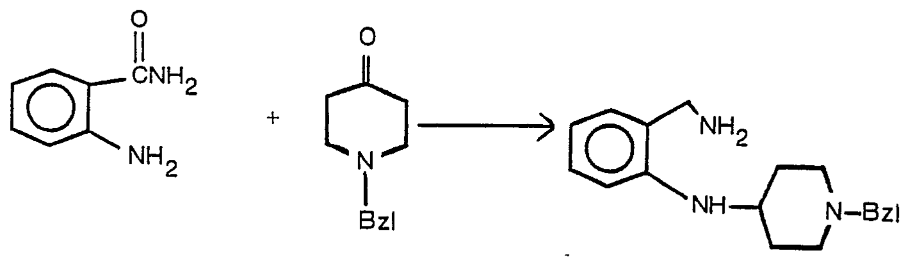

In this reference example, the mixture of 13.6 g of anthranilamide, 18.9 g of 1-benzyl-4-piperidone, 1.0 g of p-toluene-sulfonic acid monohydrate and 200 ml of benzene is refluxed in a Dean Stark apparatus for 6 hours to distil off water. The resultant suspension of the Schiff-base is concentrated. The residue is mixed with 250 ml of dry dioxane to form a suspension. Separately, 100 ml of dry dioxane and 12.4 g of lithium aluminum hydride are mixed. The above Schiff-base suspension is added little by little to the mixture with stirring without cooling. The mixture is stirred for one hour and then refluxed with heating for 18 hours. The reaction solution is cooled to room temperature and poured little by little into 1.3 l of ice water. The resultant suspension is poured little by little into a funnel previously coated with a filter aid and subjected to suction filtration. The cake on the funnel is washed with 500 ml of chloroform and filtered. The organic layer is washed with water, dried and concentrated to obtain 22.9 g of a crude product. The crude product is recrystallized from hot ethanol to obtain 18.7 g of the desired product.

Melting point: 117.0—118.0°C

Elementary analysis (%): $C_{19}H_{25}N_3$

| | C | H | N |
|---|---|---|---|
| Calculated | 77.25 | 8.53 | 14.22 |
| Found | 77.45 | 8.66 | 13.98 |

Reference Example 6

1-Benzyl-4-[3,4-dihydro-2(1H)-quinazolinon-1-yl]-piperidine:

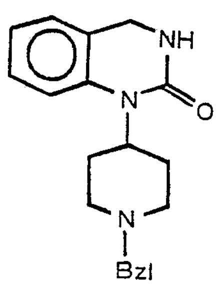

In this reference example, to the mixture of 23.0 g of 1-benzyl-4-[N-(ortho-aminomethylphenyl)-amino]-piperidine and 200 ml of acetonitrile, 17.4 g of 1,1′-carbonyldiimidazole is added over a period of 3 hours with stirring at 40° to 60°C. Then, the resultant mixture is refluxed with heating for one hour, brought back to room temperature and stirred at room temperature for 2 hours. The crystals deposited are separated by filtration, successively washed with water and methanol and dried to obtain 16.8 g of a crude product. The crude product is recrystallized from methanol to obtain 12.18 g of the desired product.

Melting point: 119.5—120.5°C

Elementary analysis (%): $C_{20}H_{23}N_3O$

| | C | H | N |
|---|---|---|---|
| Calculated | 74.74 | 7.21 | 13.07 |
| Found | 74.76 | 7.31 | 13.16 |

Reference Example 7

4-[3,4-Dihydro-2(1H)-quinazolinon-1-yl]-piperidine:

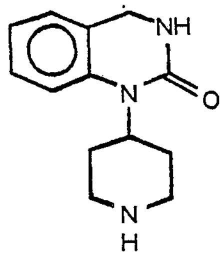

In this reference example, 16.0 g of 1-benzyl-4-[3,4-dihydro-2(1H)-quinazolinon-1-yl]-piperidine, 4.0 g of 10% Pd—C, 50 ml of 1N hydrochloric acid, 150 ml of water and 300 ml of methanol are mixed and stirred at 40°C. Hydrogen gas is introduced to the mixture for 5 hours with stirring. Then, the Pd—C is removed by filtration and the filtrate is concentrated. The white crytalline residue is dissolved in 50 ml of water. The solution is adjusted to pH 10 with aqueous 5N sodium hydroxide and extracted with chloroform. The extract is washed with water, dried and thereafter concentrated. Then, 50 ml of a mixture of ethyl acetate and n-hexane (1:1 by volume) is added to the residue. After trituration, the solid is separated by filtration, washed with the same mixed solvent to obtain 9.9 g of a crude product. The crude product is recrystallized from water—ethanol to obtain 5.1 g of the desired product.

Melting point: 153.0—155.0°C

Elementary analysis (%): $C_{13}H_{17}N_3O$

| | C | H | N |
|---|---|---|---|
| Calculated | 67.51 | 7.41 | 18.17 |
| Found | 67.54 | 7.56 | 18.12 |

Reference Example 8

1-Benzyl-4-[N-(o-hydroxybenzyl)-amino]-piperidine:

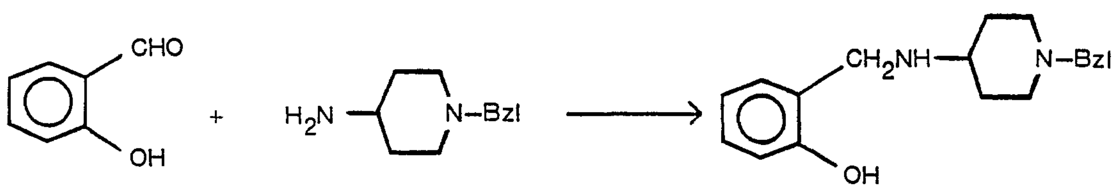

In this reference example, 9.76 g of salicylaldehyde, 15.12 g of 1-benzyl-4-amino-piperidine and 100ml of methanol are mixed and stirred at room temperature for one hour. While the resultant mixture is cooled with ice, 3.2 g of sodium borohydride is added thereto over a period of one hour and 30 minutes. Then, the mixture is stirred at room temperature for 2 hours. The resultant reaction mixture is poured into 1 l of ice water. The light yellow crystals deposited are separated by filtration and then dissolved in 200 ml of ethyl acetate. The ethyl acetate solution is washed with a saturated aqueous sodium chloride, dried and subsequently concentrated under reduced pressure to obtain 19.0 g of light yellow crystals. The crystals are recrystallized from ethanol to obtain 12.0 g of the desired product.

Melting point: 92.5—93.0°C

Elementary analysis (%): $C_{19}H_{24}N_2O$

| | C | H | N |
|---|---|---|---|
| Calculated | 76.99 | 8.16 | 9.45 |
| Found | 76.97 | 8.10 | 9.37 |

Reference Example 9

1-Benzyl-4-(2-oxo-3,4-dihydro-2H-1,3-benzoxazin-3-yl)-piperidine:

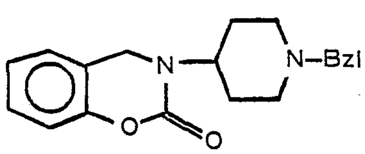

In this reference example, 17.7 g of 1-benzyl-4-[N-(o-hydroxybenzyl)-amino]-piperidine obtained by the procedure of Reference Example 8, 19.4 g of 1,1′-carbonyl-dimidazole and 150 ml of tetrahydrofuran are mixed, stirred for 2 hours and 30 minutes, refluxed and then concentrated under reduced pressure.

Subsequently, the formed crystalline residue is mixed with water and separated by filtration to obtain 18.0 g of crude crystals. The crude crystals are recrystallized from a mixture of n-hexane and ethyl acetate [50:7 (V/V)] to obtain 14.85 g of the desired product.

Melting point: 106.0—107.0°C

Elementary analysis (%): $C_{20}H_{22}N_2O_2$

| | C | H | N |
|---|---|---|---|
| Calculated | 74.51 | 6.88 | 8.69 |
| Found | 74.80 | 6.98 | 8.74 |

Reference Example 10

4-(2-Oxo-3,4-dihydro-2H-1,3-benzoxazin-3-yl)-piperidine hydrochloride:

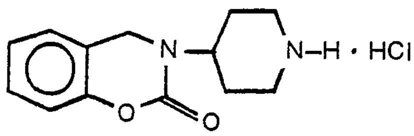

In this reference example, 16.0 g of 1-benzyl-4-(2-oxo-3,4-dihydro-2H-1,3-benzoxazin-3-yl)-piperidine and 4.0 g of 10% palladium carbon are added to a mixture of 150 ml of water, 50 ml of 1N hydrochloric acid and 300 ml of methanol. While the resultant mixture is stirred, hydrogen gas is passed therethrough overnight. Then, the reaction mixture is filtered and the filtrate is concentrated. The white crystals as a residue are mixed with methanol and separated by filtration to obtain 11.4 g of the desired product.

Melting point: 288.0—290.0°C

Elementary analysis (%): $C_{13}H_{17}N_2O_2Cl$

| | C | H | N |
|---|---|---|---|
| Calculated | 58.10 | 6.38 | 10.42 |
| Found | 57.92 | 6.40 | 10.26 |

Reference Example 11

1-Benzyl-4-(2-aminoanilino)-piperidine trihydrochloride:

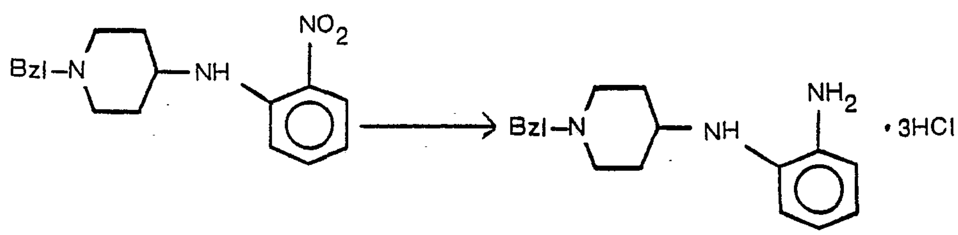

In this reference example, 600 mg of 1-benzyl-4-(2-nitroanilino)-piperidine is dissolved in methanol. The solution is mixed with 60 mg of 5% palladium carbon and stirred at 30—32°C in the atmosphere of hydrogen under the atmospheric pressure. The stirring is stopped after 2 hours and 30 minutes, and the catalyst is removed by filtration. The filtrate is mixed with 1 ml of a solution of 215 mg of hydrogen chloride gas in 1 ml of methanol and then concentrated. The residue is mixed with ether filtered and dried to obtain 640 mg of the desired product as a powder.

Infrared absorption spectrum (KBr): 1625, 1280 $cm^{-1}$

Elementary analysis (%): $C_{18}H_{26}Cl_3N_3$

| | C | H | N |
|---|---|---|---|
| Calculated | 55.32 | 6.71 | 10.75 |
| Found | 55.22 | 6.75 | 10.68 |

Reference Example 12

1-Benzyl-4-(1H-benzotriazol-1-yl)-piperidine:

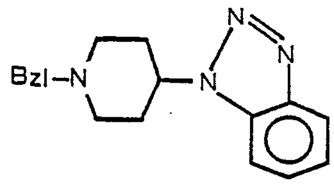

In this reference example, 2.0 g of 1-benzyl-4-(2-aminoanilino)-piperidine trihydrochloride obtained in the same manner as that of Reference Example 11 is dissolved in 30 ml of water and the solution is cooled to 0°C. To the cooled solution, 5 ml of a solution of 360 mg of sodium nitrite in 5 ml of water is added dropwise under the current of nitrogen gas over a period of 30 minutes. After completion of the dropwise addition, the reaction mixture is stirred at 0—5°C for one hour and then adjusted to pH 10.7. The reaction mixture is extracted with 30 ml of chloroform three times. The chloroform layer is washed with water and dried. The chloroform layer is concentrated to dryness under reduced pressure. The residue is subjected to silica gel chromatography [Wako Gel C—200, 150 ml, with a chloroform-methanol (10:1) as the eluent]. The eluate is concentrated to obtain 0.71 g of the desired product.

Melting point: 110—111°C

Infrared absorption spectrum (KBr): 1461, 1140, 1083 $cm^{-1}$

Elementary analysis (%): $C_{18}H_{20}N_4$

| | C | H | N |
|---|---|---|---|
| Calculated | 73.94 | 6.90 | 19.16 |
| Found | 74.12 | 6.88 | 19.27 |

Reference Example 13

4-(1H-benzotriazol-1-yl)-piperidine acetate:

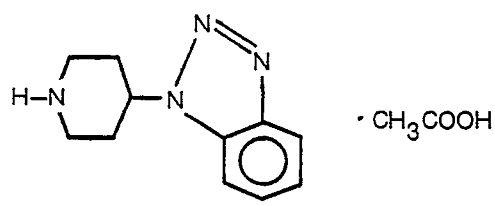

A solution of 8.76 g 1-benzyl-4-(1H-benzotriazol-1-yl)-piperidine obtained in the same manner as that of Reference Example 12 in 200 ml of methanol is mixed with 9.0 g of acetic acid and 20 g of 5% palladium carbon and shaken in the atmosphere of hydrogen at 50°C under the initial pressure of 5 atmospheres. The reaction mixture is filtered and the filtrate is concentrated. The residue is recrystallized from ethyl acetate to obtain 8.6 g of the desired product.

Melting point: 132.5—135.5°C

IR (KBr): 1583, 1175 $cm^{-1}$

Elementary analysis (%): $C_{11}H_{14}N_4 \cdot C_2H_4O_2$

| | C | H | N |
|---|---|---|---|
| Calculated | 59.53 | 6.92 | 21.36 |
| Found | 59.31 | 6.97 | 21.62 |

Reference Example 14

1-Benzyl-4-[N-(2-nitrophenyl)-N-acetylamino]-piperidine hydrobromide:

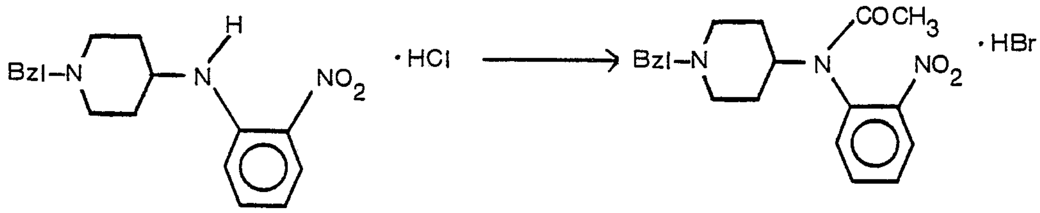

In this reference example, 1.67 g of 1-benzyl-4-(2-nitroanilino)-piperidine hydrochloride is suspended in 30 ml of dioxane. The suspension is mixed with 13 ml of acetyl bromide and refluxed for 4 hours. The resultant reaction mixture is cooled and then the deposited crystals are separated by

filtration. The crystals are washed with ether and then dried to obtain 1.96 g of crude crystals. The crude crystals are recrystallized from 25 ml of ethanol to obtain 1.33 g of the desired product.

Melting point: 253—255°C

IR (KBr): 1665, 1658, 1530, 1378 $cm^{-1}$

Elementary analysis (%): $C_{20}H_{24}BrN_3O_3$

| | C | H | N |
|---|---|---|---|
| Calculated | 55.31 | 5.57 | 9.67 |
| Found | 55.41 | 5.54 | 9.38 |

Reference Example 15

4-(2-Methyl-benzimidazol-3-yl)-piperidine:

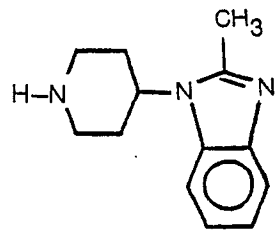

In this reference example, 14.8 g of 1-benzyl-4-[N-(2-nitrophenyl)-N-acetylamino]-piperidine hydrobromide obtained in the same manner as that of Reference Example 14, 250 ml of methanol and 1.0 g of 5% palladium carbon are mixed and stirred at room temperature under the atmospheric pressure in the current of hydrogen for 12 hours. The resultant reaction mixture is filtered. The filtrate is mixed with 29 ml of concentrated hydrochloric acid and stirred overnight. The reaction solution is concentrated and the residue is dissolved in 50 ml of water. The solution is adjusted to pH 11.0 with 1N aqueous sodium hydroxide. The solution is extracted with 50 ml of chloroform three times, and the extract is washed with 20 ml of a saturated aqueous sodium chloride and dried. The extract is freed from the solvent by distillation. The residue is mixed with n-hexane and stirred for crystallization. The crystals are separated by filtration, recrystallized from a mixed solvent of n-hexane and ethanol to obtain 6.7 g of 4-(2-methyl-benzimidazol-3-yl)-piperidine.

Melting point: 130—132°C

IR (KBr): 1468 $cm^{-1}$

Elementary analysis (%): $C_{13}H_{17}N_3$

| | C | H | N |
|---|---|---|---|
| Calculated | 72.52 | 7.96 | 19.52 |
| Found | 72.33 | 8.01 | 19.62 |

Reference Example 16

1-tert.-Butoxycarbonyl-4-(2-cyanoimino-3,4-dihydro-1H-quinazolin-3-yl)-piperidine:

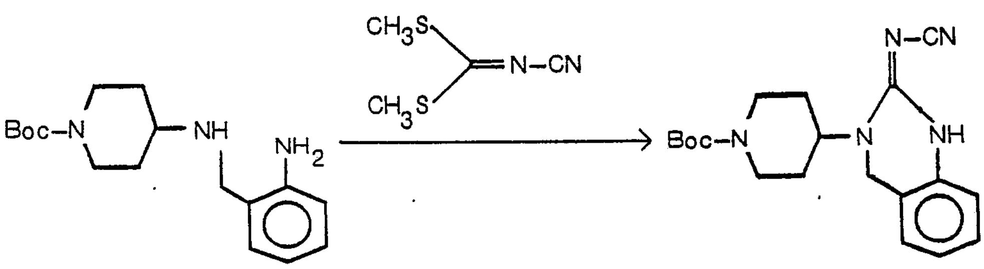


In this reference example, a solution of 28.0 g of 1-tert.-butoxycarbonyl-4-(2-aminophenylmethylamino)-piperidine, 2 g of dimethyl-N-cyanodithioiminocarbonate and 28 g of diazabicycloundecene in 150 ml of dioxane is heated at 63°C for 10 hours. The solution is then cooled and the deposited crystals are separated by filtration. The crystals are dried and then recrystallized from methanol to obtain 18 g of 1-tert.-butoxycarbonyl-4-(2-cyanoimino-3,4-dihydro-1H-quinazolin-3-yl)-piperidine.

Melting point: 249—250°C

IR (KBr): 2300, 1688—1680, 1627, 1585 $cm^{-1}$

Elementary analysis (%): $C_{19}H_{25}N_5O_2$

| | C | H | N |
|---|---|---|---|
| Calculated | 64.20 | 7.09 | 19.71 |
| Found | 64.43 | 7.24 | 19.69 |

Reference Example 17

4-(2-Cyanoimino-3,4-dihydro-1H-quinazolin-3-yl)-piperidine hydrochloride:

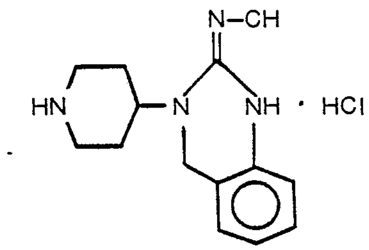

In this reference example, 13.0 g of the product obtained in Reference Example 16 is suspended in 100 ml of ethyl acetate. The suspension is cooled to 0°C. To the cooled suspension, 60 ml of ethyl acetate solution of 5.8N HCl is added dropwise over a period of one hour. After the dropwise addition, the solution is further stirred at the same temperature for 30 minutes and then filtered. The resultant crystals are washed with 20 ml of ethyl acetate three times and dried to obtain 9.9 g of the desired compound.

Melting point: 260—262°C (decomposition)

IR (KBr): 2300, 1623, 1588 $cm^{-1}$

Elementary analysis (%): $C_{14}H_{18}ClN_4$

| | C | H | N |
|---|---|---|---|
| Calculated | 57.63 | 6.22 | 24.00 |
| Found | 57.61 | 6.33 | 23.87 |

Reference Example 18

1-tert.-Butoxycarbonyl-4-[2-(methylthiocyanoiminomethyl)-aminoanilino]-piperidine

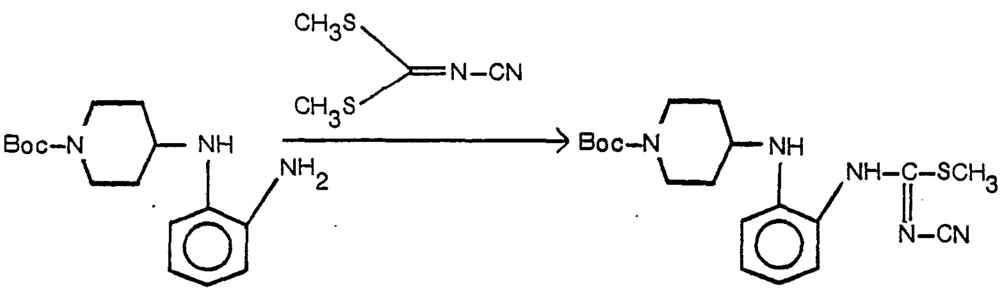

In this reference example, a solution of 1.0 g of 1-tert.-butoxycarbonyl-4-(2-aminoanilino)-piperidine, 1.0 g of dimethyl-N-cyanodithioiminocarbonate and 1.2 g of diazabicycloundecene in 50 ml of dioxane is heated to 80°C for 7 hours. The solution is freed from dioxane by distillation under reduced pressure. The residue is mixed with water and ethyl acetate and shaken and then the organic layer is separated. The organic layer is washed with 10 ml of 0.6N hydrochloric acid four times. The organic layer is further washed with a saturated aqueous sodium bicarbonate and water and then dried. The dried organic layer is concentrated to dryness under reduced pressure. The residue is subjected to silica gel chromatography (Wako Gel Q—200, 50 ml, with $CHCl_3$ as the eluent). The eluate is concentrated and the residual crystals are recrystallized from ethanol to obtain 0.6 g of the desired product.

Melting point: 169—171°C

IR (KBr): 2201, 1695, 1690, 1605, 1575 $cm^{-1}$

Elementary analysis (%): $C_{19}H_{27}N_5O_2S$

| | C | H | N |
|---|---|---|---|
| Calculated | 58.59 | 6.99 | 17.98 |
| Found | 58.81 | 7.08 | 18.00 |

Reference Example 19

1-tert.-Butoxycarbonyl-4-(2-cyanoimino-1H-benzimidazol-1-yl)-piperidine:

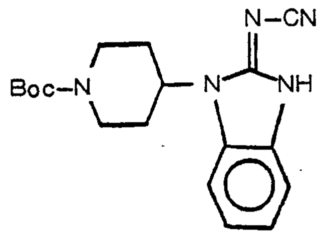

In this reference example, 2.48 g of the product obtained in the same manner as that of Reference Example 18 is suspended in 22 ml of methanol. This suspension is mixed with 2.1 g of mercuric acetate and stirred at room temperature for one hour. After completion of the reaction, the reaction solution is concentrated to dryness under reduced pressure. The residue is mixed with 30 ml of chloroform. The chloroform solution is washed four times with 10 ml of 0.32N hydrochloric acid. The chloroform solution is further washed with 10 ml of a saturated aqueous sodium chloride four times and then dried. The solution is concentrated to dryness under reduced pressure and the residual crystals are recrystallized from ethanol to obtain 1.88 g of the desired product.

Melting point: 242—243°C

IR (KBr): 2198, 1699—1690, 1633, 1613 $cm^{-1}$

Elementary analysis (%): $C_{18}H_{23}N_5O_2$

| | C | H | N |
|---|---|---|---|
| Calculated | 63.32 | 6.79 | 20.52 |
| Found | 63.47 | 6.89 | 20.47 |

Reference Example 20

4-(2-Cyanoimino-1H-benzimidazol-1-yl)-piperidine hydrochloride:

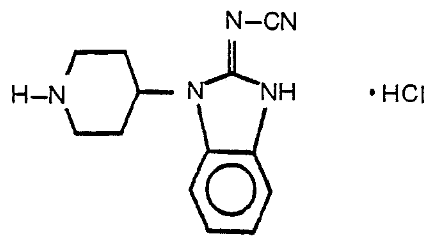

In this reference example, 18 g of the product obtained in the same manner as that of Reference Example 19 is suspended in 530 ml of ethyl acetate. To this suspension, 180 ml of ethyl acetate solution of 5.8N HCl is added dropwise at room temperature over a period of 30 minutes. The mixture is stirred for 2 hours and then filtered. The resultant crystals are washed with 20 ml of ethyl acetate five times and then dried to obtain 13.3 g of the desired product.

Melting point: 273—276°C (decomposition)

IR (KBr): 2195, 1630, 1601 $cm^{-1}$

Elementary analysis (%): $C_{13}H_{16}ClN_5$

| | C | H | N |
|---|---|---|---|
| Calculated | 56.22 | 5.81 | 25.21 |
| Found | 56.43 | 5.97 | 24.98 |

Reference Example 21

1-Benzyl-4-(3,4-dihydro-2,2-dioxo-1H-2,1,3-benzothiadiazin-3-yl)-piperidine:

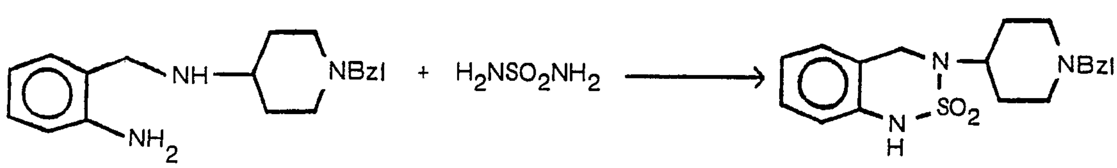

In this reference example, 590 mg of 1-benzyl-4-[N-(o-aminobenzyl)-amino]-piperidine, 600 mg of sulfurylamide and 12 ml of pyridine are mixed and refluxed with heating for 2 hours. The reaction mixture is cooled to room temperature and poured into 50 ml of ice water. The white crystals deposited

are separated by filtration, washed with 10 ml of water and dried to obtain 520 mg of the desired product as crystals.

NMR (δ ppm in $CDCl_3$): 1.37—2.27 (6H, m), 2.66—3.10 (2H, m), 3.42 (2H, s), 3.83 (1H, m), 4.62 (2H, s), 5.76 (1H, s) (exchangeable proton), 6.33—7.46 (9H, m)

IR (KBr): 1340, 1165 ($SO_2$) $cm^{-1}$

Reference Example 22

4-(3,4-Dihydro-2,2-dioxo-1H-2,1,3-benzothiadiazin-3-yl)-piperidine:

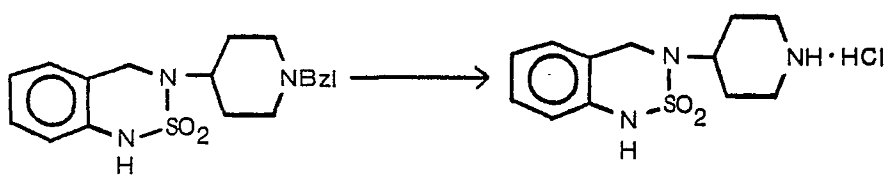

In this reference example, 7.14 g (20 m.mols) of 1-benzyl-4-(3,4-dihydro-2,2-dioxo-1H-2,1,3-benzothiadiazin-3-yl)-piperidine, 2 g of 10% palladium carbon, 20 ml of 1N hydrochloric acid, 60 ml of water and 120 ml of methanol are mixed. While the mixture is stirred at 40°C, hydrogen gas is blown into the mixture at 40°C for 20 hours. The reaction mixture is filtered to remove palladium carbon. The filtrate is concentrated to dryness to obtain 5.0 g of the desired product.

NMR (δ ppm in $d_6DMSO$): 1.47—2.20 (4H, m), 2.60—4.35 (5H, m), 4.63 (2H, s), 6.60—7.40 (4H, m), 9.10 (2H, broad s) (exchangeable proton), 10.46 (1H, s) (exchangeable proton)

IR (KBr): 1330, 1155 ($SO_2$) $cm^{-1}$

Reference Example 23

1-Benzyl-4-(3,4-dihydro-2,2-dioxo-2,1,3-benzothiadiazin-1-yl)-piperidine monohydrochloride:

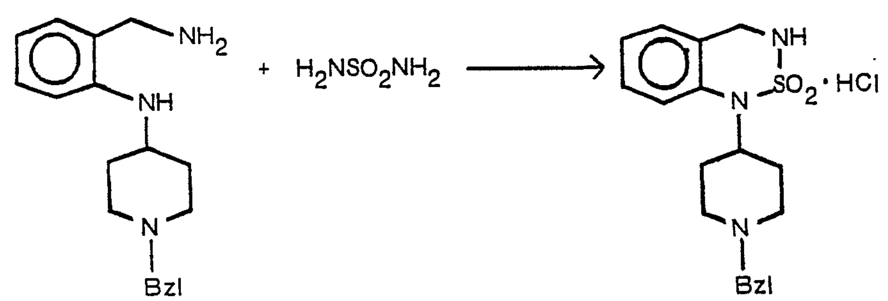

In this reference example, 33.0 g (112.4 m.mols) of 1-benzyl-4-[N-(o-aminomethylphenyl)-amino]-piperidine, 21.6 g (224.8 m.mols) of sulfuryl amide and 250 ml of pyridine are mixed and refluxed with heating for 8 hours. The reaction solution is cooled to room temperature, concentrated to 70 ml and poured into 500 ml of ice water. The mixture is mixed with 300 ml of chloroform, stirred and adjusted to pH 9 with 2N aqueous sodium hydroxide. The chloroform layer is separated with separating funnel and the remaining water layer is further extracted with chloroform (300 ml × 3). The chloroform layers are combined, washed with water (300 ml × 4), dried and concentrated to obtain an oily residue. This residue is dissolved in 150 ml of ethanol. To the solution, 50 ml of 5.7N hydrogen chloride in ethyl acetate is added. The resultant solution is concentrated under reduced pressure. The crystalline residue is mixed with 50 ml of ethanol and filtered to obtain 29.0 g of the desired product. This compound is tested for NMR after it has been freed from hydrochloric acid, and it is tested for IR in its unaltered form of hydrochloride.

NMR (δ ppm in $CDCl_3$): 1.66—2.33 (6H, m), 2.6—3.2 (2H, m), 3.48 (2H, s), 4.0 (1H, m), 4.43 (2H, s), 4.80 (1H, br.s) (exchangeable proton), 7.0—7.4 (9H, m)

IR (KBr): 1330, 1165 ($SO_2$) $cm^{-1}$

Reference Example 24

4-(3,4-Dihydro-2,2-dioxo-2,1,3-benzothiadiazin-1-yl)piperidine monohydrochloride:

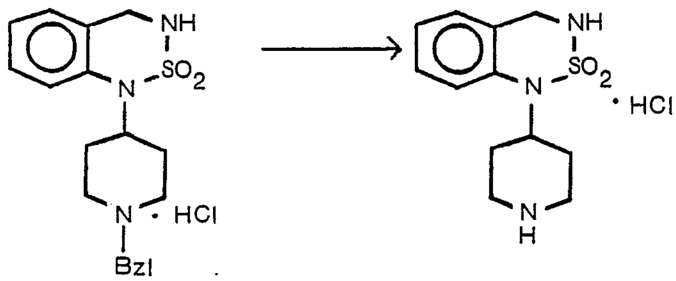

While a mixture of 16 g of 1-benzyl-4-(3,4-dihydro-2,2-dioxo-2,1,3-benzothiadiazin-1-yl)-piperidine-monohydrochloride, 4 g of 10% palladium carbon, 160 ml of water and 240 ml of methanol is stirred at 40°C, hydrogen gas is blown into the mixture for 20 hours. Then, the resultant reaction mixture is freed from palladium carbon by filtration. The filtrate is concentrated to dryness to obtain 11.2 g of crude crystals of the desired product. This compound is tested for NMR after it has been freed from hydrochloric acid, and it is tested for IR in its unaltered form of hydrochloride.

NMR ($\delta$ ppm in $CDCl_3$): 1.60—3.26 (8H, m), 3.70—4.26 (3H, m) (exchangeable proton 2H), 4.40 (2H, s), 6.86—7.46 (4H, m)

IR (KBr): 1340, 1160 ($SO_2$) $cm^{-1}$

Reference Example 25

3-[1-(tert.-Butoxycarbonyl)-4-piperidinylidene]-2-oxyindole:

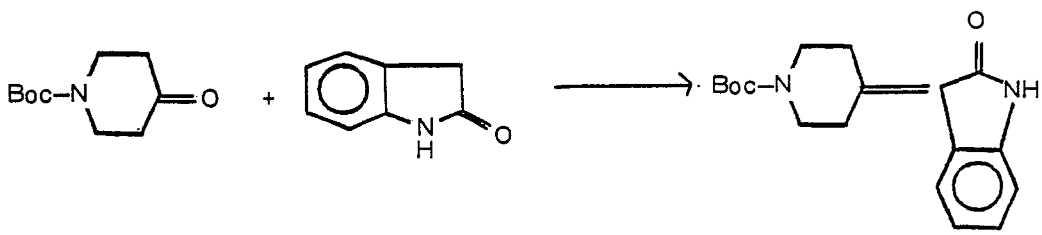

In this reference example, an ethanol solution of 2.4 g of ammonia is prepared by bubbling ammonia gas through 20 ml of ethanol. To this solution, 1.51 g of 1-tert.-butoxycarbonyl-4-piperidone and 1.01 g of oxyindole are added at 5°C. The resultant mixture is gradually heated to 70°C. The mixture is stirred at the same temperature for one hour and then freed from ammonia and the solvent by distillation under reduced pressure. The residue is mixed with a small amount of ethanol and then stirred. The crystals consequently deposited are separated by filtration and dried to obtain 1.7 g of crude crystals. The crude crystals are recrystallized from ethanol to obtain 1.62 g of the desired product.

Melting point: 204—205°C

IR (KBr): 1700, 1690, 1660 $cm^{-1}$

Elementary analysis (%): $C_{18}H_{22}N_2O_3$

| | C | H | N |
|---|---|---|---|
| Calculated | 68.77 | 7.05 | 8.91 |
| Found | 68.66 | 7.05 | 8.82 |

Reference Example 26

3-(4-Piperidinylidene)-2-oxyindole:

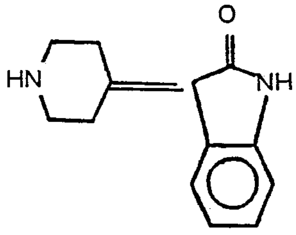

In this reference example, 7.5 g of 3-[1-(tert.-butoxycarbonyl)-4-piperidinylidene]-2-oxyindole obtained in the same manner as that of Reference Example 25 is added by portions to 25 g of

trifluoroacetic acid which is cooled to 0°C. The resultant mixture is stirred at the same temperature for 2 hours and then concentrated to dryness under reduced pressure. The residue is mixed with water, and the resultant solution is adjusted to pH 10.6 with 0.1N aqueous sodium hydroxide. The deposited crystals are separated by filtration and washed with water. The crude crystals are dried and then recrystallized from ethyl acetate to obtain 4.4 g of the desired product.

Melting point: 234—238°C (decomposition)

IR (KBr): 1697, 1680 $cm^{-1}$

Elementary analysis (%): $C_{13}H_{14}N_2O$

| | C | H | N |
|---|---|---|---|
| Calculated | 72.87 | 6.59 | 13.08 |
| Found | 72.59 | 6.60 | 12.81 |

Reference Example 27

4-(2-Oxyindol-3-yl)piperidine hydrochloride:

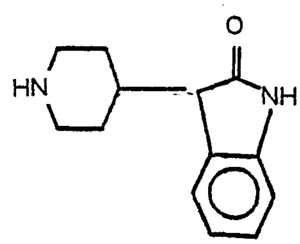

In this reference example, 0.5 g of 5% palladium carbon is added to a solution of 4.0 g of 3-(4-piperidinylidene)-2-oxyindole obtained in the same manner as that of Reference Example 26 in 200 ml of methanol. The mixture is stirred in the atmosphere of hydrogen under the atmospheric pressure for 10 hours. The mixture is filtered and the filtrate is concentrated. The residue is dissolved in 10 ml of ethanol. The crystals which are deposited therein by blowing dry hydrogen chloride gas into the solution are separated by filtration and then dried. The crude crystals are recrystallized from ethanol to obtain 3.8 g of the desired product.

Melting point: 284—286°C (decomposition)

IR (KBr): 1718 $cm^{-1}$

Elementary analysis (%): $C_{13}H_{16}N_2O{\cdot}HCl$

| | C | H | N |
|---|---|---|---|
| Calculated | 61.78 | 6.78 | 11.08 |
| Found | 61.65 | 6.86 | 11.04 |

Reference Example 28

1-Benzyl-4-(2-nitro-cyclohexylamino)-piperidine:

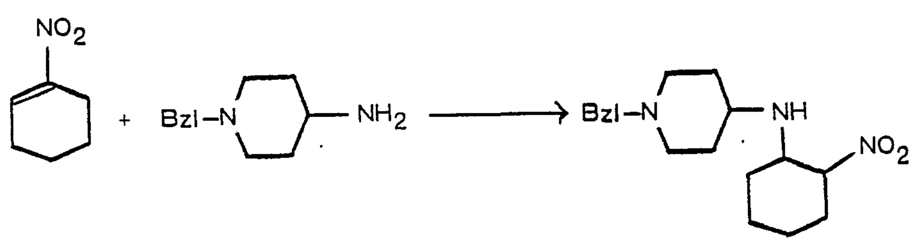

In this reference example, a solution of 16.6 g of 1-nitro-cyclohexene and 23 g of 1-benzyl-4-amino-piperidine in 150 ml of tetrahydrofuran is stirred overnight at room temperature. The reaction solution is filtered and the filtrate is concentrated. The residue is dissolved in 250 ml of 0.6N hydrochloric acid. The solution is shaken with ether twice and then the water layer is separated. The water layer is adjusted to pH 9.5 with 1N aqueous sodium hydroxide. The oily substance separated is extracted with 100 ml of ether three times. The extract is washed with water and then dried. The dried extract is concentrated under reduced pressure to obtain 30 g of an oily residue. This oily residue is subjected to silica gel chromatography (Wako Gel Q—200, with ethyl acetate as the eluent) to obtain 22.2 g of the desired product in an oily form.

IR (neat): 1545 $cm^{-1}$

NMR ($CDCl_3$): 0.75—3.4, 3.42, 3.85—4.5, 7.25 ppm

Elementary analysis (%): $C_{18}H_{27}N_3O_2$

| | C | H | N |
|---|---|---|---|
| Calculated | 68.11 | 8.57 | 13.24 |
| Found | 68.10 | 8.78 | 13.21 |

Reference Example 29

1-Benzyl-4-(2-amino-cyclohexylamino)-piperidine:

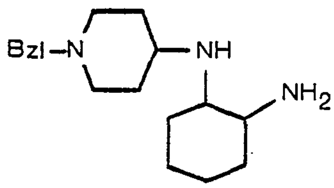

In this reference example, 1.0 g of 1-benzyl-4-(2-nitro-cyclohexylamino)-piperidine obtained in Reference Example 28 is dissolved in 25 ml of ethanol. Then, 1.0 g of a Raney-nickel catalyst is added thereto and the mixture is stirred at room temperature in the atmosphere of hydrogen under the atmospheric pressure. The stirring is ceased when the solution has absorbed the theoretical amount of hydrogen, and the reaction solution is filtered. The filtrate is concentrated. Consequently, 0.89 g of 1-benzyl-4-(2-amino-cyclohexylamino)-piperidine is obtained as the residue.

IR (neat): 1580, 1450—1422 $cm^{-1}$

Although this compound is in a free form, it can be converted into a crystalline hydrochloride by introducing hydrogen chloride gas into an ethanol solution of the compound.

The hydrochloride is recrystallized from methanol. The trihydrochloride of 1-benzyl-4-(2-amino-cyclohexylamino)-piperidine exhibits the following properties.

Melting point: 245—247°C

Elementary analysis (%): $C_{18}H_{32}N_3Cl_3$

| | C | H | N |
|---|---|---|---|
| Calculated | 54.48 | 8.13 | 10.59 |
| Found | 54.25 | 8.38 | 10.43 |

Reference Example 30

1-Benzyl-4-(octahydro-2H-benzimidazol-2-one-1-yl)-piperidine:

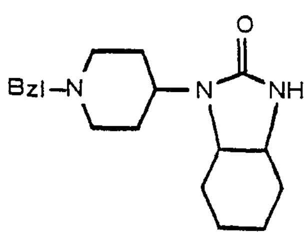

In this reference example, a solution of 631 mg of 1-benzyl-4-(2-amino-cyclohexylamino)-piperidine in a free form obtained in Reference Example 29 and 550 mg of 1,1′-carbonyldiimidazole in 10 ml of acetonitrile is stirred at room temperature for 12 hours. The deposited crystals are separated by filtration, washed with acetonitrile and then dried to obtain 383 mg of crude crystals. The crude crystals are recrystallized from 34 ml of isopropanol to obtain 300 mg of the desired product.

Melting point: 192—193°C

IB (KBr): 1680 $cm^{-1}$

Elementary analysis (%): $C_{19}H_{27}N_3O$

| | C | H | N |
|---|---|---|---|
| Calculated | 72.80 | 8.68 | 13.41 |
| Found | 72.71 | 8.97 | 13.39 |

Reference Example 31

4-(Octahydro-2H-benzimidazol-2-one-1-yl)-piperidine hydrochloride:

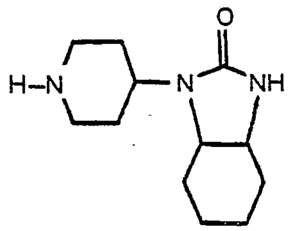

In this reference example, a solution of 6.1 g of 1-benzyl-4-(octahydro-2H-benzimidazol-2-one-1-yl)-piperidine obtained in the same manner as that of Reference Example 30 and 1 g of 5% palladium carbon in 100 ml of methanol, 100 ml of water and 20 ml of 12N hydrochloric acid is shaken in the atmosphere of hydrogen under 4 to 5 atmosphere in an autoclave. The shaking is ceased when the absorption of hydrogen has been completed. The reaction mixture is subjected to filtration to remove the catalyst and the filtrate is concentrated. The residue is mixed with 20 ml of isopropanol and stirred to crystallize the reaction product. The crystals are dried and recrystallized from ethanol to obtain 3.9 g of the desired product.

Melting point: 300°C (coloring)

IR (KBr): 1658, 1637 (shoulder) $cm^{-1}$

Elementary analysis (%): $C_{12}H_{22}ClN_2O$

| | C | H | N |
|---|---|---|---|
| Calculated | 55.48 | 8.54 | 16.18 |
| Found | 55.49 | 8.80 | 16.01 |

**Claims**

1. Compounds of the formula:

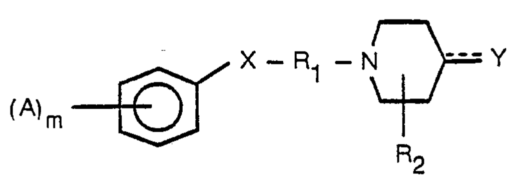

wherein A is hydroxy, halogen, $C_{1-5}$ alkyl, $C_{1-5}$ alkoxy, alkenyloxy containing up to 5 carbon atoms, alkynyloxy containing up to 5 carbon atoms, $C_{1-5}$ alkylthio, carboxy, $C_{2-6}$ alkoxycarbonyl, nitro, amino, $C_{1-5}$ alkylamino, $C_{1-5}$ alkanoylamino, sulfamoyl, mono- or di($C_{1-5}$ alkyl)aminosulfonyl, $C_{1-5}$ alkylsulfonyl, carbamoyl, cyano or trifluoromethyl;

m is 0 or an integer of 1—5, and when m is 2 or more, the A groups may be the same or different and any two A's may together form a $C_{1-5}$ alkylenedioxy group;

X is carbonyl, hydroxymethylene or methylene;

$R_1$ is straight-chain alkylene having 1—4 carbon atoms with or without $C_{1-5}$ alkyl substituent(s):

$R_2$ is hydrogen or $C_{1-5}$ alkyl;

and Y is a monovalent or divalent group of the formula 1, 2 or 3:

1) 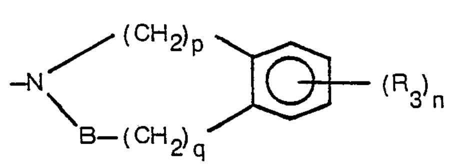

wherein p and q are each 0 and 1 provided that p and q are not both 1 at the same time; and when p and q are both 0, B is —N=N—, 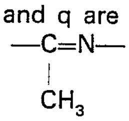 or 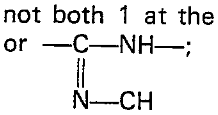;

p is 1 and q is 0, B is —CONH—, —COO—, 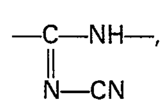, or —$SO_2$NH—; and when

p is 0 and q is 1, B is —CONH or —$SO_2$NH—;

and $R_3$ is hydroxy, $C_{1-5}$ alkoxy, halogen, trifluoromethyl, trifluoromethoxy, trifluoromethylthio, nitro or amino;

n is 0 or an integer of 1—4, and when n is 2 or more, the $R_3$ groups may be the same or different and any two $R_3$ groups may together form a $C_{1-5}$ alkylenedioxy group;

2) 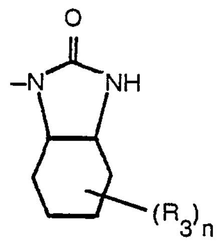

wherein $R_3$ and n are as defined above, and

3) 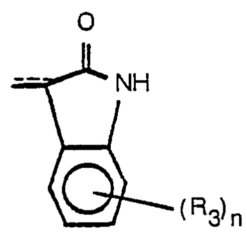

wherein $R_3$ and n are as defined above; and pharmacologically acceptable acid addition salts thereof.

2. Compounds according to claim 1 represented by the formula:

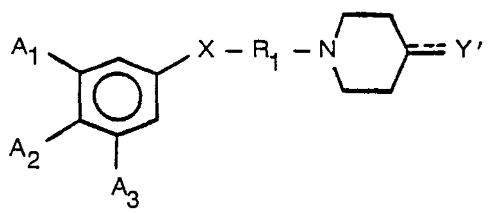

wherein $A_1$, $A_2$ and $A_3$, which may be the same or different, are hydrogen or are as defined for A;

X and $R_1$ are as defined in claim 1; and

Y' is a monovalent or divalent group of the formula:

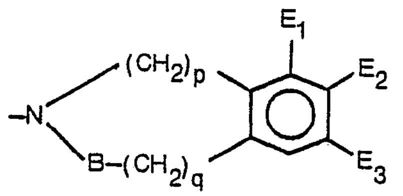 , 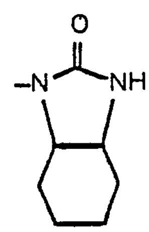 or 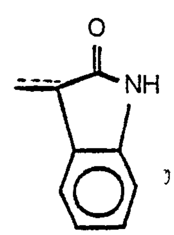

where B, p and q are as defined in claim 1;

and $E_1$, $E_2$, $E_3$ are H or as defined in claim 1 for $R_3$ and pharmacologically acceptable acid addition salts thereof.

3. Compounds or salts according to claim 2 wherein at least one of $A_1$, $A_2$ and $A_3$ is $C_{1-5}$ alkoxy or halogen, or $A_1$ and $A_2$ or $A_2$ and $A_3$ together form a $C_{1-5}$ alkylenedioxy group.

4. Compounds or salts according to claim 2 or 3 wherein X is carbonyl or hydroxymethylene.

5. Compounds or salts according to claim 2, 3 or 4 wherein $R_1$ is methylene with or without $C_{1-5}$ alkyl substituent(s).

6. A salt according to any one of the preceding claims which is an acid addition salt of said compound with hydrochloric, hydrobromic, hydriodide, nitric, sulfuric, phosphoric, acetic, benzoic, maleic, fumaric, succinic, tartrate, citric, oxalic, glyoxylic, aspartic, methanesulfonic, ethanesulfonic, propanesulfonic, methanedisulfonic, $\alpha,\beta$-ethanedisulfonic or benzenesulfonic acid.

7. A pharmaceutical composition which comprises pharmaceutically acceptable carrier(s) and a compound or salt as claimed in any one of the preceding claims.

8. A process for the preparation of the compounds claimed in claim 1, which comprises reacting a compound of the formula:

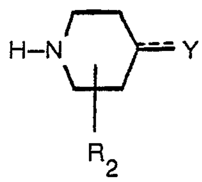

where Y and $R_2$ are as defined in claim 1, with the proviso that any reactive substituent $R_3$ group in the group Y may be protected by a labile protecting group;
with a compound of the formula

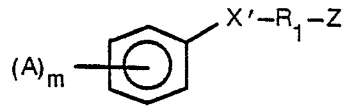

where A, m and $R_1$ are as defined in claim 1, with the proviso that any reactive A substituents may be protected by a labile protecting group;

X' is carbonyl or methylene; and

Z is halogen or other eliminable group reactive with the NH group of the first reactant;
and further, if desired, reducing the X' group, when carbonyl, in the product so formed to form a hydroxymethylene group X, and/or removing the labile protecting group(s) in Y and/or A.

**Revendications**

1. Composés de formule:

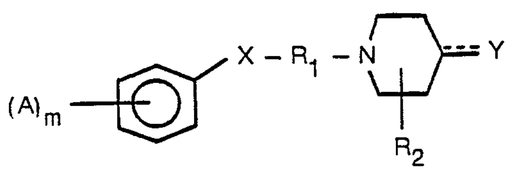

dans laquelle A représente un hydroxy, halogène, alkyle en $C_{1-5}$, alkoxy en $C_{1-5}$, alcényloxy contenant jusqu'à 5 atomes de carbone, alkynyloxy contenant jusqu'à 5 atomes de carbone, alkylthio en $C_{1-5}$, carboxy, alkoxycarbonyle en $C_{2-6}$, nitro, amino, alkylamino en $C_{1-5}$, alcanoylamino en $C_{1-5}$, sulfamoyle, mono-ou di(alkyle en $C_{1-5}$)-aminosulfonyle, alkylsulfonyle en $C_{1-5}$, carbamoyle, cyano ou trifluorométhyle;

m est 0 ou un entier de 1—5, et lorsque m est égal à 2 ou plus, les groupes A peuvent être identiques ou différents et deux A quelconques peuvent former ensemble un groupe alkylènedioxy en $C_{1-5}$;

X est un groupe carbonyle, hydroxyméthylène ou méthylène;

$R_1$ est un alkylène à chaine linéaire possédant 1—4 atomes de carbone avec ou sans substituant(s) alkyle(s) en $C_{1-5}$;

$R_2$ est l'hydrogène ou un alkyle en $C_{1-5}$; et

Y est un groupe monovalent ou divalent de formule 1, 2 ou 3:

1) 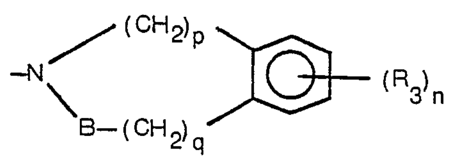

dans laquelle p et q sont chacun 0 ou 1 pourvu que p et q ne soient pas tous les deux 1 en même temps; et lorsque p et q sont tous les deux 0, B est —N=N—, $-\underset{CH_3}{\underset{|}{C}}=N-$ ou $-\underset{N-CN}{\underset{\|}{C}}-NH-$;

p est 1 et q est 0, B est —CONH—, —COO—, $-\underset{N-CN}{\underset{\|}{C}}-NH-$, ou —$SO_2NH$—; et lorsque

p est 0 et q est 1, B est —CONH ou —$SO_2NH$—;

et $R_3$ est un hydroxy, alkoxy en $C_{1-5}$, halogène, trifluorométhyle, trifluorométhoxy, trifluorométhylthio, nitro ou amino;

n est 0 ou un entier de 1—4, et lorsque n est 2 ou plus, les groupes $R_3$ peuvent être identiques ou différents et deux groupes $R_3$ quelconques peuvent former ensemble un groupe alkylènedioxy $C_{1-5}$;

2) 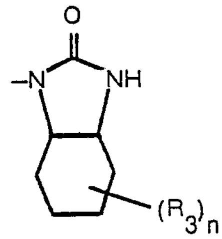

dans laquelle $R_3$ et n sont tels que définis ci-dessus, et

3) 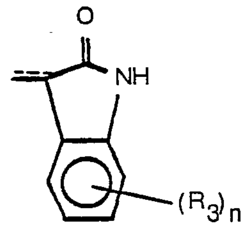

dans laquelle $R_3$ et n sont tels que définis ci-dessus; et leurs sels d'addition d'acide pharmacologiquement acceptables.

2. Composés selon la revendication 1 représentés par la formule:

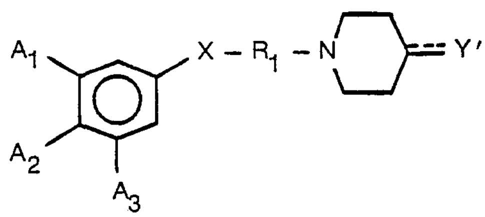

dans laquelle $A_1$, $A_2$ et $A_3$, qui peuvent être identiques ou différents, sont l'hydrogène ou sont tels que définis pour A;

X et $R_1$ sont tels que définis dans la revendication 1; et

Y' est un groupe monovalent ou divalent de formule:

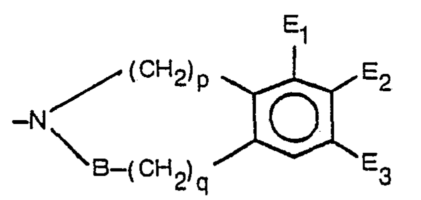 , 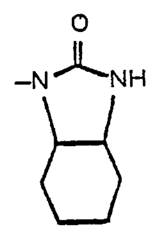 ou 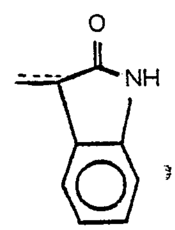 ;

où B, p et q sont tels que définis dans la revendication 1;

et $E_1$, $E_2$ et $E_3$ sont H ou tels que définis dans la revendication 1 pour $R_3$

et leurs sels d'addition d'acide pharmacologiquement acceptables.

3. Composés ou sels selon la revendication 2 dans lesquels au moins l'un des groupes $A_1$, $A_2$ et $A_3$ est un alkoxy en $C_{1-5}$ ou un halogène, ou $A_1$ et $A_2$ ou $A_2$ et $A_3$ forment ensemble un groupe alkylènedioxy $C_{1-5}$.

4. Composés ou sels selon la revendication 2 ou 3 dans lequels X représente carbonyle ou hydroxyméthylène.

5. Composés ou sels selon la revendication 2, 3 ou 4 dans lesquels $R_1$ est méthylène avec ou sans substituant(s) alkyle(s) $C_{1-5}$.

6. Sel selon l'une quelconque des revendications précédentes qui est un sel d'addition d'acide dudit composé avec l'acide chlorhydrique, bromhydrique, iodhydrique, nitrique, sulfurique, phosphorique, acétique, benzoïque, maléique, fumarique, succinique, tartrique, citrique, oxalique, glyoxylique, aspartique, méthanesulfonique, éthanesulfonique, propanesulfonique, méthanedisulfonique, $\alpha,\beta$-éthanedisulfonique ou benzène sulfonique.

7. Composition pharmaceutique qui comprend un support(s) pharmaceutiquement acceptable(s) et un composé ou un sel tel que revendiqué dans l'une quelconque des revendications précédentes.

8. Procédé de préparation des composés revendiqués dans la revendication 1, caractérisé en ce qu'il comprend la réaction d'un composé de formule:

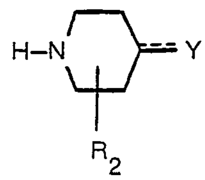

dans laquelle Y et $R_2$ sont tels que définis dans la revendication 1, à condition que tout groupe substituant réactif $R_3$ dans le groupe Y puisse être protégé par un groupe protecteur labile;

avec un composé de formule:

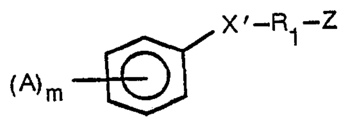

dans laquelle A, m et $R_1$ sont tels que définis dans la revendication 1, à condition que tout substituent A réactif puisse être protégé par un groupe protecteur labile;

X' est carbonyle ou méthylène; et

Z est halogène ou un autre groupe éliminable réactif avec le groupe NH du premier composé de la réaction;

et en outre, si on le désire, la réduction du groupe X', lorsqu'il est le carbonyle, en le produit ainsi obtenu pour former un groupe X hydroxyméthylène, et/ou l'élimination du groupe(s) protecteur(s) labile(s) dans Y et/ou A.

**Patentansprüche**

1. Verbindungen der Formel

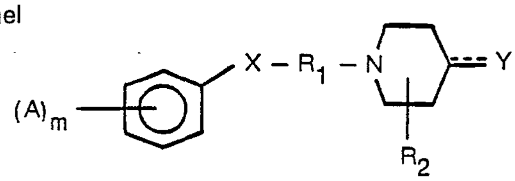

in der A ein Wasserstoffatom, Halogenatom, $C_{1-5}$-Alkylrest, $C_{1-5}$-Alkoxyrest, ein Alkenyloxyrest mit höchstens 5 Kohlenstoffatomen, Alkinyloxyrest mit höchstens 5 Kohlenstoffatomen, $C_{1-5}$-Alkylthiorest, Carboxyrest, $C_{2-6}$-Alkoxycarbonylrest, eine Nitrogruppe, Aminogruppe, ein $C_{1-5}$-Alkylaminorest, $C_{1-5}$-Alkanoylaminorest, Sulfamoylrest, mono- oder di-($C_{1-5}$-Alkyl)-Aminosulfonylrest, $C_{1-5}$-Alkylsulfonylrest, Carbamoylrest, eine Cyan- oder Trifluormethylgruppe bedeutet;

m Null oder eine ganze Zahl von 1 bis 5 ist, und wenn m 2 oder mehre ist, so sind die Reste A gleich oder verschieden und jeweils 2 der Reste A können zusammen eine $C_{1-5}$-Alkyendioxygruppe bilden;

X bedeutet einen Carbonyl-, Hydroxymethylen- oder Methylenrest;

$R_1$ bedeutet einen geradkettigen Alkylenrest mit 1 bis 4 Kohlenstoffatomen, mit oder ohne einem oder mehreren $C_{1-5}$-Alkylsubstituenten;

$R_2$ bedeutet ein Wasserstoffatom oder einen $C_{1-5}$-Alkylrest; und

Y stellt eine einwertige oder zweiwertige Gruppe der nachstehenden Formeln 1, 2 oder 3 dar:

1) 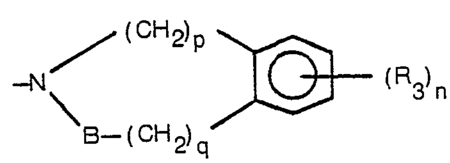

in der p und q jeweils Null oder 1 bedeutet, vorausgesetzt, daß p und q nicht gleichzeitig 1 sind; und sofern p und q beide Null bedeuten, ist der Rest B die Gruppe —N=N—,

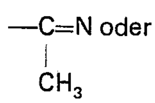 oder 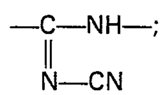;

sofern p 1 ist und q den Wert Null hat, ist B —CONH—, —COO—,

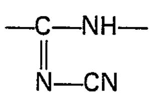 oder $—SO_2NH—$; und sofern

p den Wert Null und q den Wert 1 haben, ist B —CONH oder —$SO_2NH$—; der Rest $R_3$ bedeutet eine Hydroxylgruppe, $C_{1-5}$-Alkoxygruppe, eine Halogenatom, eine Trifluormethylgruppe, Trifluormethoxygruppe, Trifluormethylthiogruppe, Nitrogruppe oder eine Aminogruppe;

n ist Null oder eine ganze Zahl von 1 bis 4, und sofern n den Wert 2 oder mehr hat, können die Reste $R_3$ gleich oder verschieden sein und jeweils 2 der $R_3$-Rest können zusammengenommen eine $C_{1-5}$-Alkylendioxygruppe bilden;

2) 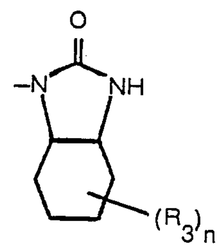

hier haben die Rest $R_3$ und n die vorstehende Bedeutung und

3) 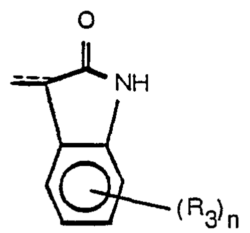

hier haben die Reste $R_3$ und n die vorstehende Bedeutung; sowie deren pharmazeutisch verträglich Säureadditionssalze.

2. Verbindungen nach Anspruch 1 der Formel

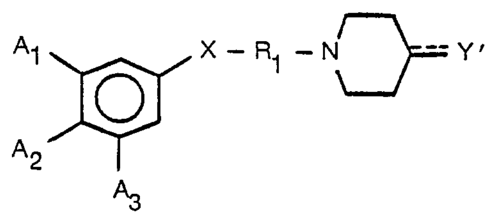

in der $A_1$, $A_2$ und $A_3$, die gleich oder verschieden sein können, ein Wasserstoffatom darstellen oder die gleiche Bedeutung haben wie der Rest A;

X und $R_1$ die in Anspruch 1 angegebene Bedeutung haben; und

Y' eine einwertige oder zweiwertige Gruppe der nachstehenden Formel darstellt:

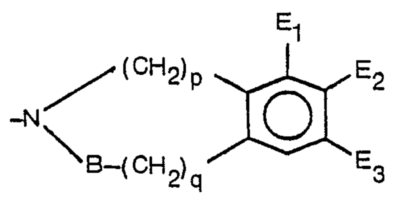 , 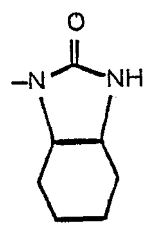 oder 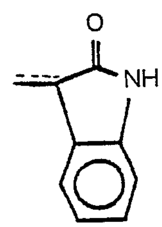 ;

wobei die Reste B, p und q die in Anspruch 1 angegebene Bedeutung haben;

und $E_1$, $E_2$ und $E_3$ ein Wasserstoffatom darstellen oder die Bedeutung von $R_3$ aus Patentanspruch 1 haben,

sowie deren pharmazeutisch verträgliche Säureadditionssalze.

3. Verbindungen oder Salze nach Anspruch 2, dadurch gekennzeichnet, daß mindestens einer der Reste $A_1$, $A_2$ und $A_3$ eine $C_{1-5}$-Alkoxygruppe oder ein Halogenatom bedeutet, oder $A_1$ und $A_2$ oder $A_2$ und $A_3$ zusammen eine $C_{1-5}$-Alkylendioxygruppe darstellen.

4. Verbindungen oder Salze nach den Ansprüchen 2 oder 3, dadurch gekennzeichnet, daß X eine Carbonylgruppe oder eine Hydroxymethylengruppe bedeutet.

5. Verbindungen oder Salze nach den Ansprüchen 2, 3 oder 4, dadurch gekennzeichnet, daß $R_1$ eine Methylengruppe mit oder ohne einem oder mehreren $C_{1-5}$-Alkylsubstituenten bedeutet.

6. Salze nach jedem der vorstehenden Patentansprüche, dadurch gekennzeichnet, daß es sich bei den Salzen um Additionssalze der genannten Verbindungen mit den nachstehenden Säuren handelt:

Chlorwasserstoffsäure, Bromwasserstoffsäure, Jodwasserstoffsäure, Salpetersäure, Schwefelsäure, Phosphorsäure, Essigsäure, Benzoesäure, Maleinsäure, Fumarsäure, Bernsteinsäure, Weinsäure, Citronensäure, Oxalsäure, Glyoxylsäure, Asparaginsäure, Methansulfonsäure, Äthansulfonsäure, Propansulfonsäure, Methandisulfonsäure, $\alpha,\beta$-Äthandisulfonsäure oder Benzolsulfonsäure.

7. Pharmazeutische Zubereitungen, enthaltend mindestens einen pharmazeutisch verträglichen Trägerstoff und eine Verbindung oder ein Salz gemäß einem der vorstehenden Patentansprüche.

8. Verfahren zur Herstellung der Verbindungen nach Anspruch 1, dadurch gekennzeichnet, daß man die Verbindung der Formel

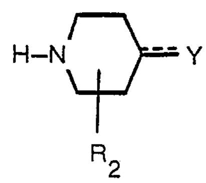

in der Y und $R_2$ die in Anspruch 1 angegebene Bedeutung haben, wobei jeder der reaktiven Reste $R_3$ in der Gruppen Y durch eine labile Schutzgruppe geschützt sein kann, mit einer Verbindung der nachstehenden Formel umsetzt

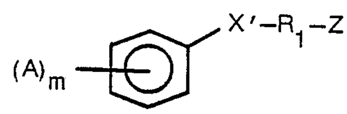

wobei in der vorstehenden Formel die Reste A, m und $R_1$ die in Anspruch 1 angegebene Bedeutung haben und jeder der reaktiven Substituenten A durch eine labile Schutzgruppe geschützt sein kann;

Y' eine Carbonyl- oder Methylengruppe bedeutet und

Z ein Halogenatom oder eine andere eliminierbare Gruppe darstellt, die befähigt ist, mit der NH-Gruppe des ersten Reaktanten zu reagieren;

und man gegebenenfalls, sofern die Gruppe X' eine Carbonylgruppe bedeutet, diese im entstandenen Produkt zu einer Hydroxymethylengruppe X reduziert, und man gegebenenfalls in den Resten Y und/oder A befindliche Schutzgruppen entfernt.